# EUROPÄISCHE PATENTSCHRIFT

(11) **EP 1 881 952 B1**
(45) Veröffentlichungstag und Bekanntmachung des Hinweises auf die Patenterteilung: **19.08.2015**
(21) Anmeldenummer: 06755164.8
(22) Anmeldetag: 11.05.2006
(51) Int. Cl.: C07C 51/25, C07C 253/18

(54) **VERFAHREN ZUR HERSTELLUNG WENIGSTENS EINES ZIELPRODUKTES DURCH PARTIELLE OXIDATION UND/ODER AMMOXIDATION VON PROPYLEN**
METHOD FOR THE PRODUCTION OF AT LEAST ONE FINAL PRODUCT BY PARTIAL OXIDATION AND/OR AMMOXIDATION OF PROPYLENE
PROCEDE DE PRODUCTION D'AU MOINS UN PRODUIT CIBLE PAR OXYDATION PARTIELLE ET/OU AMMOXYDATION DE PROPYLENE

(30) Priorität: 12.05.2005 DE 102005022798; 12.05.2005 US 679971 P
(43) Veröffentlichungstag der Anmeldung: 30.01.2008
(73) Patentinhaber: BASF SE, 67056 Ludwigshafen (DE)
(72) Erfinder: DIEFENBACHER, Armin, 76726 Germersheim (DE); HECHLER, Claus, 67063 Ludwigshafen (DE); ADAMI, Christoph, 69469 Weinheim (DE); DIETERLE, Martin, 68167 Mannheim (DE)
(86) Internationale Anmeldenummer: PCT/EP2006/062253
(87) Internationale Veröffentlichungsnummer: WO 2006/120233

(56) Entgegenhaltungen:
- DE-A1- 10 245 585
- DE-A1- 10 246 119
- DE-A1- 10 316 039

## Beschreibung

Vorliegende Erfindung betrifft ein Verfahren zur Herstellung wenigstens eines Zielprodukts P durch partielle Oxidation- und/oder Ammoxidation von Propylen, bei dem man
a) vorab gereinigtes Propan in einem ersten Reaktionsschritt im Beisein und/oder unter Ausschluss von molekularem Sauerstoff wenigstens einer Dehydrierung aus der Gruppe umfassend die homogene Dehydrierung, die heterogen katalysierte Dehydrierung, die homogene Oxidehydrierung und die heterogen katalysierte Oxidehydrierung unterwirft, wobei ein nicht umgesetztes Propan und gebildetes Propylen enthaltendes Gasgemisch 1 erhalten wird, und
b) gegebenenfalls aus der Gesamtmenge oder aus einer Teilmenge des Gasgemischs 1, von den darin enthaltenen, von Propan und Propylen verschiedenen, Bestandteilen eine Teil- oder die Gesamtmenge abtrennt und/oder in andere Verbindungen wandelt, und dabei ein Propan und Propylen enthaltendes Gasgemisch 1' erzeugt, und in wenigstens einem weiteren Reaktionsschritt
c) Gasgemisch 1, oder Gasgemisch 1' oder ein Gemisch aus gebildetem Gasgemisch 1' und verbliebenem Gasgemisch 1 als Bestandteil eines Gasgemisches 2 einer heterogen katalysierten Gasphaseh-Partialoxidation und/oder partiellen Gasphasen-Ammoxidation von im Gasgemisch 1 und/oder Gasgemisch 1' enthaltenem Propylen unterwirft, wobei ein das wenigstens eine Zielprodukt P enthaltendes Gasgemisch 3 erhalten wird,
d) in wenigstens einem Abtrennschritt aus dem Gasgemisch 3 Zielprodukt abtrennt und von dem dabei verbleibenden Restgas wenigstens Propan in den ersten Reaktionsschritt zurückführt.

Acrylsäure ist als ein Partialoxidationsprodukt des Propylens ein bedeutendes Monomeres, das als solches oder in Form seiner Alkylester zur Erzeugung von z.B. als Klebstoffe geeigneten oder Wasser superabsorbierenden Polymerisaten Verwendung findet (vgl. z.B. WO 02/055469 und WO 03/078378). Ganz generell sind die partiellen Oxidations- und/oder Ammoxidationsprodukte des Propylens wichtige Zwischenprodukte zur Herstellung von Polymerisaten und den anderen Propylenpartialoxidations bzw. -ammoxidationsprodukten (z.B. Acrolein, Acrylnitril, Propylenoxid).

Die Herstellung von Acrylsäure und anderen Propylenpartialoxidationsprodukten nach einem eingangs beschriebenen Verfahren ist bekannt (vgl. z.B. DE-A 102 45 585 und DE-A 102 46 119).

Aus den vorgenannten Schriften ist ebenfalls bekannt, dass das Gasgemisch 2 in einem solchen Verfahren möglichst weitgehend von C₄-Kohlenwasserstoffen (verkürzt C₄-KW) frei sein sollte (darunter werden in dieser Schrift alle Verbindungen verstanden, die aus 4 Kohlenstoffatomen und Wasserstoff aufgebaut sind; dazu gehören n-Butan, iso-Butan, trans-Buten-2, cis-Buten-2, Buten-1, iso-Buten, Butadien 1,3, Butadien 1,2, 1-Butin und 2-Butin; in entsprechender Weise sind C₂-Kohlenwasserstoffe aus 2 Kohlenstoffatomen und Wasserstoff aufgebaute Verbindungen (verkürzt C₂-KW); dazu gehören insbesondere Ethan und Ethylen; von eher untergeordneter Bedeutung ist das Acetylen, da es im Unterscheid zu Ethan und Ethylen üblicherweise in eher vernachlässigbaren Mengen als Propanbegleiter auftritt), da diese Verbindungen die in der Partialoxidation erforderlichen Katalysatoren in der Regel vergiften. Als eine mögliche Abhilfemaßnahme wird dazu in beiden Schriften empfohlen, gegebenenfalls eine rektifikative Vorabtrennung von in Roh-Propan enthaltenen C₄-Kohlenwasserstoffe vorzunehmen.

Ferner ist aus den oben genannten Schriften bekannt, dass C₂-Kohlenwasserstoffe keine solchen Katalysatorgifte bilden, weshalb die DE-A 102 45 585 und die DE-A 102 46 119 keine analoge Spezifikation für C₂-Kohlenwasserstoffe im Gasgemisch 2 aufweisen. Vielmehr wird in den genannten Schriften davon ausgegangen, dass sich C₂-Kohlenwasserstoffe (insbesondere das Ethan, aber auch das Ethylen) bei der Partialoxidation als inerte Verdünnungsgase verhalten. Ein inertes Verhalten ist im Rahmen einer wie eingangs beschriebenen Kreisgasfahrweise des Propan jedoch insofern von Nachteil, als der C₃-KW Kreis in notwendiger Weise eines Auslasses (d.h. einer C₂-KW Abtrennung von C₃-KW) für solche inerten Bestandteile bedarf, da sich diese ansonsten im Rahmen der Kreisfahrweise unbegrenzt aufpegeln. Enthält das Roh-Propan neben C₄-KW gleichzeitig C₂-KW als Verunreinigungen (was in der Regel der Fall ist), würde man deshalb normalerweise wenigstens einen C₂-KW Auslass (insbesondere im Fall von Ethan und Ethylen als C₂-KW Verunreinigungen des Roh-Propan) mit einer rektifikativen C₄-KW Vorabtrennung von in Roh-Propan enthaltenen C₄-Kohlenwasserstoffen zusammenlegen, da der Roh-Propanstrom im Vergleich zu allen Gasströmen innerhalb der Kreisführung, sowohl was seine chemische Zusammensetzung als auch was sein Volumen anbetrifft, ein vergleichsweise einfach und wenig aufwendig zu handhabender Gasstrom ist.

Umgekehrt würde man auf eine solche C₂-Vorabtrennung dann möglichst weitgehend verzichten, wenn der beim relevanten Verfahren vom Propan zu durchlaufende Kreis in natürlicher Weise einen Auslass für C₂-KW aufweisen würde (ein solcher Auslass besteht in natürlicher Weise prima facie ausschließlich für das wenigstens eine Zielprodukt P). Dies nicht zuletzt deshalb, weil rektifikative Auftrennungen von C₂- bis C₄-KW enthaltenden Gemischen grundsätzlich unter erhöhtem Druck vorgenommen werden müssen. Andernfalls bedarf es besonders tiefer Temperaturen zur Erzeugung der innerhalb der Rektifikationskolonne erforderlichen Rücklaufflüssigkeit. Je mehr theoretischer Böden es in einer Rektifikationskolonne bedarf, desto größer wird aber der bei einer Druckkolonne zu leistende konstruktive Aufwand (z.B. allein aus Gründen einer sicheren Statik).

Erfindungsgemäß wurde nun in überraschender Weise gefunden, dass insbesondere die C₂-Kohlenwasserstoffe Ethan und Ethylen als Routinebegleiter von Propan in Roh-Propan unter den Bedingungen einer heterogen katalysierten Partialoxidation und/oder Ammoxidation von Propylen zu z.B. Acrolein, Acrylsäure, Propylenoxid und/oder Acrylnitril normalerweise lediglich in einem solchen Umfang inert sind, dass im Rahmen der Kreisgasfahrweise ein natürlicher Auslass von im Gasgemisch 2 enthaltenen C₂-KW gewährleistet ist. Sowohl Ethan als auch Ethylen werden unter den üblichen Bedingungen einer Propylenpartialoxidation und/oder Ammoxidation in ausreichender Weise zu Acetonitril, Acetaldehyd und/oder Essigsäure aufoxidiert, dass mit der Zielproduktabtrennung in der Regel in ausreichender Weise ein C₂-KW Auslass in Form von Acetonitril, Essigsäure und/oder Acetaldehyd (die dem Zielprodukt alle ähnlicher sind als die Vorläufer C₂-KW) einhergeht.

Demgemäss wird erfindungsgemäß ein Verfahren zur Herstellung wenigstens eines Zielproduktes P durch partielle Oxidation und/oder Ammoxidation von Propylen, bei dem man
a) vorab gereinigtes Propan in einem ersten Reaktionsschritt im Beisein und/oder unter Ausschluss von molekularem Sauerstoff wenigstens einer Dehydrierung aus der Gruppe umfassend die homogene Dehydrierung, die heterogen katalysierte Dehydrierung, die homogene Oxidehydrierung und die heterogen katalysierte Oxidehydrierung unterwirft, wobei ein nicht umgesetztes Propan und gebildetes Propylen enthaltendes Gasgemisch 1 erhalten wird, und
b) gegebenenfalls aus der Gesamtmenge oder aus einer Teilmenge des Gasgemischs 1, von den darin enthaltenen, von Propan und Propylen verschiedenen, Bestandteilen eine Teil- oder die Gesamtmenge abtrennt und/oder in andere Verbindungen wandelt, und dabei ein Propan und Propylen enthaltendes Gasgemisch 1' erzeugt, und in wenigstens einem weiteren Reaktionsschritt
c) Gasgemisch 1, oder Gasgemisch 1' oder ein Gemisch aus gebildetem Gasgemisch 1' und verbliebenem Gasgemisch 1 als Bestandteil eines Gasgemischs 2 einer heterogen katalysierten Gasphasen-Partialoxidation und/oder partiellen Gasphasen-Ammoxidatiön von im Gasgemisch 1 und/oder Gasgemisch 1' enthaltenem Propylen unterwirft, wobei ein das wenigstens eine Zielprodukt P enthaltendes Gasgemisch 3 erhalten wird,
d) in wenigstens einem Abtrennschritt aus dem Gasgemisch 3 Zielprodukt abtrennt und von dem dabei verbleibenden Restgas wenigstens Propan in den ersten Reaktionsschritt zurückführt, zur Verfügung gestellt, das dadurch gekennzeichnet ist,
   dass das vorab gereinigte Propan aus einem Roh-Propan, das
   ≥90 Gew.-% Propan,
   ≤ 99 Gew.-% Propan und Propylen (häufig ≤ 98 Gew.-%, oder ≤ 97 Gew.-%, oder ≤ 96 Gew.-%, oder ≤ 95 Gew.-%),
   ≥ 100 gew.ppm C₂-Kohlenwasserstoffe und
   ≥ 100 gew.ppm C₄-Kohlenwasserstoffe
   enthält, mit der Maßgabe erzeugt wird, dass man das Roh-Propan in eine (in der Regel trennwirksame Einbauten aufweisende) Rektifikationskolonne führt, und oberhalb der Zufuhrstelle das gereinigte Propan mit der Maßgabe entnimmt, dass der auf das enthaltene Propan bezogene Gehalt an C₂-Kohlenwasserstoffen in Gew.-% im gereinigten Propan nicht weniger als 80 % und nicht mehr als 110% des entsprechenden Gehaltes im Roh-Propan und der auf das enthaltene Propan bezogene Gehalt an C₄-Kohlenwasserstoffen in Gew.-% im gereinigten Propan höchstens 50 % des entsprechenden Gehaltes im Roh-Propan beträgt.

In der Regel wird das Roh-Propan beim erfindungsgemäßen Verfahren ≥ 200 gew.ppm C₂-KW, häufig ≥ 300 gew.ppm C₂-KW, vielfach ≥ 400 gew.ppm C₂-KW, oder ≥ 500 gew.ppm C₂-KW, oft ≥ 600 gew.ppm C₂-KW, oder ≥ 700 gew.ppm C₂-KW und gegebenenfalls ≥ 800 gew.ppm C₂-KW, oder ≥ 900 gew.ppm C₂-KW, oder ≥ 1000 gew.ppm C₂-KW enthalten.

Selbstverständlich kann das Roh-Propan beim erfindungsgemäßen Verfahren auch ≥ 1200 gew.ppm C₂-KW, oder ≥ 1400 gew.ppm C₂-KW, oder ≥ 1600 gew.ppm C₂-KW, oder ≥ 1800 gew.ppm C₂-KW, oder ≥ 2000 gew.ppm C₂-KW enthalten. Mögliche Gehalte an C₂-KW in erfindungsgemäß zu verwendendem Roh-Propan können auch ≥ 3000 gew.ppm, oder ≥ 5000 gew.ppm, bzw. ≥ 7000 gew.ppm, oder auch ≥ 10000 gew.ppm betragen. Definitionsgemäß liegt der Gehalt an C₂-KW in erfindungsgemäß zu verwendendem Roh-Propan in notwendiger Weise unterhalb von 10 Gew.-%, häufig bei Werten ≤ 8 Gew.-%, vielfach bei Werten ≤ 7 Gew.-%, oder ≤ 6 Gew.-%, oder ≤ 5 Gew.-%.

Im Normalfall entfallen wenigstens 90 Gew.-%, vielfach wenigstens 92 Gew.-%, oder wenigstens 94 Gew.-%, oder wenigstens 96 Gew.-%, oder wenigstens 98 Gew.-%, oder wenigstens 99 Gew.-% des Gehaltes an C₂-KW im Roh-Propan auf Ethan und Ethylen. Der Acetylengehalt liegt, bezogen auf die Gesamtmenge an im Roh-Propan enthaltenen C₂-KW, häufig bei Werten ≤ 1 Gew.-%, vielfach bei Werten ≤ 0.5 Gew.-%, und oft bei Werten ≤ 0,3 Gew.-%, oder ≤ 0,1 Gew.-%.

Häufig entfallen von der im Roh-Propan enthaltenen Gesamtmenge an C₂-KW wenigstens 50 Gew.-%, vielfach wenigstens 60 Gew.-%, häufig wenigstens 70 Gew.-%, oft wenigstens 80 Gew.-% und teilweise wenigstens 90 Gew.-% auf Ethan.

Der Ethylengehalt des Roh-Propan kann in vielen Fällen, bezogen auf die Gesamtmenge an enthaltenem KW, jedoch bis zu 50 Gew.-% betragen.

In der Regel wird das Roh-Propan beim erfindungsgemäßen Verfahren ≥ 200 gew.ppm C₄-KW, häufig ≥ 300 gew.ppm C₄-KW, vielfach ≥ 400 gew.ppm C₄-KW, oder ≥ 500 gew.ppm C₄-KW, oft ≥ 600 gew.ppm C₄-KW, oder ≥ 700 gew.ppm C₄-KW und gegebenenfalls ≥ 800 gew.ppm C₄-KW, oder ≥ 900 gew.ppm C₄-KW, oder ≥ 1000 gew.ppm C₄-KW enthalten.

Selbstverständlich kann das Roh-Propan beim erfindungsgemäßen Verfahren auch ≥ 1200 gew.ppm C₄-KW, oder ≥ 1400 gew.ppm C₄-KW, oder ≥ 1600 gew.ppm C₄-KW, oder ≥ 1800 gew.ppm C₄-KW, oder ≥ 2000 gew.ppm C₄-KW enthalten.

Mögliche Gehalte an C₄-KW in erfindungsgemäß zu verwendendem Roh-Propan können auch ≥ 3000 gew.ppm, oder ≥ 5000 gew.ppm , bzw. ≥ 7000 gew.ppm, oder auch ≥ 10000 gew.ppm betragen. Definitionsgemäß liegt der Gehalt an C₄-KW in erfindungsgemäß zu verwendendem Roh-Propan in notwendiger Weise unterhalb von 10 Gew.-%, häufig bei Werten ≤ 8 Gew.-%, vielfach bei Werten ≤ 7 Gew.-%, oder ≤ 6 Gew.-%, oder ≤ 5 Gew.-%

In zahlreichen Fällen entfallen wenigstens 80 Gew.-%, vielfach wenigstens 90 Gew.-%, oder wenigstens 92 Gew.-%, oder wenigstens 94 Gew.-%, oder wenigstens 96 Gew.-% des Gehaltes an C₄-KW im Roh-Propan auf Butan (n-Butan und iso-Butan). Von in Roh-Propan enthaltenem Butan entfallen in der Regel ≥ 50 Gew.-%, oft ≥ 60 Gew.-%, vielfach ≥ 70 Gew.-% auf iso-Butan. Der entsprechend bezogene n-Butan-Gehalt liegt meist bei ≥ 10 Gew.-%.

Der Gesamtgehalt an Butenen liegt, bezogen auf die Gesamtmenge an in Roh-Propan enthaltenem C₄-KW häufig bei Werten ≤ 1 Gew.-%, vielfach bei Werten ≤ 0,5 Gew.-%, und oft bei Werten ≤ 0,3 Gew.-%, oder ≤ 0,1 Gew.-%.

Normalerweise enthält das Roh-Propan jedoch ≥ 10 gew.ppm an Butenen.

Enthält das Roh-Propan auch fünf und mehr C-Atome aufweisende Kohlenwasserstoffe, so werden diese bei der erfindungsgemäßen rektifikativen Vorabreinigung gemeinsam mit den C₄-Kohlenwasserstoffen abgetrennt. Im Regelfall liegt der Gesamtgehalt an C_{≥5}-KW im Roh-Propan signifikant unterhalb des C₄-Gehaltes des Roh-Propan (normalerweise weniger als 50 Gew.-% des C4-KW-Gehaltes oder noch weniger). Das gleiche trifft auch auf im Roh-Propan gegebenenfalls enthaltenes Methan zu. Jedoch mit dem Unterschied, dass sein Schicksal im wesentlichen jenem der C₂-KW gleichen würde (d.h., der erfindungsgemäße C₂-KW-Auslass ist in der Regel in entsprechender Weise auch C₁-KW-Auslass).

Oft liegt der Gesamtgehalt an C_{≥5}-KW und Methan (aber auch die beiden individuellen Gehalte) im Roh-Propan bei Werten von ≤ 0,5 Gew.-%, bzw. ≤ 0,3 Gew.-%, bzw. ≤ 0,1 Gew.-%.

Erfindungsgemäß vorteilhaft wird das erfindungsgemäße Verfahren so durchgeführt, dass der auf das enthaltene Propan bezogene Gehalt an C₄-Kohlenwasserstoffen in Gew.-% im gereinigten Propan höchstens 40 %, bevorzugt höchstens 30 %, noch besser höchstens 20 %, besonders bevorzugt höchstens 10 % und ganz besonders bevorzugt höchstens 5 % bzw. höchstens 1 % des entsprechenden Gehaltes im Roh-Propan beträgt.

Ferner wird das erfindungsgemäße Verfahren mit Vorteil so durchgeführt, dass der auf das enthaltene Propan bezogene Gehalt an C₂-Kohlenwasserstoffen in Gew.-% im gereinigten Propan nicht weniger als 85 %, vorzugsweise nicht weniger als 90 %, besonders bevorzugt nicht weniger als 95 %, noch besser nicht weniger als 100 % und ganz besonders bevorzugt mehr als 100 % (in der Regel nicht mehr als 110 % und meist nicht mehr als 105 %) des entsprechenden Gehaltes im Roh-Propan beträgt. D.h., ganz besonders bevorzugt wird das erfindungsgemäße Verfahren so durchgeführt, dass das gereinigte Propan die C₂-Kohlenwasserstoffe, bezogen auf das darin enthaltene Propan, im Vergleich zum in gleicher Weise bezogenen Gehalt an C₂-Kohlenwasserstoffen im Roh-Propan, angereichert enthält. Erfindungsgemäße Grundlage für das Vorgenannte ist der bereits beschriebene natürliche Auslass für C₂-KW im Rahmen der erfindungsgemäßen Kreisfahrweise.

Erfindungsgemäß ist es weiterhin günstig, wenn die erfindungsgemäße rektifikative Vorabreinigung des Roh-Propan so durchgeführt wird, dass das der Rektifikationskolonne dabei entnommene vorab gereinigte Propan sowohl einen Gehalt an iso-Butan als auch an Gesamt-C₄-KW von ≤ 100 gew.ppm, oder ≤ 900 gew.ppm, oder ≤ 800 gew.ppm, oder ≤ 700 gew.ppm, oder vorzugsweise ≤ 600 gew.ppm, noch besser von ≤ 500 gew.ppm, bzw. ≤ 400 gew.ppm, oder ≤ 300 gew.ppm, und noch besser ≤ 200 gew.ppm aufweist. In vielen Fällen wird der Gehalt des erfindungsgemäß vorab gereinigten Propan an iso-Butan jedoch ≥ 100 gew.ppm betragen.

Innerhalb der für die erfindungsgemäße Vorabtrennung zu verwendenden Rektifikationskolonne werden absteigende Flüssigkeitsphase (Rücklaufflüssigkeit) und aufsteigende Dampfphase im Gegenstrom zueinander geführt. Infolge der zwischen den Stoffströmen bestehenden Temperatur- und Konzentrationsgradienten findet ein Wärme- und Stoffaustausch statt, der die gewünschte Stoffauftrennung bedingt. In der Regel befinden sich zur Erhöhung der Stoffaustauschfläche trennwirksame Einbauten in einer Rektifikationskolonne. Als solche Einbauten kommen für das erfindungsgemäße Verfahren grundsätzlich trennwirksame Einbauten jedweder Art in Betracht. Das können z.B. Packungen, Füllkörperschüttungen und/oder Stoffaustauschböden jedweder Art sein. Stoffaustauschböden, auf denen Gleichgewicht herrscht zwischen ablaufender Flüssigkeit und aufsteigendem Dampf werden als theoretische Böden bezeichnet. Dieser Begriff lässt sich auf alle anderen für Gegenstromrektifikationen geeigneten trennwirksamen Einbauten übertragen (z.B. Packungen und Füllkörperschüttungen). In dieser Schrift wird deshalb in zweckmäßiger Weise ganz allgemein von theoretischen Trennstufen gesprochen. Dabei wird als theoretische Trennstufe diejenige Raumeinheit definiert, die eine Anreicherung bzw. Abreicherung entsprechend dem thermodynamischen Gleichgewicht bewirkt.

Erfindungsgemäß bevorzugte trennwirksame Einbauten sind Stoffaustauschböden. Als solche kommen für das erfindungsgemäße Verfahren Siebböden (z.B. solche mit Flüssigkeitszwangsführung und solche ohne Flüssigkeitszwangsführung (z.B. alle in der DE-A 103 47 664 beschriebenen)) sowie besonders bevorzugt Ventilböden in Betracht. Unter Ventilböden sollen in dieser Schrift Querstromböden verstanden werden, die Bodenbohrungen mit hubbegrenzten Teller-, Ballast- oder Hebeventilen (Schwimmklappen) aufweisen, die die Größe der Dampfdurchtrittsöffnung der jeweiligen Kolonnenbelastung anpassen. Der aufsteigende Gasstrom wird abgelenkt, strömt parallel zum Boden in die gestaute Rücklaufflüssigkeit und bildet eine Sprudelschicht aus. Bewehrte Ablaufrohre führen den Rücklauf von Boden zu Boden. Häufig sind sie zweiflutig gestaltet. Sie können aber auch drei- und mehrflutig (z.B. vierflutig) gestaltet sein.

Die für die erfindungsgemäße Rektifikation erforderliche Wärmezufuhr erfolgt in zweckmäßiger Weise z.B. über innen- und/oder außerliegende Wärmeaustauscher herkömmlicher Bauart und/oder mittels Doppelwandheizung. Häufig werden außenliegende Umlaufverdampfer mit Natur- oder Zwangsumlauf eingesetzt.

Der Einsatz mehrerer in Reihe oder parallel geschalteter Verdampfer ist erfindungsgemäß möglich. Als Wärmeträger kann beispielsweise Wasserdampf verwendet werden, der im Rahmen der Wärmeabfuhr bei der Gasphasen-Partialoxidation normalerweise in natürlicher Weise anfällt.

In der Regel lässt sich die erfindungsgemäße Zielsetzung im Rahmen der erfindungsgemäßen Vorabreinigung des Roh-Propan in Anwendung nur einer Rektifikationskolonne erzielen, die wenigstens 5 theoretische Trennstufen, häufig wenigstens 8 theoretische Trennstufen, vielfach wenigstens 10 theoretische Trennstufen und oft wenigstens 14 theoretische Trennstufen aufweist. Normalerweise wird man jedoch nicht mehr als 25 theoretische Trennstufen, häufig nicht mehr als 23 theoretische Trennstufen und vielfach nicht mehr als 21 theoretische Trennstufen benötigen. In vielen Fällen wird die Anzahl der theoretische Trennstufen bei 15 bis 20, z.B. bei 18 liegen.

Aufgrund der vergleichsweise geringen Anzahl erforderlicher Trennstufen (enthält das Roh-Propan andere, von Propan verschiedene, C₃-KW als Bestandteile (z.B. Propylen), erfolgt innerhalb der C₃-KW im Rahmen der erfindungsgemäßen Vorabreinigung im wesentlichen keine Auftrennung; eine solche ist auch nicht erforderlich, da Propylen der angestrebte Reaktand im Gasgemisch 2 ist; Cyclopropan ist normalerweise allenfalls in vernachlässigbaren Mengen Bestandteil von Roh-Propan), kann das erfindungsgemäße Verfahren ohne übermäßigen konstruktiven Aufwand bei Kopfdrucken (in der Rektifikationskolonne) von ≥ 5 bar durchgeführt werden. Dz., erfindungsgemäße Kopfdrucke können ≥ 7 bar, oder ≥ 9 bar, oder ≥11 bar, oder ≥ 13 bar, oder ≥ 15 bar betragen. Im Normalfall wird der Kopfdruck jedoch bei Werten ≤ 25 bar, oder ≤ 23 bar, oder ≤ 21 bar liegen.

Aufgrund der vorgenannten Druckverhältnisse liegt die Temperatur im Kolonnensumpf beim erfindungsgemäßen Verfahren normalerweise bei Werten ≤ 100°C. Erfindungsgemäß günstige Sumpftemperaturen belaufen sich auf 40 bis 90°C, bevorzugt 50 bis 90°C und besonders bevorzugt 60 bis 80°C. Solchermaßen vergleichsweise niedere Sumpftemperaturen gewährleisten ein vergleichsweise geringes Ausmaß an Belagbildung bzw. Verkrustung (Fouling, Polymere) im Sumpfbereich.

Das vorab gereinigte Propan kann sowohl am Kopf der Rektifikationskolonne als auch über Seitenentnahme aus der Rektifikationskolonne entnommen werden. Häufig erfolgt diese Entnahme flüssig (z.B. über einen Kaminboden). Im Fall einer Seitenentnahme befinden sich oberhalb der Entnahmestelle üblicherweise höchstens noch bis zu zwei theoretische Trennstufen.

Aufgrund der beschriebenen Druckverhältnisse ist es beim erfindungsgemäßen Verfahren normalerweise ausreichend, den Kopfkondensator (der u.a. die Rücklaufflüssigkeit erzeugt) mit Wasser zu kühlen. Bei diesem handelt es sich erfindungsgemäß zweckmäßig entweder um einen indirekten Rohrbündelwärmeaustauscher oder um einen Plattenwärmeaustauscher, die der Rektifikationskolonne aufgesetzt oder in die Rektifikationskolonne integriert sein können. In typischer Weise betragen die Temperaturen des durch die Wärmeaustauscher geführten (dem Kopfkondensator zugeführten) Kühlwassers ≥ 0°C und ≤ 40°C. D.h., typische Kühlwassertemperaturen liegen bei ≥ 5°C und ≤ 35°C, oder ≥ 10°C und ≤ 30°C. Häufig wird man eine Kühlwassertemperatur von 20°C verwenden.

In der Regel wird der Kopfkondensator eine Entlüftung aufweisen, die es ermöglicht schwerst kondensierbare Bestandteile des Roh-Propan wie z.B. N₂, CO₂ etc. auszulassen.

Das Verhältnis aus der Menge (kg/h) von am Kolonnenkopf rückgeführter Rücklaufflüssigkeit zu der Menge von in die Rektifikationskolonne zugeführtem Roh-Propan wird bei der erfindungsgemäßen Vorabreinigung in typischer Weise 1 bis 2,5, oft 1,5 bis 2,5 und häufig 1,5 bis 2,0 betragen.

Erfindungsgemäß besonders vorteilhaft wird man die Bildung der Rücklaufflüssigkeit so vornehmen, dass eine Teilmenge der diesbezüglich zu kondensierenden Gasphase gasförmig verbleibt, so dass das für das erfindungsgemäße Verfahren im ersten Schritt benötigte vorab gereinigte Propan demselben unmittelbar gasförmig aus der Rektifikationskolonne entnommen zugeführt werden kann (gasförmige Kopf- bzw. Seitenentnahme).

Die Zufuhr des Roh-Propan in die Rektifikationskolonne wird man beim erfindungsgemäßen Verfahren anwendungstechnisch zweckmäßig in allen Fällen so durchführen, dass sich wenigstens eine theoretische Trennstufe oberhalb der Zufuhrstelle und wenigstens eine theoretische Trennstufe unterhalb der Zufuhrstelle befindet. Im Regelfall sollte die Anzahl der theoretischen Trennstufen oberhalb der Zufuhrstelle (Z_{O}) größer als die Anzahl der theoretischen Trennstufen unterhalb der Zufuhrstelle (Zu) sein.

Häufig wird das Verhältnis Zo zu Zu beim erfindungsgemäßen Verfahren 1,1 bis 2, häufig 1,1 bis 1,5 und vielfach 1,1, bis 1,3 betragen.

Der überwiegend aus C₄-KW (vor allem n-Butan und iso-Butan) bestehende Kolonnensumpf wird der Rektifikationskolonne kontinuierlich entnommen und anwendungstechnisch zweckmäßig einer stofflichen Weiterverwertung (z.B. Synthesegasherstellung, Partialoxidation, Verbrennung) zugeführt. In einfachster Weise kann diese stoffliche Weiterverwertung beispielsweise so aussehen, dass die Sumpfflüssigkeit als co-feed (zu den paraffinischen Kohlenwasserstoffen) einem Cracker (z.B. einem Steamcracker und/oder einem Raffineriecracker) zugeführt wird, in welchem durch thermische Spaltung von paraffinischen Kohlenwasserstoffen niedere gesättigte und ungesättigte Kohlenwasserstoffe (z.B. C₃- und C₄-KW) erzeugt und in sogenannten Splitterkolonnen rektifikativ aufgetrennt werden (vgl. z.B. US-A 3,392,216). Wird die Sumpfflüssigkeit in dieser Weise weiterverwertet, kann die erfindungsgemäße rektifikative Vorabtrennung auf besonders einfache Weise mit vergleichsweise geringer Schärfe dahingehend durchgeführt werden, dass die Sumpfflüssigkeit noch bis zu 30 Gew.-% C₃-KW enthält (normalerweise beträgt dieser C₃-KW Gehalt ≤ 20 bzw. ≤ 10 Gew.-%). Werden alternative stoffliche Weiterverwertungen angestrebt, wird man die Sumpfflüssigkeit in der Regel stärker an C₃-KW entreichern. Diese Entreicherung kann gegebenenfalls auch durch rektifikative Behandlung der Sumpfflüssigkeit in einer zweiten Rektifikationskolonne erfolgen, wie es z.B. in der DE-A 24 13 463 beschrieben ist. Dabei wird weiteres erfindungsgemäß verwendbares vorab gereinigtes Propan erhalten.

Bevorzugt wird die erfindungsgemäß einzusetzende Rektifikationskolonne einschließlich der bevorzugten trennwirksamen Stoffaustauschböden aus Edelstahl gefertigt. Zweckmäßig kann die Rektifikationskolonne mit Materialien wie z.B. Glas- oder Mineralwolle, Hartschaum, Kork oder Armaflex^{®} von außen wärmegedämmt sein.

Würden in der Rektifikationskolonne Packungen und/oder Füllkörperschüttungen als trennwirksame Einbauten verwendet, so können diese z.B. aus Ringen, Wendeln, Sattelkörpern, Raschig-, Intas- oder Pall-Ringen, Barrel- oder Intalox-Sättel, Top-Pak oder Geflechten bestehen. Natürlich können auch alle in dieser Schrift genannten möglichen Kolonneneinbauten in gemischter Form in der Rektifikationskolonne enthalten sein.

Der Druckverlust über die Rektifikationskolonne liegt normalerweise bei Werten ≤ 1 bar. Die Zufuhr des Roh-Propan in die Rektifikationskolonne erfolgt anwendungstechnisch zweckmäßig so, dass das Roh-Propan dabei zu wenigstens 95 Gew.-%, bevorzugt zu wenigstens 97 Gew.-%, oder zu wenigstens 99 Gew.-% flüssig vorliegt. Die Temperatur des flüssigen Roh-Propan kann dabei derjenigen Temperatur entsprechen, die an der Zulaufstelle innerhalb der Rektifikationskolonne besteht (die Temperierung kann beispielsweise durch Wärmeaustausch mit der Umgebungsluft erfolgen). Sie kann aber auch unterhalb dieser Temperatur liegen. Üblicherweise erfolgt die Zufuhr des Roh-Propan in die Rektifikationskolonne über eine Druckminderungsvorrichtung (z.B. eine Drossel).

In der Regel wird im Rahmen der erfindungsgemäßen Vorabreinigung erfindungsgemäß verwendbares vorab gereinigtes Propan erhalten, das normalerweise zu wenigstens 99 Gew.-%, vorzugsweise zu wenigstens 99,5 Gew.-%, besonders bevorzugt zu wenigstens 99,7 Gew.-%, ganz besonders bevorzugt zu wenigstens 99,9 Gew.-% und am besten zu wenigstens 99,95 Gew.-%, oder zu wenigstens 99,99 Gew.-% aus Propan, Propylen, Ethan und Ethylen besteht. In typischer Weise wird der C₂-KW Anteil ≤ 5 Gew.-%, häufig ≤ 3 Gew.-%, vielfach ≤ 2 Gew.-%, oft ≤ 1 Gew.-% und manchmal ≤ 0,5 Gew.-% betragen (meist wird er jedoch bei ≥ 0,1 Gew.-% liegen). In der Regel entfallen davon ≥ 50 Gew.-%, oft ≥ 60 Gew.-%, häufig ≥ 80 Gew.-%, oder ≥ 90 Gew.-%, oder ≥ 95 Gew.-%, oder ≥ 98 Gew.-%, oder ≥ 99 Gew.-% auf Ethan.

Dieses vorab gereinigte Propan kann nachfolgend anspruchsgemäß eingesetzt werden. Dies kann z.B. so wie in den Schriften DE-A 102 45 585, DE-A 102 46 119, WO 01/96270, US-A 3,161,670, DE-A 33 13 873, WO 01/96271, WO 03/011804, WO 03/076370, DE-A 103 16 039, DE-A 10 2004 032 129, EP-A 117 146, WO 04/031106, DE-A 103 16 039, DE-A 195 08 558, DE-A 198 37 520, DE-A 198 37 519, DE-A 198 37 517, WO 97/36849, EP-A 11 06 598, EP-A274 681, EP-A 731 077, der deutschen Anmeldung DE-A 10 2005 009 885, der deutschen Anmeldung DE-A 10 2005 009 891 und der DE-A 10 2004 003 212 beschrieben erfolgen.

Dabei wird unter Oxidehydrierung von Propan in dieser Schrift eine Dehydrierung verstanden, die durch anwesenden Sauerstoff erzwungen und bei der intermediär kein freier Wasserstoff gebildet wird bzw. nachweisbar ist. Im Unterschied zur konventionellen Dehydrierung, die endotherm verläuft, ist die Wärmetönung der Oxidehydrierung exotherm. Die Oxidehydrierung von Propan kann unter Einwirkung erhöhter Temperatur sowohl homogen (d.h., ohne Beisein eines z.B. festen Katalysators; vgl. z.B. US 3,798,283) als auch heterogen katalysiert (z.B. an festen Katalysatoren; vgl. z.B. DE-A 20 58 054 und DE-A 195 30 494) durchgeführt werden. Häufig laufen beide Reaktionen parallel ab. Das gleiche gilt im wesentlichen für die konventionelle Dehydrierung bei der der Dehydrierschritt ohne aktive Mitwirkung von Sauerstoff erfolgt (vgl. z.B. EP-A 731 077 und WO 01/96270). D.h., als primäres Nebenprodukt entsteht hier Wasserstoff und nicht Wasser wie im Fall der Oxidehydrierung. In einer sekundären Reaktion kann der gebildete molekulare Wasserstoff selbstredend teilweise oder vollständig verbrannt werden.

Unter einer vollständigen Oxidation von Propylen wird in dieser Schrift verstanden, dass der im Propylen insgesamt enthaltene Kohlenstoff in Oxide des Kohlenstoffs (CO, CO₂) umgewandelt wird. Alle davon verschiedenen Umsetzungen des Propylens unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die Ammoxidation. Bei geschickter Wahl des Ammoniakgehaltes verlaufen Partialoxidation und Ammoxidation parallel sich überlagernd (vgl. DE-A 102 45 585). Die in dieser Schrift bevorzugten partiellen Oxidations- und/oder Ammoxidationsprodukte des Propylens sind Acrolein, Acrylsäure, Propylenoxid und Acrylnitril.

Als Oxidationsmittel enthält das Gasgemisch 2 molekularen Sauerstoff, der z.B. in reiner Form oder im Gemisch mit sich bezüglich der Partialoxidation-/ammoxidation im wesentlichen inert verhaltenden Gasen (z.B. als Luft) im Gasgemisch 2 enthalten sein kann, Häufig sind die Reaktanden im Gasgemisch 2 auch aus Gründen der Wärmeabfuhr und aus Gründen der sicheren Reaktionsführung durch wenigstens ein Inertgas (z.B. N₂, H₂O, CO, CO₂, gesättigte z.B. C₁-C₅-Kohlenwasserstoffe (z.B. gemäß DE-A 19 24 431 und EP-A 293 224), He und/oder Ar etc.) verdünnt.

Alle Ausführungen in dieser Schrift sind insbesondere dann zutreffend, wenn die Partialoxidation des im Gasgemisch 2 enthaltenen Propylens die Partialoxidation von Propylen zu Acrolein und/oder Acrylsäure ist.

Dabei weist das Gasgemisch 2 mit Vorteil die nachfolgenden Gehalte auf:

| | |
|---|---|
| 6 bis 9 Vol.-% | Propylen, |
| 8 bis 18 Vol.-% | molekularer Sauerstoff, |
| 6 bis 35 Vol.-% | Propan und |
| 32 bis 72 Vol-% | molekularer Stickstoff. |

Das molare Verhältnis V₁ von im Gasgemisch 2 enthaltenem Propan zu im Gasgemisch 2 enthaltenem Propylen beträgt dabei erfindungsgemäß günstig 1 bis 4. Das molare Verhältnis V₂ von im Gasgemisch 2 enthaltenem molekularem Stickstoff zu im Gasgemisch 2 enthaltenem molekularem Sauerstoff beträgt dabei erfindungsgemäß zweckmäßig 2 bis 6. Das molare Verhältnis V₃ von im Gasgemisch 2 enthaltenem molekularem Sauerstoff zu im Gasgemisch 2 enthaltenem Propylen beträgt dabei erfindungsgemäß vorteilhaft 1,3 bis 2,4.

Weiterhin ist bekannt, dass es im Sinne einer Vermeidung von unerwünschter Vollverbrennung an Propylen bei der Partialoxidation und/oder-ammoxidation generell günstig ist, wenn der Propangehalt im Gasgemisch 2 ein vergleichsweise begrenzter ist. Erfindungsgemäß bevorzugt beträgt der Propangehalt im Gasgemisch 2 ≤ 60 Vol.-%, oder ≤ 50 Vol.-%. Besonders günstig sind Propangehalte im Gasgemisch 2 von 20 bis 40 Vol.-%, z.B. etwa 30 Vol.-%.

Lässt man einen für die Nitrilerzeugung gegebenenfalls mitzuverwendenden Ammoniakgehalt außer acht (d.h., berücksichtigt man ihn auch nicht in der Bezugsbasis für die Vol.-%), sind für das erfindungsgemäße Verfahren in der Regel solche Gasgemische 2 geeignet, die enthalten:

| | |
|---|---|
| 7 bis 15 Vol.-% | O₂, |
| 5 bis 10 Vol.-% | Propylen, |
| 15 bis 40 Vol.-% | Propan, häufig 25 bis 35 Vol.-%, |
| 25 bis 60 Vol-% | Stickstoff, häufig 40 bis 60 Von.-%, |
| 1 bis 5 Vol.-% | Summe aus CO, CO₂ und H₂O und |
| 0 bis 5 Vol.-% | sonstige Bestandteile (z.B. H₂). |

Im übrigen kann das erfindungsgemäße Verfahren wie die verschiedenen im Stand der Technik beschriebenen Grundvarianten durchgeführt werden (vgl. insbesondere die DE-A 102 45 585). D.h., in der einfachsten Variante werden alle Reaktionsschritte des erfindungsgemäßen Verfahrens in einer (einzigen) Reaktionszone und an einer in selbiger befindlichen Katalysatorbeschickung durchgeführt wie es z.B. die EP-A 608 838, die EP-A 529 853, die DE-A 198 35 248, die DE-A 101 45 958 und die DE-A 101 45 958 sowie die DE-A 102 45 585 und die in diesen Schriften zitierte Literatur am Beispiel der Herstellung von Acrolein und/oder Acrylsäure beschreiben.

Bei der zu verwendenden Aktivmasse der Katalysatorbeschickung kommen dazu vorzugsweise Multimetalloxidmassen in Betracht, die nachfolgende Elementkombination in der Stöchiometrie I,

Mo₁V_{b}M¹_{c}M²_{d} (I),

mit
M₁ = Te und/oder Sb,
M² = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,
b = 0,01 bis 1,
c = > 0 bis 1, und
d= > 0 bis 1
enthalten oder aus dieser Elementkombination in oxidischer Form bestehen.

Dies sind dann insbesondere Multimetalloxidaktivmassen der allgemeinen Stöchiometrie II

Mo₁V_{b}M¹_{c}M²_{d}Oₙ (II),

wobei die Variablen die bezüglich der Stöchiometrie l ausgeführt Bedeutung aufweisen und n = eine Zahl ist, die durch die Wertigkeit und Häufigkeit der von Sauerstoff verschiedenen Elemente in (II) bestimmt wird.

Erfindungsgemäß bevorzugt ist M¹ = Te und M² = Nb, Ta, W und/oder Ti.

Vorzugsweise ist M² = Nb. Der stöchiometrische Koeffizient b beträgt mit Vorteil 0,1 bis 0,6. In entsprechender Weise beläuft sich der Vorzugsbereich für den stöchiometrischen Koeffizienten c auf 0,01 bis 1 bzw. 0,05 bis 0,4 und günstige Werte für d betragen 0,01 bis 1 bzw. 0,1 bis 0,6.

Vorzugsweise weist die Multimetalloxidaktivmasse der Stöchiometrie II die in der DE-A 102 45 585 offenbarte Kristallstruktur und Oberflächenbeschaffenheit auf. Die beschriebenen Multimetalloxidaktivmassen können als solche (d.h. in Pulverform) oder zu geeigneten Geometrien geformt (vgl. z.B. die Schalenkatalysatoren der DE-A 100 51 419 sowie die geometrischen Varianten der DE-A 101 22 027) für die Einzonengestaltung des erfindungsgemäßen Verfahrens (ausgenommen den Abtrennschritt) eingesetzt werden. Sie eignen sich insbesondere zur Herstellung von Acrolein und/oder Acrylsäure sowie zur Herstellung von Acrylnitril. Basis für diese Einzonenfahrweise ist, dass die zu verwendenden Katalysatoren alle Reaktionsschritte des erfindungsgemäßen Verfahren zu katalysieren in der Lage sind.

Erfindungsgemäß wesentlich ist, dass bei der beschriebenen Einzonenfahrweise Ethan und Ethylen in erfindungsgemäßer Weise zu Acetonitril, Acetaldehyd und/oder Essigsäure partialoxidiert und/oder ammoxidiert werden.

Die beschriebene Einzonenfahrweise kann sowohl in einem Katalysatorfestbett als auch in einem Katalysatorwirbelbett bzw. Fließbett (Wanderbett) durchgeführt werden. Entsprechende Verfahrensbeschreibungen finden sich in den Schriften des Standes der Technik. Wird das erfindungsgemäße Verfahren z.B. zur Herstellung von Acrylsäure in der Einzonenfahrweise als Festbettreaktion ausgeführt, erfolgt die Durchführung in zweckmäßiger Weise in einem Rohrbündelreaktor, dessen Kontaktrohre mit dem Katalysator beschickt sind. Um die Kontaktrohre wird im Normalfall als Wärmeträger eine Flüssigkeit, in der Regel eine Salzschmelze geführt. Alternativ kann auch ein Thermoplattenreaktor eingesetzt werden, wobei sich die Katalysatorbeschickung als ebene Anordnung zwischen Kühlplatten befindet.

Das Reaktionsgasgemisch wird in den Kontaktrohren über den Reaktor betrachtet entweder im Gleichstrom oder im Gegenstrom zum Salzbad geführt. Das Salzbad selbst kann relativ zu den Kontaktrohre eine reine Parallelströmung ausführen. Selbstverständlich kann dieser aber auch eine Querströmung überlagert sein. Insgesamt kann das Salzbad um die Kontaktrohre auch eine mäanderförmige Strömung ausführen, die nur über den Reaktor betrachtet im Gleichstrom oder im Gegenstrom zum Reaktionsgasgemisch geführt ist. Für das erfindungsgemäße Verfahren geeignete Rohrbündelreaktoren offenbaren z.B. die Schriften EP-A 700 714 und EP-A 700 893.

Die verschiedenen möglichen Zusammensetzungen des Reaktionsgasausgangsgemisches für die Einzonenvariante des erfindungsgemäßen Verfahrens können dem im Zusammenhang mit dieser Verfahrensvariante zitierten Stand der Technik entnommen werden. Für die Herstellung von Acrylsäure bewegt sich die Zusammensetzung des Reaktionsgasausgangsgemisches in typischer Weise innerhalb des folgenden Rahmens (molare Verhältnisse):
Propan: Sauerstoff: H₂O sonstige Bestandteile (vor allem inerte Verdünnungsgase)= 1: (0,1-10): (>0-50): (>0-50).
Vorzugsweise beträgt das vorgenannte Verhältnis 1: (0,5-5) : (1-30): (1-30).

Die vorgenannten Bereiche gelten insbesondere dann, wenn als sonstige Bestandteile überwiegend molekularer Stickstoff eingesetzt wird. Die Reaktionstemperatur beträgt typisch 250 bis 550°C (die Bedingungen für die Ammoxidation sind vergleichbar, sieht man davon ab, dass das Reaktionsgasgemisch zusätzlich Ammoniak umfasst (vgl. z.B. EP-A 529 853).

Die Belastung einer Festbettkatalysatorbeschickung mit Propan kann bei der Einzonenvariante des erfindungsgemäßen Verfahrens z.B. 10 bis 500 Nl/l (Festbett)•h betragen. Die Belastung mit Reaktionsgasausgangsgemisch liegt häufig im Bereich von 100 bis 10000 Nl/l•h, vielfach im Bereich 500 bis 5000 Nl/l•h.

Die für die Einzonenfahrweise empfohlenen Multimetalloxidaktivmassen können selbstredend auch in mit feinteiligen, z.B. kolloidalen, Materialien wie Siliziumdioxid, Titandioxid, Aluminiumoxid, Zirkonoxid und Nioboxid verdünnter Form im erfindungsgemäßen Verfahren eingesetzt werden.

Das Verdünnungsmassenverhältnis kann dabei bis zu 9 (Verdünner) : 1 (Aktivmasse) betragen. D.h., mögliche Verdünnungsmassenverhältnisse betragen z.B. 6 (Verdünner) : 1 (Aktivmasse) und 3 (Verdünner) : 1 (Aktivmasse). Die Einarbeitung der Verdünner kann gemäß der DE-A 101 22 027 dabei vor oder nach der Calcination erfolgen. Selbstverständlich können für die erfindungsgemäße Einzonenfahrweise aber auch andere Katalysatorsysteme eingesetzt werden, wie sie z.B. die JP-A 3-170445 beschreibt. Wird das erfindungsgemäße Verfahren in einer Reaktionszone verwirklicht, liegt einer der Fälle vor, bei denen das Gasgemisch 1 und das Gasgemisch 2 identisch sind.

Erfindungsgemäß bevorzugt wird das erfindungsgemäße Verfahren in mehr als einer Reaktionszone verwirklicht, wie es z.B. die EP-A 938463, die EP-A 117146, die DE-A 3313573, die GB-A 2118939, die US-A 3161670, die WO 01/96270, die EP-A 731077, die DE-A 19837520, die DE-A 19837517, die DE-A 19837519, die DE-A 19837518, die DE-A 19837520, die DE-A 10139297 und die DE-A 10211275 beschreiben.

Mehr als eine Reaktionszone meint dabei in erster Linie, dass wenigstens ein Reaktionsschritt des erfindungsgemäßen Verfahrens unter Bedingungen durchgeführt wird, die wenigstens teilweise unabhängig von jenen des wenigstens einen anderen Reaktionsschrittes innerhalb des erfindungsgemäßen Verfahrens gewählt werden können, oder, allerdings nur in zweiter Linie, dass innerhalb ein und desselben Reaktionsschritts längs des Reaktionspfads wenigstens teilweise voneinander unabhängige Reaktionsbedingungen verwirklicht werden (letzteres ist z.B. dann der Fall, wenn für einen Reaktionsschritt sogenannte Mehrzonenfahrweisen (mit voneinander unabhängig einstellbaren Temperaturzonen) angewendet werden, wie es z.B. die DE-A 19948241, die DE-A 19927624, die DE-A 19910508, die DE-A 19910506 und die DE-A 19948248 beschreiben). D.h., umfasst das erfindungsgemäße Verfahren z.B. zwei Reaktionsschritte, so könnte für den ersten Reaktionsschritt z.B. ein anderer Katalysator bzw. eine andere Katalysatorbeschickung eingesetzt werden als für den zweiten Reaktionsschritt. Oder man könnte so vorgehen, daß bei Verwendung identischer Katalysatoren bzw. Katalysatorbeschickungen für beide Reaktionsschritte die Reaktionstemperaturen für die beiden Reaktionsschritte unabhängig voneinander gewählt und eingestellt werden können. Natürlich kann auch beides überlagert angewendet werden.

Der Vorteil der Mehrzonenfahrweise liegt darin begründet, dass sie prinzipiell eine verbesserte Anpassung der Reaktionsbedingungen an die Erfordernisse der einzelnen Reaktionsschritte des erfindungsgemäßen Verfahrens ermöglichen.

Dieser Vorteil ist von der heterogen katalysierten Gasphasen-Partialoxidation von Propylen zu Acrylsäure mit molekularem Sauerstoff wohlbekannt.

Sie verläuft längs der Reaktionskoordinate prinzipiell in zwei längs der Reaktionskoordinate aufeinanderfolgenden Schritten ab, von denen der erste zum Acrolein und der zweite vom Acrolein zur Acrylsäure führt.

Dieser Reaktionsablauf eröffnet in an sich bekannter Weise die Möglichkeit, die erfindungsgemäße Partialoxidation des im Gasgemisch 2 enthaltenen Propylen in zwei hintereinander angeordneten Oxidationszonen auszuführen, wobei in jeder der beiden Oxidationszon der zu verwendende oxidische Katalysator in optimierender Weise angepasst werden kann (diese Anpassungsmöglichkeit gestattet es auch, die Partialoxidation des Propylens auf der Höhe des Acroleins zu stoppen und das Acrolein zu isolieren). So wird für die erste Oxidationszone (Propylen → Acrolein) in der Regel ein Katalysator auf der Basis von die Elementkombination Mo-Bi-Fe enthaltenden Multimetalloxiden bevorzugt, während für die zweite Oxidationszone (Acrolein → Acrylsäure) normalerweise Katalysatoren auf der Basis von die Elementkombination Mo-V enthaltenden Multimetalloxiden bevorzugt werden (z.B. auch jene, die in dieser Schrift für die Einzonenfahrweise empfohlen wurden). Prinzipiell können diese beiden Reaktionsschritte aber auch in einer einzigen Reaktionszone und an einem einzigen Katalysator durchgeführt werden.

Ganz generell wird man beim erfindungsgemäßen Verfahren in zweckmäßiger Weise den ersten Reaktionsschritt in einer getrennten Reaktionszone durchführen.

Im Fall einer Oxidehydrierung des Propan kann diese als homogene und/oder heterogen katalysierte Oxidehydrierung von Propan zu Propylen mit molekularem Sauerstoff in der Gasphase durchgeführt werden. Als Quelle des molekularen Sauerstoff kann dabei Luft, reiner molekularer Sauerstoff oder an molekularem Sauerstoff angereicherte Luft eingesetzt werden (im Rahmen der Oxidehydrierung geht normalerweise eine Partialoxidation der C₂-KW einher; dies erleichtert einen entsprechenden Auslaß nach der Oxidehydrierung).

Gestaltet man die Reaktionszone als eine homogene Oxidehydrierung, so lässt sich diese prinzipiell so durchführen, wie es z.B. in den Schriften US-A 3,798,283 , CN-A 1 105 352, Applied Catalysis, 70(2)1991, S. 175-187, Catalysis Today 13, 1992, S. 673-678 und in der Anmeldung DE-A 196 22 331 beschrieben ist. Eine zweckmäßige Sauerstoffquelle ist Luft. Die Temperatur der homogenen Oxidehydrierung wird zweckmäßigerweise im Bereich von 300 bis 700°C, vorzugsweise im Bereich von 400 bis 600°C, besonders bevorzugt im Bereich von 400 bis 500°C liegend gewählt. Der Arbeitsdruck kann 0,5 bis 100 bar, insbesondere 1 bis 10 bar betragen. Die Verweilzeit liegt üblicherweise bei 0,1 beziehungsweise, 0,5 bis 20 Sekunden, vorzugsweise bei 0,1 beziehungsweise 0,5 bis 5 Sekunden.

Als Reaktor kann zum Beispiel ein Rohrofen oder ein Rohrbündelreaktor verwendet werden, wie zum Beispiel ein Gegenstrom-Rohrofen mit Rauchgas als Wärmeträger oder ein Rohrbündelreaktor mit Salzschmelze als Wärmeträger. Das Propan zu Sauerstoff-Verhältnis im Ausgangsgemisch beträgt vorzugsweise 0,5:1 bis 40:1, insbesondere zwischen 1:1 bis 6:1, stärker bevorzugt zwischen 2:1 bis 5:1. Das Ausgangsgemisch kann auch weitere, bevorzugt inerte (unter inerten Bestandteilen sollen in dieser Schrift ganz generell vorzugsweise solche Bestandteile verstanden werden, die sich beim relevanten Reaktionsschritt zu weniger als 5 mol.-%, bevorzugt zu weniger als 3 mol.-% und besonders bevorzugt zu weniger als 1 mol.-% umsetzen; ganz besonders bevorzugt setzten sie sich überhaupt nicht um), Bestandteile, wie Wasser, Kohlendioxid, Kohlenmonoxid, Stickstoff, Edelgas, andere Kohlenwasserstoffe (z.B. im Roh-Propan enthaltene Nebenbestandteile), und/oder Propylen etc. umfassen, wobei es sich hierbei auch um zurückgeführte (Kreisgas) Bestandteile handeln kann.

Gestaltet man die Propandehydrierung als eine heterogen katalysierte Oxidehydrierung, so lässt sich diese prinzipiell durchführen wie beschrieben zum Beispiel in den Schriften US 4,788,371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US 3,862,256, US 3,887,631, DE-A 195 30 454, US 4,341,664, J. of Catalysis 167, 560-569 (1997), J. of Catalysis 167, 550-559 (1997), Topics in Catalysis 3 (1996) 265-275, US 5,086,032, Catalysis Letters 10 (1991), 181-192, Ind. Eng. Chem. Res. 1996, 35, 14-18, US 4,255,284, Applied Catalysis A: General, 100 (1993), 111-130, J. of Catalysis 148, 56-67 (1994), V. Cortés Corberán und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 198 37 520, DE-A 198 37 517, DE-A 198 37 519 und DE-A 198 37 518. Dabei kann als Sauerstoffquelle auch Luft eingesetzt werden. Häufig weist jedoch die Sauerstoffquelle hier zu mindestens 90 mol.-% an molekularem Sauerstoff, und vielfach wenigstens 95 mol-% Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb=Oxide oder Vanadylpyrophosphat, gegebenenfalls mit Promotor, umfassen. Ein Beispiel für einen güns-tigen Oxidehydrierkatalysator ist ein Katalysator, wie er auch für die Einzonenfahrweise empfohlen wurde, der ein Mischmetalloxid mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel , Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weiterhin besonders geeignete Oxidehydrierungskatalysatoren sind die Multimetalloxidmassen beziehungsweise -katalysatoren A der DE-A-197 53 817 und die Katalysatoren der DE-A 19838312, wobei die in der erstgenannten Schrift als bevorzugt genannten Multimetalloxidmassen beziehungsweise -katalysatoren A ganz besonders günstig sind. Das heißt, als Aktivmassen kommen insbesondere Multimetalloxidmassen der allgemeinen Formel III

M¹ₐMo_{1-b}M²_{b}Oₓ (III),

wobei
M¹ = Co, Ni, Mg, Zn, Mn und/oder Cu,
M² = W, V, Te, Nb, P, Cr, Fe, Sb, Ce, Sn und/oder La,
a = 0,5-1,5
b = 0-0,5
sowie
x = eine Zahl, die durch die Wertigkeit und Häufigkeit der von Sauerstoff verschiedenen Elemente in (III) bestimmt wird,

in Betracht. Ihre Herstellung und Formgebung kann wie in der DE-A 102 45 585 beschrieben erfolgen.

Für die heterogen katalysierte Oxidehydrierung des Propans liegt die Reaktionstemperatur vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie zum Beispiel in der EP-A 700 893 und in der EP-A 700 714 sowie der in diesen Schriften zitierten Literatur beschrieben sind. Die mittlere Verweilzeit des Reaktionsgasgemisches in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators. Zweckmäßigerweise liegt es im Bereich von 0,5:1 bis 40:1, insbesondere von 1:1 bis 6:1, stärker bevorzugt von 2:1 bis 5:1. In der Regel nimmt die Propylenselektivität mit steigendem Propanumsatz ab. Vorzugsweise wird deshalb die Propan zu Propylen-Reaktion so durchgeführt, dass relativ niedrige Umsätze mit Propan bei höhen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan (Summe aus im vorab gereinigten Propan und gegebenenfalls rückgeführtem Kreisgas enthaltenem Propan das beim einfachen Durchgang umgesetzt wird). In der Regel beträgt die Selektivität der Propylenbildung 50 bis 98 mol-%, stärker bevorzugt 80 bis 98 mol-%, wobei der Begriff "Selektivität" die Mole an Propylen bezeichnet, die pro Mol umgesetztem Propan erzeugt werden, ausgedrückt als molarer Prozentsatz.

In der Regel enthält das bei der oxidativen Propandehydrierung eingesetzte Ausgangsgemisch 5 bis 95 mol-% Propan (bezogen auf 100 mol-% Ausgangsgemisch). Neben Propan und Sauerstoff kann das Ausgangsgemisch für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z.B. im Roh-Propan enthaltene Nebenbestandteile, und/oder Propylen umfassen. Die heterogene Oxidehydrierung kann auch in Anwesenheit von Verdünnungsmitteln, wie zum Beispiel Wasserdampf, durchgeführt werden.

Jede beliebige Reaktorsequenz kann zur Durchführung der homogenen Oxidehydrierung oder der heterogen katalysierten Oxidehydrierung des Propans eingesetzt werden, die dem Fachmann bekannt ist. Zum Beispiel kann die Oxidehydrierung in einem einzigen Reaktor oder in einer Kaskade aus zwei oder mehreren Reaktoren, zwischen denen gegebenenfalls Sauerstoff eingeführt wird, durchgeführt werden. Es besteht auch die Möglichkeit, die homogene und die heterogen katalysierte Oxidehydrierung miteinander kombiniert zu praktizieren.

Generell kann die Propandehydrierung in der ersten Reaktionszone auch als eine heterogen katalysierte Propandehydrierung unter weitgehendem Sauerstoffausschluss wie z.B. in der DE-A 3313573, in der WO 01/96270, der DE-A 10131297 oder der DE-A 10211275 beschrieben oder wie folgt durchgeführt werden.

Da die heterogen katalysierte Dehydrierreaktion unter Volumenzunahme abläuft, kann der Umsatz durch Erniedrigung des Partialdrucks der Produkte gesteigert werden. Dies lässt sich in einfacher Weise, zum Beispiel durch Dehydrierung bei vermindertem Druck und/oder durch Zumischen von im wesentlichen inerten Verdünnungsgasen, wie zum Beispiel Wasserdampf, erreichen, der für die Dehydrierreaktion im Normalfall ein Inertgas darstellt. Die Zugabe von Wasser kann in bevorzugter Weise insbesondere auch dazu dienen, diejenigen Teile des Reaktionsapparates, welche mit stark reduzierender Atmosphäre (vor allem bei hoher Temperatur) in Kontakt sind, vor korrosiver Beschädigung wie z.B. durch "Metal Dusting" zu schützen. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in der nachfolgenden wenigstens einen partiellen Oxidations- und/oder Ammoxidationszone (in dieser Schrift als Kurzbezeichnung auch wenigstens eine Partialzone) mitverwendet werden. Wasserdampf lässt sich aber auch in einfacher Weise teilweise oder vollständig aus dem Produktgemisch der Dehydrierung abtrennen (zum Beispiel durch Kondensieren), was die Möglichkeit eröffnet, bei der Weiterverwendung des dabei erhältlichen modifizierten Produktgemisches in der wenigstens eine Partialzone den Anteil des Verdünnungsgases N₂ zu erhöhen. Weitere für die heterogen katalysierte Propandehydrierung geeignete Verdünnungsmittel sind zum Beispiel CO, Methan, Ethan, CO₂, Stickstoff und Edelgase wie He, Ne und Ar. Alle genannten Verdünnungsmittel können entweder für sich oder in Form unterschiedlichster Gemische mitverwendet werden. Es ist von Vorteil, dass die genannten Verdünnungsmittel in der Regel auch in der wenigstens einen Partialzone geeignete Verdünnungsmittel sind. Generell sind, wie bereits gesagt, sich in der jeweiligen Reaktionszone inert verhaltende (das heißt zu weniger als 5 mol-%, bevorzugt zu weniger als 3 mol-% und noch besser zu weniger als 1 mol-% sich chemisch verändernde) Verdünnungsmittel bevorzugt. Prinzipiell kommen für die heterogen katalysierte Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen.

Unter anderem können damit alle Dehydrierkatalysatoren eingesetzt werden, die in der WO 01/96270, der EP-A 731077, der DE-A 10211275, der DE-A 101 31 297, der WO 99/46039, der US-A 4,788,371, der EP-A 705 136, der WO 99/29420, der US-A 4,220,091, der US-A 5,430,220, der US-A 5,877,369, der EP-A 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren sowie bezüglich des anspruchsgemäßen Einsatzes des vorab gereinigten Propan zitierten Schriften des Standes der Technik.

Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes, Study Number4192 OD, 1993, 430 Ferguson Drive, Mountain View, California, 94043-5272 U.S.A.".

Charakteristisch für die partielle heterogen katalysierte Dehydrierung von Propan ist, dass sie endotherm verläuft. Das heißt, die für die Einstellung der erforderlichen Reaktionstemperatur benötigte Wärme (Energie) muss entweder dem Reaktionsgasausgangsgemisch vorab und/oder im Verlauf der heterogen katalysierten Dehydrierung zugeführt werden.

Ferner ist es insbesondere für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberflache abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten.

Erfindungsgemäß kann es daher zweckmäßig sein, die Propandehydrierung (z.B. mit vergleichsweise geringem Propanumsatz) (quasi) adiabat durchzuführen. Das heißt, man wird das Reaktionsgasausgangsgemisch in der Regel zunächst auf eine Temperatur von 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch ein Katalysatorbett ausreichend sein, um den gewünschten Umsatz zu erzielen, wobei sich das Reaktionsgasgemisch um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere, Standzeiten des verwendeten Katalysatorbetts.

Prinzipiell ist die heterogen katalysierte Propandehydrierung mit vergleichsweise geringem Propanumsatz, ob adiabat oder isotherm gefahren, sowohl in einem Festbettreaktor als auch in einem Wanderbett- oder Wirbelbettreaktor durchführbar.

Bemerkenswerterweise ist beim erfindungsgemäßen Verfahren zu seiner Realisierung, insbesondere im adiabatischen Betrieb, ein einzelner Schachtofenreaktor als Festbettreaktor ausreichend, der vom Reaktionsgasgemisch axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb wärmeisoliert ist, wird dabei mit dem heißen, Propan enthaltenden, Reaktionsgas axial durchströmt. Die Katalysatorgeometrie kann dabei sowohl kugelförmig als auch ringförmig oder strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgases kann der Reaktor zum Beispiel aus zwei in einer Mantelhülle befindlichen, konzentrisch ineinander gestellten, zylindrischen Gitterrosten bestehen und die Katalysatorschüttung in deren Ringspalt angeordnet sein. Im adiabaten Fall wäre die Mantelhülle gegebenenfalls wiederum thermisch isoliert.

Als Katalysatorbeschickung für eine heterogen katalysierte Propandehydrierung mit vergleichsweise geringem Propanumsatz bei einmaligem Durchgang eignen sich insbesondere die in der DE-A 199 37 107 offenbarten, vor allem alle beispielhaft offenbarten, Katalysatoren.

Nach längerer Betriebsdauer sind vorgenannte Katalysatoren zum Beispiel in einfacher Weise dadurch regenerierbar, dass man bei einer Eintrittstemperatur von 300 bis 600°C, häufig bei 400 bis 550°C, zunächst in ersten Regenerierungsstufen mit Stickstoff und/oder Wasserdampf (bevorzugt) verdünnte Luft über das Katalysatorbett leitet. Die Katalysatorbelastung mit Regeneriergas kann dabei z.B. 50 bis 10000 h⁻¹ und der Sauerstoffgehalt des Regeneriergases 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel N₂) zu spülen.

Anschließend ist es in der Regel empfehlenswert, noch mit reinem molekularem Wasserstoff oder mit durch Inertgas (vorzugsweise Wasserdampf) verdünntem molekularem Wasserstoff (der Wasserstoffgehalt sollte ≥ 1 Vol.-% betragen) im ansonsten gleichen Bedingungsraster zu regenerieren.

Die heterogen katalysierte Propandehydrierung kann mit vergleichsweise geringem Propanumsatz (≤ 30 mol-%) in allen Fällen bei den gleichen Katalysatorbelastungen (sowohl das Reaktionsgas insgesamt, als auch das in selbigem enthaltende Propan betreffend) betrieben werden wie bei hohem Propanumsatz (> 30 mol-%). Diese Belastung mit Reaktionsgas kann zum Beispiel 100 bis 10000 h-¹, häufig 300 bis 5000 h⁻¹, das heißt vielfach etwa 500 bis 3000 h⁻¹ betragen.

In besonders eleganter Weise lässt sich die heterogen katalysierte Propandehydrierung (vor allem bei geringem Propanumsatz) in einem Hordenreaktor verwirklichen.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten über mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für Propanumsätze ≤ 30 mol.-% insbesondere bei Verwendung der in der DE-A 199 37107 beschriebenen Katalysatoren, insbesondere der beispielhaften Ausführungsformen, ausreichend, das Reaktionsgasgemisch auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist.

Noch geschickter ist es, die katalytische Dehydrierung autotherm, d.h., z.B. die vorstehend geschilderte Zwischenerhitzung auf direktem Weg durchzuführen (autotherme Fahrweise). Dazu wird dem Reaktionsgasgemisch entweder bereits vor Durchströmung des ersten Katalysatorbettes und/oder zwischen den nachfolgenden Katalysatorbetten in begrenztem Umfang molekularer Sauerstoff zugesetzt. Je nach verwendetem Dehydrierkatalysator wird so eine begrenzte Verbrennung der im Reaktionsgasgemisch enthaltenen Kohlenwasserstoffe (damit geht regelmäßig auch eine Partialoxidation von C₂-KW einher), gegebenenfalls bereits auf der Katalysatoroberfläche abgeschiedener Kohle beziehungsweise kohleähnlicher Verbindungen und/oder von im Verlauf der heterogen katalysierten Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht.

In der Regel sollte eine wie vorstehend beschriebene Sauerstoffeinspeisung so vorgenommen werden, dass der Sauerstoffgehalt des Reaktionsgasgemisches, bezogen auf die darin enthaltene Menge an Propan und Propylen, 0,5 bis 30 Vol.-% betragt. Als Sauerstoffquelle kommen dabei sowohl reiner molekularer Sauerstoff oder mit Inertgas, zum Beispiel CO, CO₂, N₂, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Reaktionsgasausgangsgemisch für die heterogen katalysierte Propandehydrierung auf die benötigte Reaktionstemperatur zu erwärmen, besteht auch darin, einen Teil des darin enthaltenen Propans und/oder H₂ mittels molekularem Sauerstoff zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die gewünschte Reaktionstemperatur zu bewirken (dabei geht in der Regel ebenfalls eine Partialoxidation von C₂-KW einher). Die resultierenden Verbrennungsprodukte, wie CO₂, H₂O sowie das den für die Verbrennung benötigten molekularen Sauerstoff gegebenenfalls begleitende N₂ bilden vorteilhaft inerte Verdünnungsgase.

Besonders elegant lässt sich die vorgenannte Wasserstoffverbrennung wie in der DE-A 102 11 275 beschrieben realisieren. D.h., in einem Verfahren der kontinuierlichen heterogen katalysierten partiellen Dehydrierung von Propan in der Gasphase, bei dem
- einer Reaktionszone ein das zu dehydrierende Propan enthaltendes Reaktionsgas kontinuierlich zugeführt wird,
- das Reaktionsgas in der Reaktionszone über wenigstens ein Katalysatorfestbett geführt wird, an welchem durch katalytische Dehydrierung molekularer Wasserstoff und wenigstens partiell Propylen gebildet werden,
- dem Reaktionsgas vor und/oder nach Eintritt in die Reaktionszone wenigstens ein molekularen Sauerstoff enthaltendes Gas zugesetzt wird,
- der molekulare Sauerstoff in der Reaktionszone im Reaktionsgas enthaltenen molekularen Wasserstoff teilweise zu Wasserdampf oxidiert und
- der Reaktionszone ein Produktgas entnommen wird, das molekularen Wasserstoff, Wasserdampf, Propylen und Propan enthält,
das dadurch gekennzeichnet ist, dass das der Reaktionszone entnommene Produktgas in zwei Teilmengen identischer Zusammensetzung aufgeteilt und eine der beiden Teilmengen als Kreisgas in die Dehydrierreaktionszone zurückgeführt und die andere Teilmenge erfindungsgemäß als Gasgemisch 1 weiterverwendet wird.

Prinzipiell kann in einem Hordenreaktor auch eine Kombination aus heterogen katalysierter oxidativer und klassischer Dehydrierung durchgeführt werden. Beispielsweise kann jedes zweite Katalysatorbett mit einem Oxidehydrierkatalysator und die anderen Katalysatorbetten mit einem klassischen Dehydrierkatalysator belegt sein. Zwischen den Betten erfolgt Sauerstoffzwischeneinspeisung. Die exotherme Oxidehydrierung kann dann alternativ zu einer Wasserstoffverbrennung genutzt werden, um das Reaktionsgasgemisch aufzuheizen.

Den Schriften EP-A 117 146, DE-A 33 13 573 und US 3,161,670 folgend, kann das Gasgemisch 1 als solches zur Erzeugung des Gasgemisches 2 verwendet werden. Bei Bedarf können aber auch aus dem Gasgemisch 1 vor dessen Verwendung zur Erzeugung des Gasgemisches 2 auch von Propan und Propylen verschiedene Bestandteile teilweise abgetrennt werden. Letzteres kann z.B. dadurch erfolgen, dass man das Gasgemisch 1, gegebenenfalls nachdem man es zuvor in einem indirekten Wärmetauscher abgekühlt hat, über eine, in der Regel zu einem Rohr gestaltete, Membran leitet, die lediglich für molekularen Wasserstoff durchlässig ist. Der so abgetrennte molekulare Wasserstoff kann bei Bedarf teilweise in die heterogen katalysierte Dehydrierung von Propan rückgeführt oder einer sonstigen Verwertung zugeführt werden. Auch kann eine Teil- oder die Gesamtmenge von im Gasgemisch 1 enthaltenem Wasserdampf vorab der Verwendung des Gasgemisches 1 zur Erzeugung des Gasgemisches 2 aus dem Gasgemisch 1 abgetrennt werden.

Alternativ kann man das vorzugsweise abgekühlte (vorzugsweise auf Temperaturen von 10 bis 70°C) Gasgemisch 1, z.B. bei einem Druck von 0,1 bis 50 atm und einer Temperatur von 0 bis 100°C, mit einem (vorzugsweise hochsiedenden) organischen Lösungsmittel (vorzugsweise ein hydrophobes), in welchem Propan und Propen bevorzugt absorbiert werden, in Kontakt bringen (z.B. durch einfaches Durchleitet). Durch nachfolgende Desorption, Rektifikation und/oder Strippung mit einem bezüglich der heterogen katalysierten Gasphasen-Partialoxidation und/oder Ammoxidation sich inert verhaltenden und/oder in dieser Reaktionszone als Reaktand benötigten Gas (z.B. Luft), werden das Propan und Propen im Gemisch in gereinigter Form rückgewonnen und zur Erzeugung des Gasgemischs 2 verwendet (im Fall der Strippung mit Luft kann das dabei erzeugte Gasgemisch 1' mit dem Gasgemisch 2 identisch sein, d.h., als solches unmittelbar zur Beschickung der heterogen katalysierten Gasphasen-Partialoxidation und/oder Ammoxidation verwendet werden). Als Alternative für den beschriebenen Trennschritt via Absorption kommen auch eine Druckwechseladsorption oder eine Druckrektifikation in Betracht. Zwar kann mit vorgenannten Trennschritten stets auch ein C₂-Auslaß verknüpft sein. Dieser kann jedoch aufgrund des erfindungsgemäßen C₂-Reaktivauslasses wesentlich einfacher gehalten werden.

Als Absorptionsmittel für die vorstehend beschriebene absorptive Abtrennung eignen sich grundsätzlich alle Absorptionsmittel, die in der Lage sind, Propan und Propen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von1 atm) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propen hat. Beispielhaft als Absorptionsmittel genannt seien aliphatische Kohlenwasserstoffe, zum Beispiel C₈-C₂₀-Alkane oder -Alkene, oder aromatische Kohlenwasserstoffe, zum Beispiel Mittelölfraktionen aus der Paraffndestillation oder Ether mit sperrigen (sterisch anspruchsvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyldiphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomere. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl^{®} (z.B von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane , Dodecane, Tridecane , Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in DE-A 33 13 573 beschriebenen Paraffinöle mit 8 bis 6 Kohlenstoffatomen. Beispiele für geeignete Handelsprodukte sind von der Firma Haltermann vertriebene Produkte wie Halpasole i, wie Halpasol 250/340 i und Halpasbl 250/275 i, sowie Druckfarbenöle unter den Bezeichnungen PKWF und Printosol. Bevorzugt sind an Aromaten freie Handelsprodukte, z.B. solche des Typs PKWFaf. Weitere geeignete Handelsprodukte sind n-Paraffin (C₁₃-C₁₇) oder Mihagol^{®}5 der Erdöl-Raffinerie - Emsland GmbH, LIN-PAR^{®}14-17 der Firma CONDEA Augusta S.p.A. (Italien) oder SASOL Italy S.p.A., Normal paraffins (heavy) C₁₄-C₁₈ der Firma SLOVNAFT in der Slowakei.

Die Gehalte (angegeben in Flächenprozenten der gaschromatographischen Analyse) der vorgenannten Produkte an linearen Kohlenwasserstoffe betragen in typischer Weise:
Summe C₉ bis C₁₃: weniger als 1%; C₁₄: 30 bis 40%; C₁₅: 20 bis 33%; C₁₆: 18 bis 26%; C₁₇: bis zu 18%; C_{≥18}: <2%.

Eine typische Zusammensetzung des Produktes der Fa. SASOL ist:
C₁₃: 0,48%; C₁₄: 39,8%; C₁₅ : 20,8%; C₁₆: 18,9%; C₁₇: 17,3%, C₁₈: 0,91%; C₁₉: 0,21%.

Eine typische Zusammensetzung des Produkts der Fa. Haltermann ist:
C₁₃: 0,58%; C₁₄: 33,4%; C₁₅: 32,8%; C₁₆: 25,5%, C₁₇: 6,8%; C_{≥18}: <0,2%.

Die Durchführung der Absorption unterliegt keinen besonderen Beschränkungen. Es können alle dem Fachmann gängigen Verfahren und Bedingungen eingesetzt werden, wie sie im in dieser Schrift zitierten Stand der Technik schon beschrieben sind. Vorzugsweise wird das Gasgemisch bei einem Druck von 1 bis 50 bar, bevorzugt 2 bis 20 bar, stärker bevorzugt 5 bis 10 bar, und einer Temperatur von 0 bis 100°C, insbesondere von 30 bis 50°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m²/m³, zum Beispiel Mellapak^{®} 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propens von dem Absorptionsmittel kann durch Strippung, Entspannungsverdampfung (Flashen) und/oder Destillation erfolgen.

Die Abtrennung des Propans und Propens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200°C, insbesondere 20 bis 100C, stärker bevorzugt 30 bis 70°C, besonders bevorzugt 40 bis 60°C. Ein für die Strippung geeignetes Gas ist beispielsweise Wasserdampf, bevorzugt sind jedoch insbesondere Sauerstoff/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor oder während des Strippprozesses ein Gas zuzusetzen, das den Explosionsbereich reduziert. Besonders geeignet dafür ist vorab gereinigtes Propan. Besonders geeignet für die Strippung sind auch Blasensäulen mit und ohne Einbauten.

Die Abtrennung des Propans und Propens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300°C, Insbesondere 150 bis 250°C.

Ein durch Strippen aus dem Absorptionsmittel gewonnenes Gasgemisch 1' kann vor seiner Verwendung zur Beschickung der wenigstens einen Partialzone noch einer weiteren Verfahrerisstufe zugeführt werden, um z.B. die Verluste an mitgestripptem Absorptionsmittel zu reduzieren (z.B. Abscheidung in Demistern und/oder Tiefenfiltern) und die wenigstens eine Partialzone so gleichzeitig vor Absorptionsmittel zu schützen oder um die Trennwirkung zwischen den C₃-Kohlenwasserstoffen und den sonstigen Bestandteilen zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z.B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen. Diese Wäsche beziehungsweise das Quenchen kann z.B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergrößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden heterogen katalysierten Gasphasen-Partialoxidation und/oder Ammoxidation normalerweise nicht stört. Nachdem das Wasser das Absorptionsmittel aus dem mit Propan und Propen beladenen Ausgangstrom herausgewaschen hat, kann das Wasser/Absorptionsmittel-Gemisch einer Phasentrennung zugeführt und der behandelte Ausgangstrom in günstigen Fällen als ein Gasgemisch 1' = Gasgemisch 2 der heterogen katalysierten Gasphasen-Partialoxidation und/oder Ammoxidation zugeführt werden.

Sowohl das C₃-frei gestrippte Absorptionsmittel als auch das in der Phasentrennung rückgewonnene Absorptionsmittel können für den Absorptionszweck wiederverwendet werden.

Generell kann das Gasgemisch 1 und/oder das aus selbigem erzeugte Gasgemisch 1' in an sich bekannter Weise in wenigstens einer weiteren Reaktionszone zur Beschickung einer heterogen katalysierten Gasphasenoxidation und/oder -ammoxidation von Propen zu Acrolein und/oder Acrylsäure und/oder Acrylnitril mit einem Beschickungs-Gasgemisch 2 eingesetzt werden. Als Oxidationsmittel kann dabei bei Bedarf dem Gasgemisch 1 und/oder dem Gasgemisch 1' reiner molekularer Sauerstoff, Luft, mit Sauerstoff angereicherte Luft oder jedes sonstige Gemisch aus Sauerstoff und Inertgas zugesetzt werden. Handelt es sich bei der Partialoxidation um die Umsetzung von Propylen zu Propylenoxid, kann z.B. wie in der EP-A 372 972 beschrieben verfahren werden.

Handelt es sich um eine partielle Ammoxidation zu Acrylnitril, kann z.B. gemäß der DE-A 2351151 verfahren werden. Im Fall einer partiellen Oxidation des Propylens zu Acrolein und/oder Acrylsäure wird man beim erfindungsgemäßen Verfahren die Zusammensetzung des Gasgemisches 2 unter Mitverwendung des Gasgemisches 1 und/oder 1' (es können auch Gemische beider verwendet werden; d.h. aus einem Teil wird abgetrennt aus einem andern nicht) z.B. so einstellen, dass es die nachfolgenden molaren Verhältnisse erfüllt:
Propan : Propen : N₂ : Oz : H₂O : sonstige = 0,5 bis 20 : 1 : 0,1 bis 40 : 0,1 bis 10 : 0 bis 20 : 0 bis 1.

Mit Vorteil betragen die vorgenannten molaren Verhältnisse erfindungsgemäß = 2 bis 10 : 1 : 0,5 bis 20 : 0,5 bis 5 : 0,01 bis 10 : 0 bis 1.

Günstig ist es auch, wenn die vorgenannten molaren Verhältnisse erfindungsgemäß = 3 bis 6 : 1 : bis 10 : 1 bis 3 : 0,1 bis 2 : 0 bis 0,5 betragen.

Erfindungsgemäß günstig sind Gasgemische 2, die > 0 bis 30 Vol.-%, oft ≥ 0,5 bis 25 Vol.-%, vielfach ≥ 1 bis 20 Vol.-%, häufig ≥ 3 bis 15 Vol.-%, zweckmäßig ≥ 5 bis 10 Vol.-% an Wasserdampf enthalten, da solche Wasserdampfgehalte den erfindungsgemäß zugrundeliegenden C₂-Auslaß begünstigen. Dies vor allem dann, wenn die nachfolgend empfohlenen Katalysatorsysteme für die heterogen katalysierte partielle Gasphasenoxidation und/oder Ammoxidation eingesetzt werden (insbesondere im Fall einer Propylenpartialoxidation zu Acrolein und/oder Acrylsäure).

Insbesondere in Kombination mit dem vorgenannten Sachverhalt (grundsätzlich aber ganz allgemein) betrifft die vorliegende Erfindung eine vorliegende Ausgestaltung des erfindungsgemäßen Verfahrens, bei dem man das für das erfindungsgemäße Verfahren benötigte vorab gereinigte Propan unmittelbar dem ersten Reaktionsschritt des erfindungsgemäßen Verfahrens zuführt.

Die vorliegende Erfindung betrifft aber auch vorteilhafte Ausgestaltungen des erfindungsgemäßen Verfahrens, bei dem man das für das Verfahren benötigte vorab gereinigte Propan höchstens teilweise (z.B. nur zu 75%, oder nur zu 50%, oder nur zu 25%) dem Reaktionsgasausgangsgemisch für den ersten Reaktionsschritt und wenigstens teilweise (in der Regel die Restmenge, gegebenenfalls die Gesamtmenge) unmittelbar der heterogen katalysierten Gasphasen-Partialoxidation und/oder Ammoxidation (z.B. dem Gasgemisch 2) zuführt.

Umfasst die heterogen katalysierte Gasphasen-Partialoxidation und/oder Ammoxidation mehrere Reaktionsschritte, so kann in jeden dieser Reaktionsschritte eine unmittelbare Zufuhr (einer Teil- oder der Gesamtmenge des vorab gereinigten Propan) von vorab gereinigtem Propan erfolgen.

Eine unmittelbare Zufuhr von vorab gereinigtem Propan (z.B. wenigstens 25 Gew.-%, oder wenigstens 50 Gew.-%, oder wenigstens 75 Gew.-%, oder 100 Gew.-% des Gesamtbedarfs des erfindungsgemäßen Verfahrens an vorab gereinigtem Propan) in wenigstens einen Reaktionsschritt der heterogen katalysierten GasphasenPartialoxidation und/oder Ammoxidation ist besonders geschickt, da daraus erfindungsgemäß automatisch eine Entlastung des ersten Reaktionsschritts von C₂-KW einhergeht. Auch mindert sie in der Regel ein Explosionsrisiko.

Wie bereits erwähnt läuft die heterogen katalysierte Gasphasen-Partialoxidation von Propylen zu Acrylsäure mit molekularem Sauerstoff grundsätzlich in zwei längs der Reaktionskoordinate aufeinanderfolgenden Reaktionsschritten ab, von denen der erste zum Acrolein und der zweite von Acrolein zu Acrylsäure führt.

Dieser Reaktionsablauf in zwei zeitlich aufeinanderfolgenden Schritten eröffnet in an sich bekannter Weise die Möglichkeit, das erfindungsgemäße Verfahren auf der Stufe des Acrolein (der Stufe überwiegender Acroleinbildung) abzubrechen und die Zielproduktabtrennung auf dieser Stufe vorzunehmen, oder das erfindungsgemäße Verfahren bis zur überwiegenden Acrylsäurebildung fortzuführen und die Zielproduktabtrennung erst dann vorzunehmen.

Führt man das erfindungsgemäße Verfahren bis zur überwiegenden Acrylsäurebildung, ist es erfindungsgemäß vorteilhaft, das Verfahren zweistufig, d. h., in zwei hintereinander angeordneten Oxidationsstufen auszuführen, wobei zweckmäßigerweise in jeder der beiden Oxidationsstufen das zu verwendende Katalysatorfestbett und vorzugsweise auch die sonstigen Reaktionsbedingungen, wie z. B. die Temperatur des Katalysatorfestbetts, in Bezug auf die vorliegende Erfindung in optimierender Weise angepasst werden.

Zwar vermögen die für die Katalysatoren der ersten Oxidationsstufe (Propylen -> Acrolein) als Aktivmässen erfindungsgemäß besonders geeigneten, die Elemente Mo, Fe, Bi enthaltenden Multimetalloxide, in gewissem Umfang auch die zweite Oxidationsstufe (Acrolein -> Acrylsäure) zu katalysieren, doch werden für die zweite Oxidationsstufe erfindungsgemäß Katalysatoren bevorzugt, deren Aktivmasse wenigstens ein die Elemente Mo und V enthaltendes Multimetalloxid ist. Die vorgenannten und alle nachfolgend als erfindungsgemäß günstig beschriebenen Multimetalloxidaktivmassen zeichnen sich dadurch aus, dass sie den erfindungsgemäß relevanten C₂-Auslaß in besonders ausgeprägter Weise ermöglichen.

Damit eignet sich das Verfahren der heterogen katalysierten Partialoxidation von Propylen an Katalysatorbetten (z.B. Wirbelbetten oder Festbetten), deren Katalysatoren als Aktivmasse wenigstens ein die Elemente Mo, Fe und Bi-enthaltendes Multimetalloxid aufweisen, insbesondere als erfindungsgemäßes einstufiges Verfahren zur Herstellung von Acrolein (sowie gegebenenfalls Acrylsäure) oder als erste Reaktionsstufe zur erfindungsgemäßen zweistufigen Herstellung von Acrylsäure.

Die Realisierung der einstufigen heterogen katalysierten Partialoxidation von Propylen zu Acrolein sowie gegebenenfalls Acrylsäure bzw. der zweistufigen heterogen katalysierten Partialoxidation von Propylen zu Acrylsäure unter erfindungsgemäßer Verwendung des Gasgemischs 2 kann dabei im einzelnen wie in den Schriften EP-A 700 714 (erste Reaktionsstufe; wie dort beschrieben, aber auch in entsprechender Gegenstromfahrweise von Salzbad und Reaktionsgasausgangsgemisch über den Rohrbündelreaktor), EP-A 700 893 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/85363, DE-A 103 13 212 (erste Reaktionsstufe), EP-A 11 59 248 (als zweistufiges Verfahren), EP-A 11 59 246 (zweite Reaktionsstufe), EP-A 11 59 247 (als zweistufiges Verfahren), DE-A 199 48 248 (als zweistufiges Verfahren), DE-A 101 01 695 (einstufig oder zweistufig), WO 04/085368 (als zweistufiges Verfahren), DE 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 99 06 36, EP-A 10 07 007 und EP-A 11 06 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe (Propylen zu Acrolein) ein erfindungsgemäßes Gasgemisch 2 eingesetzt wird. Die übrigen Parameter betreffend wird wie in den Ausführungsbeispielen der genannten Schriften verfahren (insbesondere die Katalysatorfestbetten und Reaktandenbelastung der Katalysatorfestbetten betreffend). Wird in den vorgenannten Ausführungsbeispielen des Standes der Technik zweistufig verfahren und erfolgt zwischen den beiden Reaktionsstufen eine Sekundärsauerstoff(Sekundärluft)einspeisung, so wird diese in entsprechender Weise vorgenommen, in ihrer Menge jedoch dahingehend angepasst, dass das molare Verhältnis von molekularem Sauerstoff zu Acrolein im Beschickungsgasgemisch der zweiten Reaktionsstufe demjenigen in den Ausführungsbeispielen der genannten Schriften entspricht.

Für die jeweilige Reaktionsstufe besonders geeignete Multimetalloxidkatalysatoren sind vielfach vorbeschrieben und dem Fachmann wohlbekannt. Beispielsweise verweist die EP-A 253 409 auf Seite 5 auf entsprechende US-Patente.

Erfindungsgemäß günstige Katalysatoren für die jeweilige Oxidationsstufe offenbaren auch die DE-A 44 31 957, die DE-A 10 2004 025 445 und die DE-A 4 431 949. Dieses gilt insbesondere für jene der allgemeinen Formel I in den beiden vorgenannten Schriften. Erfindungsgemäß besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe der heterogen katalysierten Gasphasen-Partialoxidation von Propylen zu Acrolein oder Acrylsäure oder deren Gemisch, kommen, wie bereits gesagt, prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 700 714 genannten Multimetalloxidaktivmassen.

Ferner eignen sich für diese Reaktionsstufe die Mo, Bi und Fe enthaltenden Multimetalloxidkatalysatoren die in den Schriften DE-A 100 46 957, DE-A 100 63 162, DE-C 3 338 380, DE-A 199 02 562, EP-A 015 565, DE-C 2 380 765, EP-A 807 465, EP-A 279 374, DE-A 33 00 044, EP-A 575 897, US 4,438,217, DE-A 198 55 913, WO 98/24746, DE-A 197 46 210 (diejenigen der allgemeinen Formen II), JP-A 91/294239, EP-A 293 224 und EP-A 700 714 offenbart werden. Dies gilt insbesondere für die beispielhaften Ausführungsformen in diesen Schriften, unter denen jene der EP-A 015 565, der EP-A 575 897, der DE-A 197 46 210 und der DE-A 198 55 913 erfindungsgemäß besonders bevorzugt werden. Besonders hervorzuheben sind in diesem Zusammenhang ein Katalysator gemäß Beispiel 1c aus der EP-A 015 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ·10SiO₂ aufweist. Ferner sind hervorzuheben das Beispiel mit der laufenden Nr. 3 aus der DE-A 198 55 913 (Stöchiometrie: MO₁₂Co₇Fe₃Bi_{0,6}K_{0,08}Si_{1,6}Ox) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm × 3,5 mm, oder 5 mm × 2 mm × 2 mm, oder 5 mm × 3 mm × 2 mm, oder 6 mm × 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt von Propylen zu Acrolein und gegebenenfalls Acrylsäure geeigneten Multimetalloxidaktivmassen lässt sich unter der allgemeinen Formel IV,

Mo₁₂BiₐFe_{b}X¹_{c}X²_{d}X³ₑX⁴_{f}Oₙ (IV),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a= 0,5 bis 5,
b= 0,01 bis 5, vorzugsweise 2 bis 4,
c= 0 bis 10, vorzugsweise 3 bis 10,
d= 0 bis 2, vorzugsweise 0,02 bis 2,
e= 0 bis 8, vorzugsweise 0 bis 5,
f= 0 bis 10 und
n= eine Zahl, die durch die Wertigkeit und Häufigkeit der von Sauerstoff verschiedenen Elemente in IV bestimmt wird,
subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden.

Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein (sowie gegebenenfalls Acrylsäure) erfindungsgemäß vorteilhaft zu verwendende Multimetalloxidaktivmassen sind ferner Massen der allgemeinen Formel V,

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (V),

in der die Variablen folgende Bedeutung haben:
Y¹ = nur Wismut oder Wismut und wenigstens eines der Elemente Tellur, Antimon, Zinn und Kupfer,
Y² = Molybdän oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶ = Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in V bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen V sind solche, in denen Y¹ nur Wismut ist.

Unter diesen werden wiederum jene bevorzugt, die der allgemeinen Formel VI,

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (VI),

in der die Varianten folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵= Phosphor, Arsen, Bor, Antimon, Zinn, Cer und/oder Blei,
Z⁶ = Silicium, Aluminium, Titan und/oder Zirkonium,
Z⁷= Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in VI bestimmt werden,
p",q"=Zahlen, deren Verhältnis p"/q" 0,1 bis 5, vorzugsweise 0,5 bis 2 beträgt,
entsprechen, wobei diejenigen Massen VI ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

An dieser Stelle sei erwähnt, dass sich alle Katalysatoren und Multimetalloxidmassen, die erfindungsgemäß für den Schritt vom Propylen zum Acrolein als geeignet empfohlen wurden, sich grundsätzlich auch für die Partialammoxidation von Propylen zu Acrylnitril eignen.

Für den zweiten Schritt (die zweite Reaktionsstufe), die heterogen katalysierte Gasphasen-Partialoxidation von Acrolein zu Acrylsäure, kommen erfindungsgemäß vorteilhaft, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

Mo₁₂VₐX¹_{b}X²_{c}X³_{d}X⁴ₑX⁵_{f}X⁶_{g}Oₙ (VII),

in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,
subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:
X¹ = W, Nb, und/oder Cr,
X² = Cu, Ni, Co, und/oder Fe,
X³ = Sb,
X⁴ = Na und/oder K,
X⁵ = Ca, Sr und/oder Ba,
X⁶= Si, Al, und/oder Ti,

a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VIII,

Mo₁₂V_{a'}Y¹_{b'}Y²_{c'}Y⁵_{f'}Y⁶_{g'}O_{n'} (VIII),

mit
Y¹ = W und/oder Nb,
Y² = Cu und/oder Ni,
Y⁵ = Ca und/oder Sr,
Y⁶ = Si und/oder Al,

a' = 2 bis 4,
b' = 1 bis 1,5,
c'= 1 bis 3,
f = 0 bis 0,5
g' = 0 bis 8 und
n' = eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Partialoxidationsreaktionsschritt "Acrolein → Acrylsäure" geeignete Multimetalloxidaktivmassen, insbesondere solche der allgemeinen Formel VII, in einfacher Weise (wie die für den ersten Partialoxidationsschritt geeigneten Multimetalloxidaktivmassen) dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter I-nertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperatureri von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure.als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Reaktionsschritt "Acrolein → Acrylsäure" zu verwendende Multimetalloxidaktivmassen sind ferner Massen der allgemeinen Formel IX,

[D]ₚ[E]_{q} (IX),

in der die Variablen folgende Bedeutung haben:
D = Mo₁₂V_{a"}Z¹_{b"}Z²_{c"}Z³_{d"}Z⁴_{e"}Z⁵_{f"}Z⁶_{g"}Oₓ,
E = Z⁷₁₂Cu_{h"}H_{i"}O_{y"},
Z¹ = W, Nb, Ta, Cr und/oder Ce,
Z² = Cu, Ni, Co, Fe, Mn und/oder Zn,
Z3 = Sb und/oder Bi,
Z⁴ = Li, Na, K, Rb, Cs und/oder H
Z⁵ = Mg, Ca, Sr und/oder Ba,
Z⁶ = Si, Al, Ti und/oder Zr,
Z⁷ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysator geometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetall-oxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung des erfindungsgemäßen Verfahrens, vom Propylen zum Acrolein (sowie gegebenenfalls Acrylsäure), kann mit den beschriebenen Katalysatoren z. B. in einem Einzonen-Vielkontaktrohr-Festbettreaktor erfolgen, wie ihn die DE-A 4 431 957 beschreibt. Dabei können Gasgemisch 2 und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas(ausgangs)gemisch 2 beträgt vorzugsweise 1500 bis 4000 bzw. 6000 Nl/l·h oder mehr. Die Propylenlast (die Propylenbelastung des Katalysatorfestbetts) beträgt typisch 90 bis 200 Nl/l·h oder bis 300 Nl/l·h oder mehr. Propylenlasten oberhalb von 120, bzw. 130, bzw. 135 Nl/l·h bzw. ≥ 140 Nl/l·h, oder ≥ 150 Nl/l·h, oder ≥ 160 Nl/l·h sind erfindungsgemäß besonders bevorzugt, da hohe Propylenbelastungen erhöhte Heißpunkte und damit eine erhöhte Partialoxidation der C₂-KW bedingen (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat (KNO₃) und 40 Gew.-% Natriumnitrit (NaNO₂) oder aus 53 Gew.-% Kaliumnitrat (KNO₃), 40 Gew.-% Natriumnitrit (NaNO₂) und 7 Gew.-% Natriumnitrat (NaNO₃), eingesetzt.

Über den Reaktor betrachtet können, wie bereits gesagt, Salzschmelze und Reakti-onsgasgemisch 2 sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):
- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 20 Gew.-% ausmacht (Abschnitt C);
- daran anschließend auf einer Länge von 20 bis 50 bzw. bis 40 % der Gesamtrohrlänge entweder nur Katalysator, oder ein Gemisch aus Katalysator und I-nertmaterial, wobei letzteres, bezogen auf die Mischung, einen Gewichtsanteil von bis zu 40 Gew.-% ausmacht (Abschnitt B); und
- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Durchführung der erfindungsgemäßen Partialoxidation, vom Propylen zum Acrolein (und gegebenenfalls Acrylsäure), kann mit den beschriebenen Katalysatoren aber auch z. B. in einem Zweizonen-Vielkontaktrohr-Festbettreaktor erfolgen, wie es die DE-A 199 10 506 oder die EP-A 11 06 598 beschreibt. In beiden vorstehend beschriebenen Fällen (sowie ganz allgemein beim erfindungsgemäßen Verfahren) liegt der erzielte Propenumsatz bei einfachem Durchgang normalerweise bei Werten ≥ 90 mol-%, bzw. ≥ 95 mol-% und die Selektivität der Acroleinbildung bei Werten ≥ 90 mol-%. Erfin-dungsgemäß vorteilhaft erfolgt die erfindungsgemäße Partialoxidation von Propen zu Acrolein, oder Acrylsäure oder deren Gemisch wie in der EP-A 1159244 und ganz besonders bevorzugt wie in der WO 04/085363 sowie in der WO 04/085362 beschrieben, jedoch mit dem Unterschied, dass als Reaktionsgasausgangsgemisch ein erfindungsgemäßes Gasgemisch 2 verwendet wird. Insbesondere können alle Ausführungsbeispiele der vorgenannten Schriften so wie in diesen Schriften beschrieben durchgeführt werden, jedoch unter Anwendung eines Gasgemischs 2 als Beschickungsgasgemisch. Insbesondere mit den in dieser Schrift als besonders bevorzugt und als beispielhaft ausgeführten Gasgemischen 2.

Die Schriften EP-A 1159244, WO 04/085363 und WO 04/085362 werden als integraler Bestandteil dieser Schrift betrachtet.

Eine bevorzugte Ausführungsform der erfindungsgemäßen Partialoxidation des Propylens zu Acrolein, oder Acrylsäure, oder deren Gemisch ist ein erfindungsgemäßes Verfahren, bei dem man das Gasgemisch 2 mit der Maßgabe über eine Festbettkatalysatorschüttung, deren Aktivmasse wenigstens ein die Elemente Mo, Fe und Bi enthaltendes Multimetalloxid ist, führt, dass
- die Festbettkatalysatorschüttung in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung aus wenigstens zwei räumlich aufeinander folgenden Festbettkatalysatorschüttungszonen besteht, wobei die völumenspezifische Aktivität innerhalb einer Festbettkatalysatorschüttungszone im wesentlichen konstant ist und in Strömungsrichtung des Gasgemisches 2 beim Übergang von einer Festbettkatalysatorschüttungszone in eine andere Festbettkatalysator-schüttungszone (zweckmäßig sprunghaft) zunimmt,
- sich die Temperaturzone A bis zu einem Umsatz des Propens von 40 bis 80 mol.-% erstreckt,
- bei einmaligem Durchgang des Gasgemisches 2 durch die gesamte Festbettkatalysatorschüttung der Propenumsatz ≥ 90 mol.-% und die Selektivität der Acroleinbildung, bezogen auf umgesetztes Propen, ≥ 90 mol.-% beträgt,
- die zeitliche Abfolge, in der das Gasgemisch 2 die Temperaturzonen A, B durchströmt, der alphabetischen Abfolge der Temperaturzonen entspricht,
- die Belastung der Festbettkatalysatorschüttung mit dem im Gasgemisch 2 enthaltenen Propen ≥ 90 Nl Propen/l Festbettkatalysatorschüttung · h beträgt, und
- die Differenz T^{maxA} - T^{maxB}, gebildet aus der höchsten Temperatur T^{maxA}, die das Gasgemisch 2 innerhalb der Temperaturzone A aufweist, und der höchsten Temperatur T^{maxB}, die das Gasgemisch 2 innerhalb der Temperaturzone B aufweist, ≥ 0°C beträgt,
und das vorzugsweise zusätzlich dadurch gekennzeichnet ist, dass der Übergang von der Temperaturzone A in die Temperaturzone B in der Festbettkatalysatorschüttung nicht mit einem Übergang von einer Festbettkatalysatorschüttungszone in eine andere Festbettkatalysatorschüttungszone zusammenfällt.

Nähere Angaben zu dieser Verfahrensweise finden sich in der WO 04/085362, die integraler Bestandteil dieser Schrift ist, sowie im weiteren Verlauf dieser Schrift bei der Beschreibung der besonders bevorzugten zweistufigen Partialoxidation von Propylen zu Acrylsäure.

Die Durchführung des zweiten Schrittes im Fall einer zweistufigen Partialoxidation von Propylen zu Acrolein, nämlich die Partialoxidation vom Acrolein zur Acrylsäure, kann mit den beschriebenen Katalysatoren z. B. in einem Einzonen-Vielkontaktrohr-Festbettreaktor erfolgen, wie es die DE-A 44 31 949 beschreibt. Dabei können Reaktionsgasgemisch und Wärmeträger über den Reaktor betrachtet im Gleichstrom geführt werden. In der Regel wird das Produktgasgemisch der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärluftzugabe erfolgen) desselben), d.h., ohne Nebenkomponentenabtrennung, in die zweite Reaktionsstufe, d.h. in die Acroleinpartialoxidation geführt.

Der für den zweiten Schritt, die Acroleinpartialoxidation, benötigte molekulare Sauerstoff kann (dabei) schon im Gasgemisch 2 für die erfindungsgemäße Propylenpartialoxidation zum Acrolein enthalten sein. Er kann aber auch teilweise oder vollständig dem Produktgasgemisch der ersten Reaktionsstufe, d. h., der erfindungsgemäßen Propylenpartialoxidation zum Acrolein, unmittelbar zugegeben werden (dies erfolgt vorzugsweise als (Sekundär)Luft, kann jedoch auch in Form von reinem Sauerstoff oder von Gemischen aus Inertgas oder Sauerstoff erfolgen). Unabhängig davon wie vorgegangen wird, weist das Beschickungsgasgemisch einer solchen Partialoxidation des Acroleins zu Acrylsäure, mit Vorteil nachfolgende Gehalte auf:

| | |
|---|---|
| 4,5 bis 8 Vol.-% | Acrolein, |
| 2,25 bzw. 4,5 bis 9 Vol.-% | molekularer Sauerstoff, |
| 6 bis 30 Vol.-% | Propan, |
| 32 bis 72 Vol.-% | molekularer Stickstoff und |
| 5 bis 15 Vol.-%, bzw. bis 30 Vol.-% | Wasserdampf. |

Bevorzugt weist das vorgenannte Beschickungsgasgemisch folgende Gehalte auf:

| | |
|---|---|
| 5,5 bis 8 Vol.-% | Acrolein, |
| 2,75 bzw. 5,5 bis 9 Vol.-% | molekularer Sauerstoff, |
| 10 bis 25 Vol.-% | Propan, |
| 40 bis 70 Vol.-% | molekularer Stickstoff und |
| 5 bis 15 Vol.-% | Wasserdampf. |

Ganz besonders bevorzugt weist das vorgenannte Beschickungsgemisch folgende Gehalte auf:

| | |
|---|---|
| 6 bis 8 Vol.-% | Acrolein (bevorzugt 6 bis 7 Vol.-%), |
| 3 bzw. 6 bis 9 Vol.-% | molekularer Sauerstoff, |
| 10 bis 20 Vol-% | Propan (bevorzugt 10 bis 16 Vol.-%), |
| 50 bis 65 Vol.-% | molekularer Stickstoff und |
| 7 bis 13 Vol.-% | Wasserdampf, |

wobei die Vorzugsbereiche unabhängig voneinander gelten, mit Vorteil jedoch simultan realisiert werden.

Ferner ist es für vorgenannte Beschickungsgasgemische von Vorteil, wenn das molare Verhältnis V₄ von im Beschickungsgasgemisch enthaltenem molekularem Sauerstoff zu im Beschickungsgemisch enthaltenem Acrolein ≥ 0,5 und ≤ 2, mit Vorteil ≥ 1 und ≤ 1,75, besonders vorteilhaft ≥1 und ≤ 1,5 und ganz besonders vorteilhaft ≥ 1 und ≤ 1,25 beträgt.

Weiterhin ist es für vorgenannte Beschickungsgasgemische von Vorteil, wenn das molare Verhältnis V₅ von im Beschickungsgasgemisch enthaltenem Propan zu in ihm enthaltenem Acrolein 1 bis 4, mit Vorzug 1,5 bis 3,5, besonders bevorzugt 1,5 bis 3 und ganz besonders bevorzugt 1,5 bzw. 2 bis 2,5 beträgt.

Wie in der ersten Reaktionsstufe liegt auch in der zweiten Reaktionsstufe der Reaktionsdruck üblicherweise im Bereich von 1 bis 3 bar, und die Gesamtraumbelastung des Katalysatorfestbetts mit Beschickungsgasgemisch liegt vorzugsweise bei 1500 bis 4000 bzw. 6000 Nl/l·h oder mehr. Die Acroleinlast (die Acroleinbelastung des Katalysatorfestbetts) beträgt typisch 90 bis 190 Nl/l·h, oder bis 290 Nl/l·h oder mehr. Acroleinlasten oberhalb von 110, bzw. 120, bzw. 130, bzw. 135 Nl/l·h, bzw. ≥ 140 Nl/l·h, oder ≥ 150 Nl/l·h, oder ≥ 160 Nl/l·h sind besonders bevorzugt, da erhöhte Heißpunkte einen erhöhten erfindungsgemäßen C₂-KW Auslass bedingen

Der Acroleinumsatz, bezogen auf einmaligen Durchgang des Beschickungsgasgemischs durch das Katalysatorfestbett, beträgt zweckmäßig normalerweise ≥ 90 mol-% und die damit einhergehende Selektivität der Acrylsäurebildung ≥ 90 mol-%.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat (KNO₃) und 40 Gew.-% Natriumnitrit (NaNO₂) oder aus 53 Gew.-% Kaliumnitrat (KNO₃), 40 Gew.-% Natriumnitrit (NaNO₂) und 7 Gew.-% Natriumnitrat (Na-NO₃) bestehend, eingesetzt. Über den Reaktor betrachtet können, wie bereits gesagt, Salzschmelze und Beschickungsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:
- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C);
- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und
- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttemperatur und Heißpunkttemperatursensitivität). Von unten nach oben zunächst mit bis zu 20 Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßiger Weise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmesser) verwendet werden. Hinsichtlich der Salzbadtemperatur gilt das in der DE-A 443 19 49 Gesagte. Sie wird in der Regel so gewählt, dass der erzielte Acro-leinumsatz bei einfachem Durchgang normalerweise ≥ 90 mol-%, bzw. ≥ 95 mol-% oder ≥ 99 mol-% beträgt.

Die Durchführung der Partialoxidation vom Acrolein zur Acrylsäure, kann mit den beschriebenen Katalysatoren aber auch z. B. in einem Zweizonen-Vielkontaktrohr-Festbettreaktor erfolgen, wie es die DE-199 10 508 beschreibt. Für den Acroleinumsatz gilt das oben Genannte. Auch im Fall der Durchführung einer wie vorstehend beschriebenen Acroleinpartialoxidation als zweite Reaktionsstufe einer zweistufigen Propylenoxidation zu Acrylsäure in einem Zweizonen-Vielkontaktrohr-Festbettreaktor wird man zur Erzeugung des Beschickungsgasgemisches für die Acroleinpartialoxidation zweckmäßig unmittelbar das Produktgasgemisch der auf den ersten Schritt gerichteten Partialoxidation (gegebenenfalls nach erfolgter indirekter oder direkter (z. B. durch Zufuhr von Sekundärluft) Zwischenkühlung desselben) verwenden (wie sie vorstehend bereits beschrieben wurde). Der für die Acroleinpartialoxidation benötigte Sauerstoff wird vorzugsweise als Luft (gegebenenfalls aber auch als reiner molekularer Sauerstoff oder als Gemisch aus molekularem Sauerstoff und einem Inertgas) zugesetzt und z. B. dem Produktgasgemisch des ersten Schritts der zweistufigen Partialoxidation (Propylen → Acrolein) unmittelbar zugegeben. Er kann aber auch, wie bereits beschrieben, bereits im Gasgemisch für die erste Reaktionsstufe enthalten sein.

Bei einer zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partial-oxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 101 21 592 beschrieben in einem Reaktor an einer Beschickung vollzogen werden.

Es sei auch nochmals erwähnt, dass ein Teil des Gasgemischs 2 für den ersten Schritt ("Propylen → Acrolein") aus der Partialoxidation kommendes Oxidationskreisgas (Restgas) sein kann.

Hierbei handelt es sich um ein molekularen Sauerstoff enthaltendes Gas, das nach der Zielproduktabtrennung (Acrolein- und/oder Acrylsäureabtrennung) vom Produktgasge-misch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan, molekularen Sauerstoff und gegebenenfalls nicht umgesetztes Propylen enthaltendes Oxidationskreisgas jedoch vorteilhaft ausschließlich in die gegebenenfalls als Propylenquelle fungierende heterogen katalysierte Propandehydrierung rückgeführt.

Insgesamt bildet ein Rohrbündelreaktor innerhalb dessen sich die Katalysatorbeschickung längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 11 06 598, die EP-A 911 313, die EP-A 979 813, die EP-A 990 636 und die DE-A 28 30 765) die einfachste Realisierungsform zweier Oxidationsstufen für die beiden Schritte der Partialoxidation von Propylen zu Acrylsäure. Gegebenenfalls wird dabei die Beschickung der Kontaktrohre mit Katalysator durch eine Inertmaterialschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertmaterialschüttung nicht. Mit Vorteil werden daher die beiden Kontaktrohr-bündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Die Reaktionstemperatur in der ersten Reaktionsstufe (Propylen → Acrolein) liegt generell in der Regel bei 300 bis 450°C, bevorzugt bei 320 bis 390°C. Die Reaktionstemperatur in der zweiten Reaktionsstufe (Acrolein → Acrylsäure) liegt generell in der Regel bei 200 bis 370°C, häufig bei 220 bis 330°C. Der Reaktionsdruck beträgt in beiden Oxidationszonen zweckmäßig 0,5 bis 5, vorteilhaft 1 bis 3 bar. Die Belastung (Nl/l·h) der Oxidationskatalysatoren mit Reaktionsgas beträgt in beiden Reaktionsstufen häufig 1500 bis 2500 Nl/l·h bzw. bis 4000 Nl/l·h. Die Belastung mit Propylen kann dabei bei 100 bis 200 oder 300 und mehr Nl/l·h liegen.

Prinzipiell können die beiden Oxidationsstufen beim erfindungsgemäßen Verfahren so gestaltet werden, wie es z. B. in der DE-A 198 37 517, der DE-A 199 10 506, der DE- A 199 10 508 sowie der DE-A 198 37 519 beschrieben ist.

In beiden Reaktionsstufen wirkt sich dabei ein Überschuss an molekularem Sauerstoff relativ zur reaktionsstöchiometrisch erforderlichen Menge in der Regel vorteilhaft auf die Kinetik der jeweiligen Gasphasenpartialoxidation aus.

Prinzipiell ist die erfindungsgemäße heterogen katalysierte Gasphasen-Partialoxidation von Propylen zu Acrylsäure aber auch in einem einzigen Einzonenrohrbündelreaktor wie folgt realisierbar. Beide Reaktionsschritte erfolgen in einem Oxidationsreaktor der mit einem oder mehreren Katalysatoren beschickt ist, deren Aktivmasse ein die Elemente Mo, Fe und Bi enthaltendes Multimetalloxid ist, das die Umsetzung beider Reaktionsschritte zu katalysieren vermag. Selbstredend kann sich auch diese Katalysatorbeschickung längs der Reaktionskoordinate kontinuierlich oder abrupt ändern. Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassende Produktgasgemisch in selbigem enthaltenes, in der ersten Oxidationsstufe als Nebenprodukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch einer Abtrennung nicht vorsieht.

Als Quellen für eine zwischen beiden Oxidationsstufen durchgeführte Sauerstoffzwischeneinspeisung kommen, wie bereits gesagt, neben Luft (bevorzugt) sowohl reiner molekularer Sauerstoff als auch mit Inertgas wie CO₂, CO, Edelgasen, N₂ und/oder gesättigten Kohlenwasserstoffen verdünnter molekularer Sauerstoff in Betracht.

Durch Zudosieren von z. B. kalter Luft zu Produktgasgemisch der ersten Reaktionsstufe (Propen → Acrolein) kann im Rahmen des erfindungsgemäßen Verfahrens auch auf direktem Weg eine Abkühlung desselben bewirkt werden, bevor es als Bestandteil eines Beschickungsgasgemischs für die zweite Reaktionsstufe weiterverwendet wird.

Erfindungsgemäß vorteilhaft erfolgt die Partialoxidation von Acrolein zu Acrylsäure wie in der EP-A 11 59 246, und ganz besonders bevorzugt wie in der WO 04/085365 sowie in der WO 04/085370 beschrieben. Erfindungsgemäß bevorzugt wird dabei als Acrolein enthaltendes Beschickungsgasgemisch jedoch ein Beschickungsgasgemisch verwendet (dies kann insbesondere das Produktgasgemisch einer erfindungsgemäßen Erststufenpartialoxidation von Propylen zu Acrolein sein), das gegebenenfalls mit soviel Sekundärluft ergänzt wurde, dass das Verhältnis von molekularem Sauerstoff zu Acrolein in dem resultierenden Beschickungsgasgemisch in jedem Fall 0,5 bis 1,5 beträgt. Insbesondere können alle Ausführungsbeispiele der vorgenannten Schriften so wie in diesen Schriften beschrieben durchgeführt werden, jedoch unter Anwendung eines Beschickungsgasgemischs gemäß dieser Schrift. Insbesondere mit den in dieser Schrift als besonders bevorzugt und als beispielhaft ausgeführten Beschickungsgasgemischen für eine Acroleinpartialoxidation. Die Schriften EP-A 1159246, WO 04/085365 und WO 04/085370 werden als integraler Bestandteil dieser Schrift betrachtet.

D.h., die erfindungsgemäße Partialoxidation von Acrolein zu Acrylsäure lässt sich vorteilhaft an einem Katalysatorfestbett mit erhöhter Acroleinbelastung durchführen, das wenigstens zwei Temperaturzonen aufweist, wie es z.B. in der EP-A 11 59 246 beschrieben ist.

D.h., eine bevorzugte Ausführungsform der erfindungsgemäßen Partialoxidation des Acroleins zu Acrylsäure ist ein erfindungsgemäßes Verfahren, bei dem man das Acrolein enthaltende Beschickungsgasgemisch mit der Maßgabe über eine Festbettkatalysatorschüttung, deren Aktivmasse wenigstens ein die Elemente Mo und V enthaltendes Multimetalloxid ist, führt, dass
- die Festbettkatalysatorschüttung in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung aus wenigstens zwei räumlich aufeinanderfolgenden Festbettkatalysatorschüttungszonen besteht, wobei die völumenspezifische Aktivität innerhalb einer Festbettkatalysatorschüttungszone im wesentlichen konstant ist und in Strömungsrichtung des Acroleins enthaltenden Beschickungsgasgemischs beim Übergang von einer Festbettkatalysatorschüttungszone in eine andere Festbettkatalysatorschüttungszone (in zweckmäßiger Weise sprunghaft) zunimmt,
- sich die Temperaturzone C bis zu einem Umsatz des Acroleins von 45 bis 85 mol-% erstreckt,
- bei einmaligem Durchgang des Acrolein enthaltenden Beschickungsgasgemischs durch die gesamte Festbettkatalysatorschüttung der Acroleinumsatz ≥ 90 mol.-% und die Selektivität der Acrylsäurebildung, bezogen auf umgesetztes Acrolein, ≥ 90 mol.-% beträgt,
- die zeitliche Abfolge, in der das Acrolein enthaltende Beschickungsgasgemisch die Temperaturzonen C, D durchströmt, der alphabetischen Abfolge der Temperaturzonen entspricht,
- die Belastung der Festbettkatalysatorschüttung mit dem im Beschickungsgasgemisch enthaltenen Acrolein ≥ 70 Nl Acrolein/l Festbettkatalysatorschüttung · h beträgt, und
- die Differenz T^{maxC} - T^{maxD}, gebildet aus der höchsten Temperatur T^{maxC}, die das Acrolein enthaltende Beschickungsgasgemisch innerhalb der Temperaturzone C aufweist, und der höchsten Temperatur T^{maxD}, die das Acrolein enthaltende Beschickungsgasgemisch innerhalb der Temperaturzone D aufweist, ≥ 0°C beträgt,
und das vorzugsweise zusätzlich dadurch gekennzeichnet ist, dass der Übergang von der Temperaturzone C in die Temperaturzone D in der Festbettkatalysatorschüttung nicht mit einem Übergang von einer Festbettkatalysatorschüttungszone in eine andere Festbettkatalysatorschüttungszone zusammenfällt.

Nähere Angaben zu dieser Verfahrensweise finden sich in der WO 04/085370, die integraler Bestandteil dieser Schrift ist, sowie im weiteren Verlauf dieser Schrift bei der Beschreibung der besonders bevorzugten zweistufigen Partialoxidation von Propylen zu Acrylsäure.

Eine solche bevorzugte zweistufige Partialoxidation von Propylen zu Acrylsäure kann vorteilhaft wie in der EP-A 1159248 sowie in der WO 04/085367 beschrieben durchgeführt werden, jedoch mit dem Unterschied, dass als Reaktionsgasausgangsgemisch für die erste Oxidationsstufe (Propylen zu Acrolein) ein erfindungsgemäßes Gasgemisch 2 verwendet wird (insbesondere auch in den Ausführungsbeispielen der EP-A 1159248 sowie der WO 04/085367; beide Schriften bilden einen integralen Bestandteil dieser Schrift).

Besonders bevorzugt wird man sie jedoch gemäß der WO 04/085369 durchführen, die integraler Bestandteil dieser Schrift ist, jedoch mit dem Unterschied, dass als Reaktionsgasausgangsgemisch für die erste Oxidationsstufe (Propylen zu Acrolein) ein erfindungsgemäßes Gasgemisch 2 verwendet wird (insbesondere auch in den Ausführungsbeispielen der WO 04/085369).

D.h., man wird zunächst ein erfindungsgemäßes Gasgemisch 2 in einer ersten Reaktionsstufe mit der Maßgabe über eine Festbettkatalysatorschüttung 1, deren Aktivmasse wenigstens ein die Elemente Mo, Fe und Bi enthaltendes Multimetalloxid ist, führen, dass
- die Festbettkatalysatorschüttung 1 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Gasgemischs 2 beim Übergang von einer Festbettkatalysatorschüttungszone in eine andere Festbettkatalysatorschüttungszone (in zweckmäßiger Weise sprunghaft) zunimmt,
- sich die Temperaturzone A bis zu einem Umsatz des Propens von 40 bis 80 mol-% erstreckt,
- bei einmaligem Durchgang des Gasgemischs 2 durch die gesamte Festbettkatalysatorschüttung 1 der Propenumsatz ≥ 90 mol-% und die Selektivität der Acroleinbildung sowie der Acrylsäurenebenproduktbildung zusammengenommen und bezogen auf umgesetztes Propens ≥ 90 mol-% beträgt,
- die zeitliche Abfolge, in der das Gasgemisch 2 die Temperaturzonen A, B durchströmt, der alphabetischen Abfolge der Temperaturzonen A, B entspricht,
- die Belastung der Festbettkatalysatorschüttung 1 mit dem im Gasgemisch 2 enthaltenen Propens ≥ 90 Nl Propen/l Festbettkatalysatorschüttung 1 · h beträgt, und
- die Differenz T^{maxA} - T^{maxB}, gebildet aus der höchsten Temperatur T^{maxA}, die das Gasgemisch 2 innerhalb der Temperaturzone A aufweist, und der höchsten Temperatur T^{maxB}, die das Gasgemisch 2 innerhalb der Temperaturzone B aufweist, ≥ 0°C beträgt,
danach die Temperatur des die erste Reaktionsstufe verlassenden Produktgasgemisches durch Kühlung gegebenenfalls verringern und dem Produktgasgemisch gegebenenfalls molekularen Sauerstoff und/oder Inertgas, vorzugsweise gegebenenfalls Luft, zugeben, und es anschließend als Acrolein, molekularen Sauerstoff und wenigstens ein Inertgas enthaltendes Beschickungsgasgemisch, das den molekularen Sauerstoff und das Acrolein in einem molaren Verhältnis O₂:C₃H₄O ≥ 0,5 enthält, in einer zweiten Reaktionsstufe mit der Maßgabe über eine Festbettkatalysatorschüttung 2, deren Aktivmasse wenigstens ein die Elemente Mo und V enthaltendes Multimetalloxid ist, führen, dass
- die Festbettkatalysatorschüttung 2 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung 2 aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Beschickungsgasgemischs beim Übergang von einer Festbettkatalysatorschüttungszone in eine andere Festbettkatalysatorschüttungszone (in zweckmäßiger Weise sprunghaft) zunimmt,
- sich die Temperaturzone C bis zu einem Umsatz des Acroleins von 45 bis 85 mol-% erstreckt,
- bei einmaligem Durchgang des Beschickungsgasgemischs durch die gesamte Festbettkatalysatorschüttung 2 der Acroleinumsatz ≥ 90 mol-% und die Selektivität der Acrylsäurebildung, bezogen auf über beide Reaktionsstufen umgesetztes Propen, ≥ 80 mol-% beträgt,
- die zeitliche Abfolge, in der das Beschickungsgasgemisch die Temperaturzonen C, D durchströmt, der alphabetischen Abfolge der Temperaturzonen C, D entspricht,
- die Belastung der Festbettkatalysatorschüttung 2 mit dem im Beschickungsgasgemisch enthaltenen Acrolein ≥ 70 Nl Acrolein/l Festbettkatalysatorschüttung 2·h beträgt, und
- die Differenz T^{maxC} - T^{maxD}, gebildet aus der höchsten Temperatur T^{maxC}, die das Beschickungsgasgemisch innerhalb der Temperaturzone C aufweist, und der höchsten Temperatur T^{maxD}, die das Beschickungsgasgemisch innerhalb der Temperaturzone D aufweist, ≥ 0°C beträgt,
mit der bevorzugten Maßgabe, dass das Verfahren vorzugsweise zusätzlich dadurch gekennzeichnet ist, dass weder der Übergang von der Temperaturzone A in die Temperaturzone B in der Festbettkatalysatorschüttung 1, noch der Übergang von der Temperaturzone C in die Temperaturzone D in der Festbettkatalysatorschüttung 2 mit einem Übergang von einer Festbettkatalysatorschüttungszone in eine andere Festbettkatalysatorschüttungszone zusammenfällt.

Unter der Temperatur einer Temperaturzone wird dabei die Temperatur des in der Temperaturzone befindlichen Teils der Festbettkatalysatorschüttung bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur der Festbettkatalysatorschüttung längs der Reaktionszone. Wesentlich ist dabei, dass die Temperierung der einzelnen Temperaturzonen im wesentlichen unabhängig voneinander erfolgt.

Da sowohl die heterogen katalysierte partielle Gasphasenoxidation von Propen zu Acrolein als auch die heterogen katalysierte partielle Gasphasenoxidation von Acrolein zu Acrylsäure eine ausgeprägt exotherme Reaktion ist, ist die Temperatur der Reaktionsgasgemische beim reaktiven Durchgang durch die Festbettkatalysatorschüttung 1 bzw. Festbettkatalysatorschüttung 2 in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz T^{maxA} - T^{maxB} nicht mehr als 80°C betragen. Erfindungsgemäß bevorzugt beträgt T^{maxA} - T^{maxB} ≥ 3°C und ≤ 70°C. Ganz besonders bevorzugt beträgt T^{maxA} - T^{maxB} beim erfindungsgemäßen Verfahren ≥ 20°C und ≤ 60°C.

Die erfindungsgemäß geforderten Differenzen T^{maxA} - T^{maxB} stellen sich bei der Ausübung des erfindungsgemäßen Verfahren im Fall von eher niederen (≥ 90 Nl/l·h und ≤ 160 Nl/l·h) Propenbelastungen der Festbettkatalysatorschüttung 1 normalerweise dann ein, wenn einerseits sowohl die Temperatur der Reaktionszone A als auch die Temperatur der Reaktionszone B im Bereich von 290 bis 380°C liegt und andererseits die Differenz zwischen der Temperatur der Reaktionszone B (T_{B}) und der Temperatur der Reaktionszone A (T_{A}), d.h., T_{B} - T_{A}, ≤ 0°C und ≥ -20°C oder ≥ -10°C bzw. ≤ 0°C und ≥ -5°C, oder häufig ≤ 0°C und ≥ -3°C beträgt.

Bei Ausübung des erfindungsgemäßen Verfahrens unter (erfindungsgemäß bevorzugt) erhöhten Propenbelastungen (≥ 160 Nl/l·h und ≤ 300 Nl/l·h, bzw. ≤ 600 Nl/l·h) stellen sich die erfindungsgemäß geforderten Differenzen T^{maxA} - T^{maxB} normalerweise dann ein, wenn einerseits sowohl die Temperatur der Reaktionszone A als auch die Temperatur der Reaktionszone B im Bereich von 290 bis 380°C liegt und T_{B} - T_{A} ≥ 0°C und ≤ 50°C, oder ≥ 5°C und ≤ 45°C, oder ≥ 10°C und ≤ 40°C, oder ≥ 15°C und ≤ 30°C oder ≤ 35°C (z.B. 20°C oder 25°C) beträgt.

Die vorgenannte Aussage betreffend die Temperaturdifferenzen T_{B}-T_{A} gilt regelmäßig auch dann, wenn die Temperatur der Reaktionszone A im bevorzugten Bereich von 305 bis 365°C bzw. im besonders bevorzugten Bereich von 310 bis 340°C liegt.

Die Propenbelastung der Festbettkatalysatorschüttung 1 kann somit beim beschriebenen Verfahren z.B. ≥ 90 Nl/l·h und ≤ 300 Nl/l·h, oder ≥ 110 Nl/l·h und ≤ 280 Nl/l·h oder ≥ 130 Nl/l·h und ≤ 260 Nl/l·h, oder ≥ 150 Nl/l·h und ≤ 240 Nl/l·h, oder ≥ 170 Nl/l·h und ≤ 220 Nl/l·h, oder ≥ 190 Nl/l·h und ≤ 200 Nl/l·h betragen.

Erfindungsgemäß bevorzugt erstreckt sich die Temperaturzone A bis zu einem Umsatz des Propens von 50 bis 70 mol-% bzw. 60 bis 70 mol-%.

In der Regel wird beim erfindungsgemäßen Verfahren die Differenz T^{maxC} - T^{maxD} nicht mehr als 75°C betragen. Erfindungsgemäß bevorzugt beträgt T^{maxC} - T^{maxD} ≥ 3°C und ≤ 60°C. Ganz besonders bevorzugt beträgt T^{maxC} - T^{maxD} beim erfindungsgemäßen Verfahren ≥ 5°C und ≤ 40°C.

Die erfindungsgemäß geforderten Differenzen T^{maxC} - T^{maxD} stellen sich bei der Ausübung des erfindungsgemäßen Verfahren im Fall von eher niederen (≥ 70 Nl/l·h und ≤ 150 Nl/l·h) Acroleinbelastungen der Festbettkatalysatorschüttung 2 normalerweise dann ein, wenn einerseits sowohl die Temperatur der Reaktionszone C als auch die Temperatur der Reaktionszone D im Bereich von 230 bis 320°C liegt und andererseits die Differenz zwischen der Temperatur der Reaktionszone D (T_{D}), und der Temperatur der Reaktionszone C (T_{C}), d.h., T_{D} - T_{C}, ≤ 0°C und ≥ -20°C oder ≥ 10°C bzw. ≤ 0°C und ≥ -5°C, oder häufig ≤ 0°C und ≥ -3°C beträgt.

Bei Ausübung des erfindungsgemäßen Verfahrens unter erhöhten Propenbelastungen und damit gleichzeitig erhöhten Acroleinbelastungen (≥ 150 Nl/l·h und ≤ 300 Nl/l·h, bzw. ≤ 600 Nl/l·h) stellen sich die erfindungsgemäß geforderten Differenzen T^{maxC} - T^{maxD} normalerweise dann ein, wenn einerseits sowohl die Temperatur der Reaktions-zone C als auch die Temperatur der Reaktionszone D im Bereich von 230 bis 320°C liegt und T_{D} - T_{C} ≥ 0°C und ≤ 40°C, oder ≥ 5°C und ≤ 35°C, bzw. 30°C, oder ≥ 10°C und ≤ 25°C, bzw. ≤ 20°C, oder ≤ 15°C beträgt.

Die vorgenannte Aussage betreffend die Temperaturdifferenzen T_{D} - T_{C} gilt regelmäßig auch dann, wenn die Temperatur der Reaktionszone C im bevorzugten Bereich von 250 bis 300°C bzw. im besonders bevorzugten Bereich von 260 bis 280°C liegt.

Die Acroleinbelastung der Festbettkatalysatorschüttung 2 kann somit beim erfindungsgemäßen Verfahren z. B. ≥ 70 Nl/l·h bzw. ≥ 90 Nl/l·h und ≤ 300 Nl/l·h , oder ≥ 110 Nl/l·h und ≤ 280 Nl/l·h oder ≥ 130 Nl/l·h und ≤ 260 Nl/l·h, oder ≥ 150 Nl/l·h und ≤ 240 Nl/l·h, oder ≥ 170 Nl/l·h und ≤ 220 Nl/l·h, oder ≥ 190 Nl/l·h und ≤ 200 Nl/l·h betragen.

Erfindungsgemäß bevorzugt erstreckt sich die Temperaturzone C bis zu einem Umsatz des Acroleins von 50 bis 85 mol-% bzw. 60 bis 85 mol-%.

Der Arbeitsdruck kann dabei in beiden Reaktionsstufen sowohl unterhalb von Normaldruck (z. B. bis zu 0,5 bar) als auch oberhalb von Normaldruck liegen. Typischerweise wird der Arbeitsdruck in den beiden Reaktionsstufen bei Werten von 1 bis 5 bar, häufig 1 bis 3 bar liegen.

Normalerweise wird der Reaktionsdruck in keiner der beiden Reaktionsstufen 100 bar überschreiten.

In der Regel wird der auf den einfachen Durchgang der Festbettkatalysatorschüttung 1 bezogene Propenumsatz bei der beschriebenen Verfahrensweise ≥ 92 mol-% oder ≥ 94 mol-% betragen. Die Selektivität der Wertproduktbildung (Summe aus Acroleinbildung und Acrylsäurenebenproduktbildung) wird dabei bei in an sich bekannter Weise geeigneter Wahl (siehe in dieser Schrift empfohlene Katalysatoren) der Festbettkatalysatorschüttung 1 regelmäßig ≥ 92 mol-%, oder ≥ 94 mol-%, häufig ≥ 95 mol-%, oder ≥ 96 mol-% bzw. ≥ 97 mol-% betragen.

In der Regel wird beim vorstehend beschriebenen Verfahren die Acroleinbelastung der Festbettkatalysatorschüttung 2 ferner etwa 10 Nl/l · h, häufig etwa 20 bzw. 25 Nl/l · h unterhalb der Propenbelastung der Festbettkatalysatorschüttung 1 liegen. Dies ist primär darauf zurückzuführen, dass in der ersten Reaktionsstufe sowohl der Umsatz des Propens als auch die Selektivität der Acroleinbildung in der Regel keine 100 % erreichen. Ein zusätzlicher Beitrag kann von einer Sekundärsauerstoffzufuhr herrühren.

In der Regel wird der auf den einfachen Durchgang der Festbettkatalysatorschüttung 2 bezogene Acroleinumsatz beim vorstehend beschriebenen Verfahren ≥ 92 mol-%, oder ≥ 94 mol-%, oder ≥ 96 mol-%, oder ≥ 98 mol-% und häufig sogar ≥ 99 mol-% oder mehr betragen.

Bei in an sich bekannter Weise geeigneter Wahl der Festbettkatalysatorschüttungen 1 und 2 (siehe die in dieser Schrift gegebenen Katalysatorempfehlungen) wird die bei der vorstehend beschriebenen Verfahrensweise über beide Reaktionsstufen bilanzierte Selektivität der Acrylsäurebildung, bezogen auf umgesetztes Propen, bei Werten ≥ 83 mol-%, häufig bei ≥ 85 mol-%, oder ≥ 88 mol-%, oft bei ≥ 90 mol-%, oder ≥ 93 mol-% liegen.

Es sei auch noch erwähnt, dass eine erfindungsgemäße Partialoxidation und/oder Ammoxidation so durchgeführt werden kann, dass man über die Katalysatorbeschickung zunächst ein Reaktionsgasgemisch leitet, das keinen Sauerstoff enthält. In diesem Fall wird der für die Partialoxidation benötigte Sauerstoff als Gittersauerstoff zur Verfügung gestellt. In einem nachfolgenden Regenerierschritt mit einem Sauerstoff enthaltenden Gas (z.B. Luft, mit Sauerstoff angereicherte Luft oder an Sauerstoff entreicherte Luft) wird das Katalysatorfestbett regeneriert, um nachfolgend wiederum für ein an Sauerstoff freies Reaktionsgasgemisch zur Verfügung zu stehen u.s.w..

Ganz generell werden die Katalysatorbetten im Rahmen der erfindungsgemäßen Partialoxidation und/oder Ammoxidation so gewählt (z.B. durch Verdünnung mit Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperatur-unterschied ≤ 70°C, häufig 20 bis 70°C. Außerdem werden die Katalysatorbetten aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 11 06 598 ≤ 9°C, oder ≤ 7°C, oder ≤ 5°C, oder ≤ 3°C beträgt.

Unter der Belastung eines einen Reaktionsschritt katalysierenden Katalysatorbetts mit Reaktionsgas wird in dieser Schrift die Menge an Reaktionsgas in Normlitern (= Nl; das Volumen in Litern, das die entsprechende Reaktionsgasmenge bei Normalbedingungen (0°C, 1 bar) einnehmen würde) verstanden, die pro Stunde durch einen Liter Katalysatorbett geführt wird. Die Belastung kann in entsprechender Weise auch nur auf einen Bestandteil das Reaktionsgases bezogen sein.

Die Abtrennung des Zielproduktes P aus dem bei der heterogen katalysierten Gasphasen-Partialoxidation und/oder partiellen Ammoxidation anfallenden Produktgasgemisch (dem Gasgemisch 3) kann in wenigstens einem Abtrennschritt in an sich bekannter Weise erfolgen. Dabei wird normalerweise so vorgegangen, dass man das wenigstens eine Zielprodukt P in einem Grundabtrennschritt aus der Gasphase in die flüssige Phase überführt (vorab wird das Gasgemisch 3 gegebenenfalls abgekühlt). Dies kann z.B. durch partielle oder vollständige sowie gegebenenfalls fraktionierende Kondensation des Zielprodukts P (z.B. Acrolein und/oder Acrylsäure) und/oder durch Absorption des wenigstens einen Zielproduktes P aus dem Gasgemisch 3 in ein wässriges oder organisches Lösungsmittel erfolgen (d.h., fraktionierende Kondensation und/oder Absorption mit Wasser bzw. wässriger Lösungen können auch überlagert angewendet werden). Fraktionierende Kondensation und/oder Absorption in Wasser bzw. in wässrige Lösungen sind erfindungsgemäß als Grundabtrennschritt generell bevorzugt, da mit ihnen ein besonders ausgeprägter erfindungsgemäßer C₂-Auslass einhergeht. Im Fall von Acrylsäure und/oder Acrolein als Zielprodukt kommen als geeignete Absorptionsmittel beispielsweise Wasser, wässrige Lösungen niederer Carbonsäuren sowie hydrophobe organische Lösungsmittel wie Gemische aus Diphenyl und Diphenylether (z.B. Diphyl^{®}) oder Gemische aus Diphyl (75 bis 99,9 Gew.-%) und Dimethylphthalat (0,1 bis 25 Gew.-%) in Betracht. Im Fall von Acrylsäure wird man das das Zielprodukt P enthaltende Produktgasgemisch (das Gasgemisch 3) bevorzugt fraktionierend kondensieren. Beispielsweise kann die Grundabtrennung (insbesondere im Fall von Acrylsäure) wie in den nachfolgenden Schriften beschrieben erfolgen (vgl. z. B. EP-A 13 88 533, EP-A 13 88 532, DE-A 102 35 847, EP-A 792 867, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 854 129, US-A 4,317,926, DE-A 198 37 520, DE-A 196 06 877, DE-A 190 50 1325, DE-A 102 47 240, DE-A 197 40 253, EP-A 695 736, EP-A 982 287, EP-A 10 41 062, EP-A 11 71 46, DE-A 43 08 087, DE-A 43 35 172, DE-A 44 36 243, DE-A 199 24 532, DE-A 103 32 758 sowie DE-A 199 24 533). Eine Acrylsäureabtrennung kann auch wie in der EP-A 982 287, der EP-A 982 289, der DE-A 103 36 386, der DE-A 101 15 277, der DE-A 196 06 877, der DE-A 197 40 252, der DE-A 196 27 847, der EP-A 920 408, der EP-A 10 68 174, der EP-A 10 66 239, der EP-A 10 66 240, der WO 00/53560, der WO 00/53561, der DE-A 100 53 086 und der EP-A 982 288 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 2004/063138, WO 2004/035514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Mit der Überführung des wenigstens einen Zielproduktes P in die kondensierte Phase werden nun erfindungsgemäß wesentlich in der Regel auch im Rahmen der Zielreaktion gebildete Folgeprodukte der als Nebenkomponenten im Reaktionsgasgemisch enthaltenen C₂-KW, z.B. Essigsäure, Acetaldehyd und/oder Acetonitril in die kondensierte Phase überführt.

D.h., mit der Zielproduktabtrennung geht beim erfindungsgemäßen Verfahren ein C₂-Auslaß aus der Kreisfahrweise einher.

Beispielsweise durch Strippen mit Luft oder Stickstoff und/oder auf dem Weg der Desorption, lassen sich diese C₂-Komponenten vergleichsweise einfach aus der das wenigstens eine Zielprodukt P enthaltenden kondensierten Phase abtrennen. Gegebenenfalls kann zu dieser Abtrennung zusätzlich nachfolgend oder ausschließlich rektifiziert werden.

Gemeinsames Merkmal der vorgenannten Trennverfahren ist, dass z.B. am Kopf der z.B. jeweiligen trennwirksame Einbauten enthaltenden Trennkolonne, in deren unteren Teil das das wenigstens ein Zielprodukt enthaltende Produktgasgemisch, normalerweise nach vorheriger direkter und/oder indirekter Kühlung desselben, zugeführt wird, normalerweise ein Restgasstrom verbleibt, der im wesentlichen diejenigen Bestandteile des Produktgasgemischs (des Gasgemischs 3) enthält, deren Siedepunkt bei Normaldruck (1 bar) ≤ -30°C beträgt (d. h., die schwer kondensierbaren oder auch leicht flüchtigen Bestandteile).

Im unteren Teil der Trennkolonne fallen normalerweise die schwerer flüchtigen Bestandteile des Produktgasgemischs, einschließlich des jeweiligen wenigstens einen Zielprodukts P und der zum Zielprodukt P ähnlich flüchtigen Nebenkomponenten, in kondensierter Phase an.

Die Restgasbestandteile sind in erster Linie Propan, gegebenenfalls in der Partialoxidation und/oder Ammoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein.

Von diesem Hauptgasrest wird erfindungsgemäß bevorzugt wenigstens eine (vorzugsweise Restgaszusammensetzung aufweisende) Propan, molekularen Sauerstoff und gegebenenfalls nicht umgesetztes Propylen sowie Acrolein enthaltende Teilmenge (vorzugsweise die Gesamtmenge, gegebenenfalls aber auch nur die Hälfte, oder zwei Drittel, oder drei Viertel dieser Gesamtmenge) als ein Propan enthaltender Zufuhrstrom in den ersten Reaktionsschritt (die Dehydrierung) rückgeführt (Oxidationskreisgas). Restgasteilmengen können aber auch in eine oder in beide Stufen der Partialoxidation rückgeführt und/oder zum Zweck der Energieerzeugung verbrannt werden.

Bei der Aufarbeitung der kondensierten Phase (zum Zweck der Abtrennung des Zielproduktes) können weitere restliche Gase anfallen, da normalerweise versucht werden wird, die insgesamt im Produktgasgemisch enthaltene Menge an nicht umgesetztem Propan in den ersten Reaktionsschritt zurückzuführen und im Rahmen der Zielprodukt-abtrennung wiederzugewinnen. Diese enthalten in der Regel zwar noch Propan sowie gegebenenfalls Propylen und unter Umständen Acrolein, häufig jedoch keinen molekularen Sauerstoff mehr. Üblicherweise werden sie mit dem Hauptrestgas zu einem Gesamtrestgas vereint in die als Propylenquelle dienende Propandehydrierung und/oder Propanoxidehydrierung rückgeführt. Es ist aber auch eine separate Verwertung solcher weiteren restlichen Gase möglich.

Durch die vorzugsweise vollständige Rückführung des Gesamtrestgases kann so im kontinuierlichen Betrieb eine kontinuierliche Umsetzung von Propan zu Acrylsäure und/oder Acrolein bzw. zu den anderen Zielprodukten P erfolgen.

Wesentlich ist dabei, dass durch die beschriebene Rückführung in die als Propylenquelle dienende Dehydrierung in letzterer eine Überführung von Propan zu Propylen mit nahezu hundertprozentiger Selektivität erreichbar ist.

Die Vorteilhaftigkeit einer solchen Verfahrensweise ist dabei sowohl bei niederen (≤ 30 mol-%) als auch bei hohen (≥ 30 mol-%) Dehydrierumsätzen (bezogen auf einmaligen Durchgang von frischem vorab gereinigtem Propan durch die Dehydrierung) gegeben. Generell ist es bei einer solchen Rückführung von Oxidationskreisgas günstig, wenn der Wasserstoffgehalt im Reaktionsgasausgangsgemisch für eine klassische heterogen katalysierte Dehydrierung in einem wenigstens stöchiometrischen Verhältnis (bezüglich einer Sauerstoffverbrennung zu Wasser) zur über das Oxidationskreisgas in dieses Reaktionsgasausgangsgemisch zurückgeführten Sauerstoffmenge steht.

Natürlich können vor einer Rückführung von Oxidationskreisgas bzw. anderen Restgasen in den ersten Reaktionsschritt aus selbigen von Propan und Propylen verschiedene Bestandteile (diese können z.B. O₂, CO, CO₂, H₂O, N₂, Edelgase, niedere Aldehyde, Alkancarbonsäuren, Maleinsäureanhydrid, Benzaldehyd etc. sein) teilweise oder im wesentlichen vollständig abgetrennt werden. Mit einer solchen Abtrennung kann gleichfalls ein C₂-Auslass verbunden sein. Die Anwendung der erfindungsgemäßen Vorabtrennung kann selbiger jedoch einfacher und/oder kleiner gestaltet werden.

Beispielsweise kann eine solche C₃-KW Abtrennung, wie bereits beschrieben, durch Absorption mit nachfolgender Desorption und/oder Strippung (sowie Absorptionsmit-telwiederverwendung) in einem hochsiedenden hydrophoben organischen Lösungsmit-tel erfolgen. Weitere Trennmöglichkeiten sind Adsorption, Rektifikation, Membranverfahren und partielle Kondensation. Bevorzugt würden solche Trennverfahren bei erhöhtem Druck ausgeführt.

Bei Verwendung von Dehydrierkatalysatoren, die gegenüber Sauerstoff oder Sauerstoff enthaltenden Verbindungen empfindlich sind, wird man diese Oxygenate vor einer Rückführung von Kreisgas in den ersten Reaktionsschritt aus dem Kreisgas abtrennen.

Eine solche Sauerstoffabtrennung kann aber auch bewusst unterlassen werden, um im ersten Reaktionsschritt durch Teilverbrennung des Propans das Reaktionsgasgemisch auf die gewünschte Dehydriertemperatur zu heben.

Vorgenannte Kreisgasfahrweise ist in entsprechender Weise anwendbar, wenn die Partialoxidation eine partielle Ammoxidation von Propylen zu Acrylnitril bzw. eine Partialoxidation von Propylen zu Propylenoxid ist. Es ist auch dann entsprechend anwendbar, wenn in der Dehydrierung das Propan durch iso-Butan ersetzt wird und das dabei resultierende iso-Buten in entsprechender Weise in einer Partialoxidation zu Methacrolein und/oder Methacrylsäure partialoxidiert wird.

An dieser Stelle sei nochmals festgehalten, dass die Grundabtrennung von Acrylsäure aus einem erfindungsgemäß erhaltenen Acrylsäure als Zielprodukt enthaltenden, Produktgasgemisch (Gasgemisch 3) bevorzugt so erfolgt, dass man das zuvor gegebenenfalls durch direkte und/oder indirekte Kühlung abgekühlte Produktgasgemisch in einer trennwirksame Einbauten enthaltenden Kolonne unter Seitenabzug einer rohen Acrylsäure (z. B. in sich selbst) aufsteigend fraktionierend kondensiert und/oder mit Wasser bzw. wässriger Lösung absorbiert, wie es die WO 2004/035514 und die DE-A 102 43 625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische (z.B. um eine TNO Waschkolonne) oder um eine mechanische Waschkolonne. Im einzelnen kann der Beschreibung der WO 01/77056, der WO 03/041832 sowie der WO 03/041833 gefolgt werden. D. h., vorzugsweise wird verbliebene Mutterlauge in die fraktionierende Kondensation rückgeführt (vgl. auch EP-A 10 15 410). Der Nebenkomponentenauslass ist normalerweise unterhalb des Seitenabzugs der rohen Acrylsäure als purge-Strom.

Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf Basis von Poly-Na-Acrylat eignet. Auch sei noch festgehalten, dass ein Vorteil der erfindungsgemäßen Verfahrensweise grundsätzlich darin besteht, dass an allen Stellen dieser Schrift, einschließlich der Ausführungsbeispiele, dort wo mit Inertmaterial verdünnte Katalysatorbeschickungen beschrieben und/oder gefordert werden, die entsprechenden Katalysatoren bei der gleichen Bettlänge auch unverdünnt eingesetzt (verwendet) werden können.

Im übrigen gilt auch für das erfindungsgemäße Verfahren das in der DE-A 102 45 585 sowie in der DE-A 102 46 119 gesetzte Anforderungsprofil.

### Beispiele

### A) Rektifikative Vorabtrennung von Roh-Propan

Die Rektifikationskolonne weist einen Innendurchmesser von 50mm auf und enthält 40 äquidistant angeordnete Glockenböden (mit jeweils einer Glocke). Der Abstand zweier unmittelbar aufeinanderfolgender Böden beträgt 50mm. Die Rektifikationskolonne ist einschließlich der Glockenböden aus Edelstahl gefertigt. Außen ist die Rektifikationskolonne mit einer Einhüllenden aus Armaflex^{®} (25mm Dicke) wärmegedämmt. Die Beheizung des Kolonnensumpfes erfolgt mittels eines Naturumlaufverdampfers (Robert-Verdampfer), dem als Wärmeträger Heißwasser der Temperatur 85°C zugeführt wird. Die Zufuhr des Roh-Propan in die Rektifikationskolonne erfolgt auf den siebzehnten Glockenboden von unten.

Das Roh-Propan weist folgende Gehalte auf:

| | |
|---|---|
| 95 Gew.-% | Propan, |
| 0,10 Gew.-% | Propen, |
| 1,00 Gew.-% | n-Buten, |
| 3,28 Gew.-% | iso-Butan, |
| 0,60 Gew.-% | Ethan, |
| 86 gew.ppm | Ethylen und |
| 96 gew.ppm | 1-Buten. |

Bezogen auf enthaltenes Propan beträgt sein C₂-KW Gehalt 0,640 Gew.-%.

Die Zahl der theoretischen Trennstufen oberhalb der Zufuhrstelle beträgt 10. Die Zahl der theoretischen Trennstufen unterhalb der Zufuhrstelle beträgt 8.

Das Roh-Propan wird der Rektifikationskolonne in einer Menge von 300 kg/h zugeführt. Es weist einen Druck von 18 bar und eine Temperatur von 16°C auf. Über eine Drosselvorrichtung wird es in die Rektifikationskolonne entspannt. Die Sumpftemperatur beträgt 72°C bei einem Sumpfdruck (Obergrenze der Flüssigphase) von 14,10 bar. Der Kopfdruck beträgt 14,01 bar. Die Kopftemperatur liegt bei 41°C. Oberhalb des letzten Glockenbodens (von unten betrachtet) ist eine Kühlschlange in die Rektifikationskolonne eingebaut. Ihr wird Kühlwasser der Temperatur 20°C zugeführt. Über einen Kaminboden werden am Kopf der Rektifikationskolonne 0,82 kg/h an vorab gereinigtem Propan mit einer Temperatur von 40°C aus der Rektifikationskolonne flüssig herausgeführt.

Das vorab gereinigte Propan weist nachfolgende Gehalte auf:

| | |
|---|---|
| 99,20 Gew.-% | Propan, |
| 0,10 Gew.-% | Propen, |
| 9 gew.ppm | n-Butan, |
| 600 gew.ppm | iso-Butan, |
| 0,63 Gew.-% | Ethan, |
| 91 gew.ppm | Ethylen und |
| 1 gew.ppm | 1-Buten. |

Bezogen auf enthaltenes Propan beträgt sein C₂-KW Gehalt 0,644 Gew.-%. 0,53 kg/h des flüssig entnommenen vorab gereinigten Propan werden mit einer Temperatur von 40°C als Rücklaufflüssigkeit auf den obersten Boden der Rektifikationskolonne rückgeführt.

Aus dem Kolonnensumpf werden 15,9 g/h an Sumpfflüssigkeit kontinuierlich entnommen. Sie weist nachfolgende Gehalte auf:

| | |
|---|---|
| 61,00 Gew.-% | iso-Butan, |
| 18,81 Gew.-% | n-Butan, |
| 19,98 Gew.-% | Propan, |
| 269 gew.ppm | Propen und |
| 0,18 Gew.-% | 1-Buten. |

Aus der Sumpfflüssigkeit kann in einer zweiten Rektifikationskolonne wie in der DE-A 24 13 461 beschrieben weiteres vorab gereinigtes Propan abgetrennt werden. Die Sumpfflüssigkeit der vorgenannten Zusammensetzung eignet sich aber auch in hervorragender Weise als co-feed in einem Cracker für paraffinische Kohlenwasserstoffe.

### B) Zweistufige Partialoxidation von Propan zu Acrylsäure im Beisein von C₂-KW

I. Das Beschickungsgasgemisch für den ersten Festbettreaktor wies folgende Gehalte auf:

| | Vol.-% |
|---|---|
| Stickstoff | 46,69 |
| Sauerstoff | 11,84 |
| Propan | 32,53 |
| Propen | 6,81 |
| Ethan | 0,07 |
| n-Butan | 0,08 |
| iso-Butan | 0,12 |
| n-Butene | 0,05 |
| iso-Buten | 0,13 |
| Wasserstoff | 0,07 |
| Kohlendioxid | 0,61 |
| Wasser | 1,00 |
| Ethylen | 0,00 |
| Kohlenmonoxid | 0,00 |

Mit 2128 Nl/l•h und bei einem Eingangsdruck von 2,1 bar wurde der erste Festbettreaktor damit beschickt.
1. Erster Festbettreaktor für den Schritt der Partialoxidation des Propens (Propylens) zu Acrolein

| | |
|---|---|
| Verwendetes Wärmeaustauschmittel: | Salzschmelze, bestehend aus |
| | 53 Gew.-% Kaliumnitrat, |
| | 40 Gew.-% Nariumnitrit und |
| | 7 Gew.-% Natriumnitrat. |

| | |
|---|---|
| Abmessung des Kontaktrohres: | 4200 mm Gesamtlänge, |
| | 26 mm Innendurchmesser, |
| | 30 mm Außendurchmesser, |
| | 2 mm Wandstärke. |

Reaktor: Bestand aus einem doppelwandigen Zylinder aus Edelstahl (zylindrisches Führungsrohr, umgeben von einem zylindrischen Außenbehälter). Die Wanddicken betrugen überall 2 bis 5 mm.
Der Innendurchmesser des äußeren Zylinders betrug 168 mm. Der Innendurchmesser des Führungsrohres betrug ca. 60 mm.
Oben und unten war der doppelwandige Zylinder durch einen Deckel beziehungsweise Boden abgeschlossen.
Das Kontaktrohr war durch das zylindrische Führungsrohr geführt im zylindrischen Behälter so untergebracht, dass es am oberen bzw. unteren Ende desselben (abgedichtet) durch den Deckel bzw. Boden jeweils um 250 mm herausgeführt war.
Das Wärmeaustauschmittel war im zylindrischen Behälter eingeschlossen. Um über die gesamte im zylindrischen Behälter befindliche Kontaktrohrlänge (3700 mm) möglichst gleichmäßige thermische Randbedingungen an der Außenwand des Kontaktrohres zu gewährleisten, wurde das Wärmeaustauschmittel durch Einperlen von Stickstoff im zylindrischen Behälter umgewälzt.
Mittels des aufsteigenden Stickstoffs wurde das Wärmeaustauschmittel im zylindrischen Führungsrohr von unten nach oben befördert, um dann im Zwischenraum zwischen zylindrischem Führungsrohr und zylindrischem Außenbehälter wieder nach unten zu strömen (eine Umwälzung gleicher Güte kann auch durch Umpumpen (z.B. Propellerpumpen) erreicht werden). Durch eine auf den Außenmantel aufgebrachte elektrische Heizung konnte die Temperatur des Wärmeaustauschmittels auf das gewünschte Niveau geregelt werden. Im übrigen bestand Luftkühlung.
- Reaktorbeschickung:: Über den Reaktor betrachtet wurden Salzschmelze und Reaktionsgasgemisch im Gegenstrom geführt. Das Reaktionsgasgemisch trat oben in den Reaktor ein. Es wurde jeweils mit einer Temperatur von 250°C ins Reaktionsrohr geführt.
Die Salzschmelze trat unten mit einer Temperatur T^{ein} = 320°C in das zylindrische Führungsrohr ein und oben mit einer Temperatur T^{aus} aus dem zylindrischen Führungsrohr aus. Der Unterschied zwischen T^{ein} und T^{aus} betrug etwa 2°C. T^{mittel} = (T^{ein} + T^{aus})/2.
- Kontaktrohrbeschickung: (von oben nach unten): Abschnitt A: 50 cm Länge Vorschüttung aus Steatit-Ringen (Steatit C 220 der Fa. Ceram.Tec) der Geometrie 7 mm x 7 mm x 4 mm (Außendurchmesser x Länge x Innendurchmesser).
Abschnitt B: 100 cm Länge Kontaktrohrbeschickung mit einem homogenen Gemisch aus 30 Gew.-% an Steatit-Ringen (Steatit C 220 der Fa. CeramTec) der Geometrie 5 mm x 3 mm x 2 mm (Außendurchmesser x Länge x Innendurchmessser) und 70 Gew.-% Vollkatalysator aus Abschnitt C.
Abschnitt C: 170 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957.
Abschnitt D: 50 cm Länge Nachschüttung aus Steatit-Ringen (Steatit C 220 der Fa. CeramTec) der Geometrie 7 mm x 7 mm x 4 mm (Außendurchmesser x Länge x Innendurchmesser).

2. Zwischenkühlung und Sauerstoffzwischeneinspeisung
Das den ersten Festbettreaktor verlassende Produktgasgemisch wurde zum Zweck der Zwischenkühlung (indirekt mittels Luft) durch ein Verbindungsrohr (Länge = 400 mm, Innendurchmesser = 26 mm, Wanddicke = 2 mm, Material = Edelstahl) geführt, das, auf einer Länge von 200 mm zentriert untergebracht, mit einer Inertschüttung aus Steatitkugeln (Steatit der Fa. CeramTec) des Durchmessers 5 bis 6 mm beschickt und unmittelbar an das Kontraktrohr des ersten Festbettreaktors angeflanscht war.
Das Gasgemisch trat mit einer Temperatur von mehr als 310°C in das Verbindungsrohr ein und verließ es mit einer Temperatur von etwa 140°C. Anschließend wurden dem Gasgemisch als Sauerstoffquelle 290 Nl/h an komprimierter Luft zugemischt.
Das dabei resultierende, an einem statischen Mischer vermischte, Beschickungsgasgemisch wurde mit einer Temperatur von 220°C dem Festbettreaktor für den Schritt der Partialoxidation des Acroleins zu Acrylsäure zugeführt.
3. Zweiter Festbettreaktor für den Schritt der Partialoxidation des Acroleins zu Acrylsäure
Es wurde ein Festbettreaktor verwendet, der mit jenem für den ersten Schritt baugleich war. Salzschmelze und Reaktionsgasgemisch wurden über den Reaktor betrachtet im Gleichstrom geführt. Die Salzschmelze trat unten ein, das Reaktionsgasgemisch ebenfalls.
Die Kontaktrohrbeschickung (von unten nach oben) war:
Abschnitt A: 20 cm Länge
Vorschüttung aus Steatit-Ringen (Steatit C 220 der Fa. CeramTec) der Geometrie 7 mm x 7 mm x 4 mm
(Außendurchmesser x Länge x Innendurchmesser).

Abschnitt B: 100 cm Länge
Katalysatorbeschickung mit einem homogen Gemisch aus 30 Gew.-% an Steatit-Ringen (Steatit C 220 der Fa. CeramTec) der Geometrie 7 mm x 3 mm x 4 mm
(Außendurchmesser x Länge x Innendurchmesser) und 70 Gew.-% Schalenkatalysator aus Abschnitt C.

Abschnitt C: 200 cm Länge
Katalysatorbeschickung mit ringförmigem
(7 mm x 3 mm x 4 mm = Außerdurchmesser x Länge x Innendurchmesser) Schalenkatalysator gemäß Herstellungsbeispiel 5 der DE-A 10046928 (an dieser Stelle können auch dazu analoge und in entsprechender Weise hergestellte Schalenkatalysatoren eingesetzt werden, deren Aktivmasse jedoch eine Stöchiometrie von Mo₁₂V_{2,8}W_{1,2}Cu_{2,4}Oₓ oder von Mo₁₂V_{3,5}W_{1,3}Cu_{2,4}Oₓ aufweist).

Abschnitt D: 50 cm Länge
Nachschüttung aus Steatit-Ringen (Steatit C 220 der Fa. CeramTec) der Geometrie 7 mm x 7 mm x 4 mm
(Außendurchmesser x Länge x Innendurchmesser).

Der zweite Reaktor wurde mit ca. 3850 g/h an Beschickungsgasgemisch belastet. T^{mittel} ist wie für den ersten Festbettreaktor definiert und betrug 274°C.
Der Propenumsatz im ersten Reaktor betrug 97,7 mol-% und der Acroleinumsatz im zweiten Reaktor betrug 99,4 mol-%.
Die Gehalte des den zweiten Festbettreaktor mit einer Temperatur von 283°C und einem Druck von 1,8 bar verlassenden Produktgasgemisches waren:

| | Vol.-% |
|---|---|
| Stickstoff | 52,87 |
| Sauerstoff | 3,03 |
| Propan | 27,48 |
| Propen | 0,14 |
| Methan | 0 |
| Ethan | 0,07 |
| n-Butan | 0,08 |
| iso-Butan | 0,34 |
| n-Buten | 0 |
| iso-Buten | 0 |
| 1,3-Butadien | 0 |
| Wasserstoff | 0,03 |
| Kohlenmonoxid | 0,42 |
| Kohlendioxid | 1,85 |
| Wasser | 7,92 |
| Acrolein | 0,03 |
| Acrylsäure | 5,3 |
| Essigsäure | 0,18 |
| Ameisensäure | 0,01 |
| Formaldehyd | 0,17 |
| Benzaldehyd | 0 |
| Maleinsäureanhydrid | 0,04 |
| Ethen | 0,02 |

II. Das Beispiel I. wird mit folgenden Unterschieden wiederholt:
Im Beschickungsgasgemisch der ersten Reaktionsstufe sind nur 44,6 Vol.-% Stickstoff, aber 2,1 Vol.-% Ethan enthalten.
Das den zweiten Festbettreaktor verlassende Produktgasgemisch enthält jetzt 0,29 Vol-% Essigsäure.
III. Das Beispiel I. wird mit folgenden Unterschieden wiederholt:
Im Beschickungsgasgemisch der ersten Reaktionsstufe sind nur 44,7 Vol.-% Stickstoff, aber 2,0 Vol.-% Ethylen enthalten.
Das den zweiten Festbettreaktor verlassende Produktgasgemisch enthält jetzt 0,38 Vol.-% Essigsäure.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zur Herstellung wenigstens eines Zielproduktes P durch partielle Oxidation und/oder Ammoxidation von Propylen, bei dem man
a) vorab gereinigtes Propan in einem ersten Reaktionsschritt im Beisein und/oder unter Ausschluß von molekularem Sauerstoff wenigstens einer Dehydrierung aus der Gruppe umfassend die homogene Dehydrierung, die heterogen katalysierte Dehydrierung, die homogene Oxidehydrierung und die heterogen katalysierte Oxidehydrierung unterwirft, wobei ein nicht umgesetztes Propan und gebildetes Propylen enthaltendes Gasgemisch 1 erhalten wird, und
b) gegebenenfalls aus der Gesamtmenge oder aus einer Teilmenge des Gasgemischs 1, von den darin enthaltenen, von Propan und Propylen verschiedenen, Bestandteilen eine Teil- oder die Gesamtmenge abtrennt und/oder in andere Verbindungen wandelt, und dabei ein Propan und Propylen enthaltendes Gasgemisch 1' erzeugt, und in wenigstens einem weiteren Reaktionsschritt
c) Gasgemisch 1, oder Gasgemisch 1' oder ein Gemisch aus gebildetem Gasgemisch 1' und verbliebenem Gasgemisch 1 als Bestandteil eines Gasgemisches 2 einer heterogen katalysierten Gasphasen-Partialoxidation und/oder partiellen Gasphasen-Ammoxidation von im Gasgemisch 1 und/oder Gasgemisch 1' enthaltenem Propylen unterwirft, wobei ein das wenigstens eine Zielprodukt P enthaltendes Gasgemisch 3 erhalten wird,
d) in wenigstens einem Abtrennschritt aus dem Gasgemisch 3 Zielprodukt abtrennt und von dem dabei verbleibenden Restgas wenigstens Propan in den ersten Reaktionsschritt zurückführt, **dadurch gekennzeichnet,**
**dass** das vorab gereinigte Propan aus einem Roh-Propan, das
≥90 Gew.-% Propan,
≤99 Gew.-% Propan und Propylen,
≥100 gew.ppm C₂-Kohlenwasserstoffe und
≥100 gew.ppm C₄-Kohlenwasserstoffe
enthält, mit der Maßgabe erzeugt wird, dass man das Roh-Propan in eine Rektifikationskolonne führt, und oberhalb der Zufuhrstelle das gereinigte Propan mit der Maßgabe entnimmt, dass der auf das enthaltene Propan bezogene Gehalt an C₂-Kohlenwasserstoffen in Gew.-% im gereinigten Propan nicht weniger als 80% und nicht mehr als 110% des entsprechenden Gehaltes im Roh-Propan und der auf das enthaltene Propan bezogene Gehalt an C₄-Kohlenwasserstoffen in Gew.-% im gereinigten Propan höchstens 50% des entsprechenden Gehaltes im Roh-Propan beträgt.

2. Verfahren nach Anspruch 1, **dadurch gekennzeichnet, dass** das Roh-Propan ≥ 200 gew.ppm C₂-Kohlenwasserstoffe enthält.

3. Verfahren nach Anspruch 1, **dadurch gekennzeichnet, dass** das Roh-Propan ≥ 500 gew.ppm C₂-Kohlenwasserstoffe enthält.

4. Verfahren nach Anspruch 1, **dadurch gekennzeichnet, dass** das Roh-Propan ≥ 800 gew.ppm C₂-Kohlenwasserstoffe enthält.

5. Verfahren nach Anspruch 1, **dadurch gekennzeichnet, dass** das Roh-Propan ≥ 1000 gew.ppm C₂-Kohlenwasserstoffe enthält.

6. Verfahren nach einem der Ansprüche 1 bis 5, **dadurch gekennzeichnet, dass** die im Roh-Propan enthaltenen C₂-Kohlenwasserstoffe zu wenigstens 90 Gew.-% aus Ethan und Ethylen bestehen.

7. Verfahren nach einem der Ansprüche 1 bis 5, **dadurch gekennzeichnet, dass** die im Roh-Propan enthaltenen C₂-Kohlenwasserstoffe zu wenigstens 94 Gew.-% aus Ethan und Ethylen bestehen.

8. Verfahren nach einem der Ansprüche 1 bis 7, **dadurch gekennzeichnet, dass** die im Roh-Propan enthaltenen C₂-Kohlenwasserstoffe zu wenigstens 50 Gew.-% aus Ethan bestehen.

9. Verfahren nach einem der Ansprüche 1 bis 7, **dadurch gekennzeichnet, dass** die im Roh-Propan enthaltenen C₂₋Kohlenwasserstoffe zu wenigstens 70 Gew.-% aus Ethan bestehen.

10. Verfahren nach einem der Ansprüche 1 bis 7, **dadurch gekennzeichnet, dass** die im Roh-Propan enthaltenen C₂-Kohlenwasserstoffe zu wenigstens 90 Gew.-% aus Ethan bestehen.

11. Verfahren nach einem der Ansprüche 1 bis 10, **dadurch gekennzeichnet, dass** das Roh-Propan ≥ 200 gew.ppm C₄-Kohlenwasserstoffe enthält.

12. Verfahren nach einem der Ansprüche 1 bis 10, **dadurch gekennzeichnet, dass** das Roh-Propan ≥ 500 gew.ppm C₄-Kohlenwasserstoffe enthält.

13. Verfahren nach einem der Ansprüche 1 bis 10, **dadurch gekennzeichnet, dass** das Roh-Propan ≥ 1000 gew.ppm C₄-Kohlenwasserstoffe enthält.

14. Verfahren nach einem der Ansprüche 1 bis 13, **dadurch gekennzeichnet, dass** die im Roh-Propan enthaltenen C₄-Kohlenwasserstoffe zu wenigstens 80 Gew.-% aus Butan bestehen.

15. Verfahren nach einem der Ansprüche 1 bis 13, **dadurch gekennzeichnet, dass** die im Roh-Propan enthaltenen C₄-Kohlenwasserstoffe zu wenigstens 90 Gew.-% aus Butan bestehen.

16. Verfahren nach Anspruch 15, **dadurch gekennzeichnet, dass** das im Roh-Propan enthaltene Butan zu ≥ 50 Gew.-% iso-Butan ist.

17. Verfahren nach Anspruch 16, **dadurch gekennzeichnet, dass** das im Roh-Propan enthaltene Butan zu ≥ 70 Gew.-% iso-Butan ist.

18. Verfahren nach einem der Ansprüche 1 bis 17, **dadurch gekennzeichnet, dass** der auf das enthaltene Propan bezogene Gehalt an C₄-Kohlenwasserstoffen in Gew.-% im vorab gereinigten Propan höchstens 30% des entsprechenden Gehaltes im Roh-Propan beträgt.

19. Verfahren nach einem der Ansprüche 1 bis 17, **dadurch gekennzeichnet, dass** der auf das enthaltene Propan bezogene Gehalt an C₄-Kohlenwasserstoffen in Gew.-% im vorab gereinigten Propan höchstens 10% des entsprechenden Gehaltes im Roh-Propan beträgt.

20. Verfahren nach einem der Ansprüche 1 bis 17, **dadurch gekennzeichnet, dass** der auf das enthaltene Propan bezogene Gehalt an C₄-Kohlenwasserstoffen in Gew.-% im vorab gereinigten Propan höchstens 1% des entsprechenden Gehaltes im Roh-Propan beträgt.

21. Verfahren nach einem der Ansprüche 1 bis 20, **dadurch gekennzeichnet, dass** der auf das enthaltene Propan bezogene Gehalt an C₂-Kohlenwasserstoffen in Gew.-% im vorab gereinigten Propan nicht weniger als 85% des entsprechenden Gehaltes im Roh-Propan beträgt.

22. Verfahren nach einem der Ansprüche 1 bis 20, **dadurch gekennzeichnet, dass** der auf das enthaltene Propan bezogene Gehalt an C₂-Kohlenwasserstoffen in Gew.-% im vorab gereinigten Propan nicht weniger als 90% des entsprechenden Gehaltes im Roh-Propan beträgt.

23. Verfahren nach einem der Ansprüche 1 bis 20, **dadurch gekennzeichnet, dass** der auf das enthaltene Propan bezogene Gehalt an C₂-Kohlenwasserstoffen in Gew.-% im vorab gereinigten Propan nicht weniger als 95% des entsprechenden Gehaltes im Roh-Propan beträgt.

24. Verfahren nach einem der Ansprüche 1 bis 20, **dadurch gekennzeichnet, dass** der auf das enthaltene Propan bezogene Gehalt an C₂-Kohlenwasserstoffen in Gew.-% im vorab gereinigten Propan nicht weniger als 100% des entsprechenden Gehaltes im Roh-Propan beträgt.

25. Verfahren nach einem der Ansprüche 1 bis 20, **dadurch gekennzeichnet, dass** der auf das enthaltene Propan bezogene Gehalt an C₂-Kohlenwasserstoffen in Gew.-% im vorab gereinigten Propan mehr als 100% des entsprechenden Gehaltes im Roh-Propan beträgt.

26. Verfahren nach einem der Ansprüche 1 bis 25, **dadurch gekennzeichnet, dass** der Gehalt des vorab gereinigten Propan an iso-Butan ≤ 1000 gew.ppm beträgt.

27. Verfahren nach einem der Ansprüche 1 bis 25, **dadurch gekennzeichnet, dass** der Gehalt des vorab gereinigten Propan an iso-Butan ≤ 600 gew.ppm beträgt.

28. Verfahren nach einem der Ansprüche 1 bis 25, **dadurch gekennzeichnet, dass** der Gehalt des vorab gereinigten Propan an iso-Butan ≤ 100 gew.ppm beträgt.

29. Verfahren nach einem der Ansprüche 1 bis 28, **dadurch gekennzeichnet, dass** die Rektifikationskolonne als trennwirksame Einbauten Stoffaustauschböden enthält.

30. Verfahren nach einem der Ansprüche 1 bis 29, **dadurch gekennzeichnet, dass** die Rektifikationskolonne als trennwirksame Einbauten Ventilböden enthält.

31. Verfahren nach einem der Ansprüche 1 bis 30, **dadurch gekennzeichnet, dass** die Rektifrkationskolonne 5 bis 25 theoretische Trennstufen aufweist.

32. Verfahren nach einem der Ansprüche 1 bis 31, **dadurch gekennzeichnet, dass** der Kopfdruck in der Rektifikationskolonne ≥ 5 bar und ≤ 25 bar beträgt.

33. Verfahren nach einem der Ansprüche 1 bis 32, **dadurch gekennzeichnet, dass** die Sumpftemperatur in der Rektifikationskolonne ≤ 100°C beträgt.

34. Verfahren nach einem der Ansprüche 1 bis 33, **dadurch gekennzeichnet, dass** die Sumpftemperatur in der Rektifikationskolonne 40 bis 90°C beträgt.

35. Verfahren nach einem der Ansprüche 1 bis 34, **dadurch gekennzeichnet, dass** die Zufuhr des Roh-Propans in die Rektifikationskolonne so erfolgt, dass die Anzahl der theoretischen Trennstufen oberhalb der Zufuhrstelle größer ist als die Anzahl der theoretischen Trennstufen unterhalb der Zufuhrstelle.

36. Verfahren nach einem der Ansprüche 1 bis 35, **dadurch gekennzeichnet, dass** das vorab gereinigte Propan am Kopf der Rektifikationskolonne entnommen wird.

37. Verfahren nach einem der Ansprüche 1 bis 36, **dadurch gekennzeichnet, dass** das vorab gereinigte Propan der Rektifikationskolonne gasförmig entnommen wird.

38. Verfahren nach einem der Ansprüche 1 bis 37, **dadurch gekennzeichnet, dass** die Rektifikationskolonne einen mit Wasser gekühlten Kopfkondensator aufweist.

39. Verfahren nach Anspruch 38, **dadurch gekennzeichnet, dass** das .Wasser dem Kopfkondensator mit einer Temperatur von ≥ 0°C und ≤ 40°C zugeführt wird.

40. Verfahren nach einem der Ansprüche 1 bis 39, **dadurch gekennzeichnet, dass** das vorab gereinigte Propan der Rektifikationskolonne flüssig entnommen wird.

41. Verfahren nach einem der Ansprüche 1 bis 40, **dadurch gekennzeichnet, dass** das vorab gereinigte Propan zu wenigstens 99 Gew.-% aus Propan, Propylen, Ethan und Ethylen besteht.

42. Verfahren nach einem der Ansprüche 1 bis 41, **dadurch gekennzeichnet, dass** alle Reaktionsschritte in einer Reaktionszone und an einer in selbiger befindlichen Katalysatorbeschickung durchgeführt werden.

43. Verfahren nach einem der Ansprüche 1 bis 42, **dadurch gekennzeichnet, dass** der erste Reaktionsschritt eine heterogen katalysierte Oxidehydrierung ist.

44. Verfahren nach einem der Ansprüche 1 bis 42, **dadurch gekennzeichnet, dass** der erste Reaktionsschritt eine heterogen katalysierte Dehydrierung ist.

45. Verfahren nach Anspruch 44, **dadurch gekennzeichnet, dass** die heterogen katalysierte Dehydrierung adiabat durchgeführt wird.

46. Verfahren nach Anspruch 44, **dadurch gekennzeichnet, dass** die heterogen katalysierte Dehydrierung autotherm durchgeführt wird.

47. Verfahren nach Anspruch 44, **dadurch gekennzeichnet, dass** die heterogen katalysierte Dehydrierung in einem Hordenreaktor durchgeführt wird.

48. Verfahren nach einem der Ansprüche 1 bis 47, **dadurch gekennzeichnet, dass** das Gasgemisch 2 > 0 bis 30 Vol.-% Wasserdampf enthält.

49. Verfahren nach einem der Ansprüche 1 bis 48, **dadurch gekennzeichnet, dass** in wenigstens einen Reaktionsschritt der heterogen katalysierten Gasphasen-Partialoxidation und/oder partiellen Gasphasen-Ammoxidation des Propylens eine unmittelbare Zufuhr von vorab gereinigtem Propan erfolgt.

50. Verfahren nach Anspruch 49, **dadurch gekennzeichnet, dass** wenigstens 25 Gew.-% des Gesamtbedarfs an vorab gereinigtem Propan wenigstens einem Reaktionsschritt der heterogen katalysierten Gasphasen-Partialoxidation und/oder partiellen Gasphasen-Ammoxidation des Propylens unmittelbar zuge-führt wird.

51. Verfahren nach Anspruch 49, **dadurch gekennzeichnet, dass** wenigstens 50 Gew.-% des Gesamtbedarfs an vorab gereinigtem Propan wenigstens einem Reaktionsschritt der heterogen katalysierten Gasphasen-Partialoxidation und/oder partiellen Gasphasen-Ammoxidation des Propylens unmittelbar zuge-führt wird.

52. Verfahren nach einem der Ansprüche 1 bis 51, **dadurch gekennzeichnet, dass** das Zielprodukt P Acrolein, Acrylnitril, Acrylsäure und/oder Propylenoxid ist.

53. Verfahren nach einem der Ansprüche 1 bis 52, **dadurch gekennzeichnet, dass** das Gasgemisch 2 einer zweistufigen heterogen katalysierten Partialoxidation des in ihm enthaltenen Propylen zu Acrylsäure unterworfen wird.

54. Verfahren nach Anspruch 53, **dadurch gekennzeichnet, dass** in der ersten Parti-aloxidationsstufe Propylen zu Acrolein mit der Maßgabe partialoxidiert wird, dass die Partialoxidation an in einem Katalysatorbett befindlichen Katalysatoren erfolgt, deren Aktivmasse wenigstens ein Multimetalloxid der allgemeinen Formel (IV),
Mo₁₂BiₐFe_{b}X¹_{c}X²_{d}X³ₑX⁴_{f}Oₙ (IV),
mit
X¹ = Nickel und/oder Kobalt,
X² = Thallium, ein Alkalimetall und/oder ein Edelkalimetall,
X³ = Zink, Phosphor, Bor, Antimon, Zinn, Cer, Blei und/oder Wolfram,
X⁴ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in IV bestimmt wird,
ist.

55. Verfahren nach Anspruch 53 oder 54, **dadurch gekennzeichnet, dass** in der zweiten Partialoxidationsstufe Acrolein zu Acrylsäure mit der Maßgabe partialoxidiert wird, dass die Partialoxidation an in einem Katalysatorbett befindlichen Katalysatoren erfolgt, deren Aktivmasse wenigstens ein Multimetalloxid der allgemeinen Formel (VII),
Mo₁₂VₐX¹_{b}X²cX³_{d}X⁴ₑX⁵_{f}X⁶_{g}Oₙ (VII),
mit
X¹ = W, Nb, Ta, Cr und/oder Ce,
X² = Cu_{,} Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, AI, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elementen in VII bestimmt wird,
ist.

56. Verfahren nach einem der Ansprüche 53 bis 55, **dadurch gekennzeichnet, dass** die Propylenbelastung des Katalysatorbetts der ersten Reaktionsstufe . ≥ 120 Nl/l•h beträgt.

57. Verfahren nach einem der Ansprüche 53 bis 56, **dadurch gekennzeichnet, dass** die zweite Reaktionsstufe mit einem Gasgemisch beschickt wird, das enthält:
| | |
|---|---|
| 4,5 bis 8 Vol.-% | Acrolein, |
| 2,25 bis 9 Vol.-% | molekularer Sauerstoff, |
| 6 bis 30 Vol.-% | Propan, |
| 32 bis 72 Vol.-% | molekularer Stickstoff und |
| 5 bis 30 Vol.-% | Wasserdampf. |

58. Verfahren nach einem der Ansprüche 53 bis 57, **dadurch gekennzeichnet, dass** die Acroleinbelastung des Katalysatorfestbetts der zweiten Reaktionsstufe ≥ 110 Nl/l•h beträgt.

59. Verfahren nach einem der Ansprüche 53 bis 58, **dadurch gekennzeichnet, dass** die Reaktionstemperatur in der ersten Reaktionsstufe 300 bis 450°C beträgt.

60. Verfahren nach einem der Ansprüche 53 bis 59, **dadurch gekennzeichnet, dass** die Reaktionstemperatur in der zweiten Reaktionsstufe 200 bis 370°C beträgt.

61. Verfahren nach einem der Ansprüche 1 bis 60, **dadurch gekennzeichnet, dass** der wenigstens eine Abtrennschritt einen Grundabtrennschritt umfasst, bei dem das wenigstens eine Zielprodukt P aus dem Gasgemisch 3 in die flüssige Phase überführt wird.

62. Verfahren nach Anspruch 61, **dadurch gekennzeichnet, dass** der Grundabtrennschritt eine fraktionierende Kondensation des Gasgemischs 3 und/oder eine Absorption des Zielprodukts P aus dem Gasgemisch 3 in Wasser oder in wässrige Lösung umfasst.

63. Verfahren nach Anspruch 62, **dadurch gekennzeichnet, dass** das Zielprodukt P Acrylsäure ist und der Grundabtrennschritt in einer trennwirksame Einbauten enthaltenden Kolonne unter Seitenabzug einer rohen Acrylsäure durchgeführt und die rohe Acrylsäure einer Suspensionskristallisation unterworfen wird.

64. Verfahren nach Anspruch 63, **dadurch gekennzeichnet, dass** das bei der Suspensionskristallisation anfallende Acrylsäuresuspensionskristallisat mittels einer Waschkolonne von verbliebener Mutterlauge abgetrennt wird.

65. Verfahren nach Anspruch 64, **dadurch gekennzeichnet, dass** die Waschkolonne eine solche mit erzwungenem Transport des Kristallbetts ist.

66. Verfahren nach Anspruch 65, **dadurch gekennzeichnet, dass** die Waschkolonne eine hydraulische Waschkolonne ist.

67. Verfahren nach Anspruch 66, **dadurch gekennzeichnet, dass** als Waschflüssigkeit die Schmelze von in der Waschkolonne vorab abgetrennten Acrylsäurekristallen verwendet wird.

68. Verfahren nach einem der Ansprüche 64 bis 67, **dadurch gekennzeichnet, dass** das abgetrennte Acrylsäuresuspensionskristallisat ausgeschmolzen wird und sich wenigstens ein Verfahren der radikalen Polymerisation anschließt, in welchem aufgeschmolzenes Acrylsäurekristallisat zur Herstellung von Polymerisaten radikalisch einpolymierisiert wird.

69. Verfahren nach einem der Ansprüche 1 bis 68, **dadurch gekennzeichnet, dass** der Rektifikationskolonne kontinuierlich Sumpfflüssigkeit entnommen und als co-feed einem Cracker für paraffinische Kohlenwasserstoffe zugeführt wird.

70. Verfahren nach einem der Ansprüche 1 bis 68, **dadurch gekennzeichnet, dass** der Rektifikationskolonne kontinuierlich Sumpfflüssigkeit entnommen und rektifikativ weiterbehandelt wird.

## Claims

1. A process for preparing at least one target product P by partial oxidation and/or ammoxidation of propylene, by
a) subjecting prepurified propane, in a first reaction step in the presence of and/or with exclusion of molecular oxygen, to at least one dehydrogenation from the group comprising homogeneous dehydrogenation, heterogeneously catalyzed dehydrogenation, homogeneous oxydehydrogenation and heterogeneously catalyzed oxydehydrogenation to obtain a gas mixture 1 comprising unconverted propane and formed propylene, and
b) optionally removing a portion or the entirety of the constituents other than propane and propylene present in the entirety or in a portion of gas mixture 1 therefrom and/or converting them to other compounds to obtain a gas mixture 1' comprising propane and propylene, and, in at least one further reaction step,
c) subjecting gas mixture 1 or gas mixture 1' or a mixture of formed gas mixture 1' and remaining gas mixture 1, as a constituent of a gas mixture 2, to a heterogeneously catalyzed gas phase partial oxidation and/or partial gas phase ammoxidation of propylene present in gas mixture 1 and/or gas mixture 1' to obtain a gas mixture 3 comprising at least one target product P,
d) removing target product from gas mixture 3 in at least one removal step and, of the remaining residual gas, recycling at least propane into the first reaction step, wherein
the prepurified propane is obtained from crude propane which comprises
≥ 90% by weight of propane,
≤ 99% by weight of propane and propylene,
≥ 100 ppm by weight of C₂ hydrocarbons and
≥ 100 ppm by weight of C₄ hydrocarbons,
with the proviso that the crude propane is conducted into a rectification column and the purified propane is removed above the feed point with the proviso that the content of C₂ hydrocarbons based on propane present in % by weight in the purified propane is not less than 80% and not more than 110% by weight of the corresponding content in the crude propane, and the content of C₄ hydrocarbons based on propane present in % by weight in the purified propane is at most 50% by weight of the corresponding content in the crude propane.

2. The process according to claim 1, wherein the crude propane comprises ≥ 200 ppm by weight of C₂ hydrocarbons.

3. The process according to claim 1, wherein the crude propane comprises ≥ 500 ppm by weight of C₂ hydrocarbons.

4. The process according to claim 1, wherein the crude propane comprises ≥ 800 ppm by weight of C₂ hydrocarbons.

5. The process according to claim 1, wherein the crude propane comprises ≥ 1000 ppm by weight of C₂ hydrocarbons.

6. The process according to any of claims 1 to 5, wherein the C₂ hydrocarbons present in the crude propane consist to an extent of at least 90% by weight of ethane and ethylene.

7. The process according to any of claims 1 to 5, wherein the C₂ hydrocarbons present in the crude propane consist to an extent of at least 94% by weight of ethane and ethylene.

8. The process according to any of claims 1 to 7, wherein the C₂ hydrocarbons present in the crude propane consist to an extent of at least 50% by weight of ethane.

9. The process according to any of claims 1 to 7, wherein the C₂ hydrocarbons present in the crude propane consist to an extent of at least 70% by weight of ethane.

10. The process according to any of claims 1 to 7, wherein the C₂ hydrocarbons present in the crude propane consist to an extent of at least 90% by weight of ethane.

11. The process according to any of claims 1 to 10, wherein the crude propane comprises ≥ 200 by weight of C₄ hydrocarbons.

12. The process according to any of claims 1 to 10, wherein the crude propane comprises ≥ 500 by weight of C₄ hydrocarbons.

13. The process according to any of claims 1 to 10, wherein the crude propane comprises ≥ 1000 by weight of C₄ hydrocarbons.

14. The process according to any of claims 1 to 13, wherein the C₄ hydrocarbons present in the crude propane consist to an extent of at least 80% by weight of butane.

15. The process according to any of claims 1 to 13, wherein the C₄ hydrocarbons present in the crude propane consist to an extent of at least 90% by weight of butane.

16. The process according to claim 15, wherein ≥ 50% by weight of the butane present in the crude propane is isobutane.

17. The process according to claim 16, wherein ≥ 70% by weight of the butane present in the crude propane is isobutane.

18. The process according to any of claims 1 to 17, wherein the content of C₄ hydrocarbons based on propane present in % by weight in the prepurified propane is at most 30% of the corresponding content in the crude propane.

19. The process according to any of claims 1 to 17, wherein the content of C₄ hydrocarbons based on propane present in % by weight in the prepurified propane is at most 10% of the corresponding content in the crude propane.

20. The process according to any of claims 1 to 17, wherein the content of C₄ hydrocarbons based on propane present in % by weight in the prepurified propane is at most 1% of the corresponding content in the crude propane.

21. The process according to any of claims 1 to 20, wherein the content based on propane present of C₂ hydrocarbons in % by weight in the prepurified propane is not less than 85% by weight of the corresponding content in the crude propane.

22. The process according to any of claims 1 to 20, wherein the content based on propane present of C₂ hydrocarbons in % by weight in the prepurified propane is not less than 90% by weight of the corresponding content in the crude propane.

23. The process according to any of claims 1 to 20, wherein the content based on propane present of C₂ hydrocarbons in % by weight in the prepurified propane is not less than 95% by weight of the corresponding content in the crude propane.

24. The process according to any of claims 1 to 20, wherein the content based on propane present of C₂ hydrocarbons in % by weight in the prepurified propane is not less than 100% by weight of the corresponding content in the crude propane.

25. The process according to any of claims 1 to 20, wherein the content based on propane present of C₂ hydrocarbons in % by weight in the prepurified propane is more than 100% by weight of the corresponding content in the crude propane.

26. The process according to any of claims 1 to 25, wherein the content of isobutane in the prepurified propane is ≤ 1000 ppm by weight.

27. The process according to any of claims 1 to 25, wherein the content of isobutane in the prepurified propane is ≤ 600 ppm by weight.

28. The process according to any of claims 1 to 25, wherein the content of isobutane in the prepurified propane is ≤ 100 ppm by weight.

29. The process according to any of claims 1 to 28, wherein the rectification column comprises, as separating internals, mass transfer trays.

30. The process according to any of claims 1 to 29, wherein the rectification column comprises, as separating internals, valve trays.

31. The process according to any of claims 1 to 30, wherein the rectification column has from 5 to 25 theoretical plates.

32. The process according to any of claims 1 to 31, wherein the top pressure in the rectification column is ≥ 5 bar and ≤ 25 bar.

33. The process according to any of claims 1 to 32, wherein the bottom temperature in the rectification column is ≤ 100°C.

34. The process according to any of claims 1 to 33, wherein the bottom temperature in the rectification column is from 40 to 90°C.

35. The process according to any of claims 1 to 34, wherein the crude propane is fed into the rectification column in such a way that the number of theoretical plates above the feed point is greater than the number of theoretical plates below the feed point.

36. The process according to any of claims 1 to 35, wherein the prepurified propane is withdrawn at the top of the rectification column.

37. The process according to any of claims 1 to 36, wherein the prepurified propane is withdrawn in gaseous form from the rectification column.

38. The process according to any of claims 1 to 37, wherein the rectification column has a water-cooled top condenser.

39. The process according to claim 38, wherein the water is fed to the top condenser with a temperature of ≥ 0°C and ≤ 40°C.

40. The process according to any of claims 1 to 39, wherein the prepurified propane is withdrawn in liquid form from the rectification column.

41. The process according to any of claims 1 to 40, wherein the prepurified propane consists to an extent of at least 99% by weight of propane, propylene, ethane and ethylene.

42. The process according to any of claims 1 to 41, wherein all reaction steps are carried out in one reaction zone and over a catalyst charge disposed therein.

43. The process according to any of claims 1 to 42, wherein the first reaction step is a heterogeneously catalyzed oxydehydrogenation.

44. The process according to any of claims 1 to 42, wherein the first reaction step is a heterogeneously catalyzed dehydrogenation.

45. The process according to claim 44, wherein the heterogeneously catalyzed dehydrogenation is carried out adiabatically.

46. The process according to claim 44, wherein the heterogeneously catalyzed dehydrogenation is carried out autothermally.

47. The process according to claim 44, wherein the heterogeneously catalyzed dehydrogenation is carried out in a tray reactor.

48. The process according to any of claims 1 to 47, wherein gas mixture 2 comprises from > 0 to 30% by volume of steam.

49. The process according to any of claims 1 to 48, wherein prepurified propane is fed directly into at least one reaction step of the heterogeneously catalyzed gas phase partial oxidation and/or partial gas phase ammoxidation of propylene.

50. The process according to claim 49, wherein at least 25% by weight of the total requirement for prepurified propane is fed directly to at least one reaction step of the heterogeneously catalyzed gas phase partial oxidation and/or partial gas phase ammoxidation of propylene.

51. The process according to claim 49, wherein at least 50% by weight of the total requirement for prepurified propane is fed directly to at least one reaction step of the heterogeneously catalyzed gas phase partial oxidation and/or partial gas phase ammoxidation of propylene.

52. The process according to any of claims 1 to 51, wherein the target product P is acrolein, acrylonitrile, acrylic acid and/or propylene oxide.

53. The process according to any of claims 1 to 52, wherein gas mixture 2 is subjected to a two-stage heterogeneously catalyzed partial oxidation of the propylene present therein to acrylic acid.

54. The process according to claim 53, wherein propylene is partially oxidized to acrolein in the first partial oxidation stage with the proviso that the partial oxidation is effected over catalysts disposed in a catalyst bed, whose active composition is at least one multimetal oxide of the general formula (IV),
Mo₁₂BiₐFe_{b}X¹_{c}X²_{d}X³ₑX⁴_{f}Oₙ (IV)
where
X¹ = nickel and/or cobalt,
X² = thallium, an alkali metal and/or an alkaline earth metal,
X³ = zinc, phosphorus, boron, antimony, tin, cerium, lead and/or tungsten,
X⁴ = is silicon, aluminum, titanium and/or zirconium,
a = from 0.5 to 5,
b = from 0.01 to 5, preferably from 2 to 4,
c = from 0 to 10, preferably from 3 to 10,
d = from 0 to 2, preferably from 0.02 to 2,
e = from 0 to 8, preferably from 0 to 5,
f = from 0 to 10 and
n = a number which is determined by the valency and frequency of the elements in IV other than oxygen.

55. The process according to claim 53 or 54, wherein acrolein is partially oxidized to acrylic acid in the second partial oxidation stage with the proviso that the partial oxidation is effected over catalysts disposed in a catalyst bed whose active composition is at least one multimetal oxide of the general formula (VII)
Mo₁₂VₐX¹_{b}X²_{c}X³_{d}X⁴ₑX⁵_{f}X⁶_{g}Oₙ (VII)
where
X¹ =W, Nb, Ta, Cr and/or Ce,
X² =Cu, Ni, Co, Fe, Mn and/or Zn,
X³ =Sb and/or Bi,
X⁴ =one or more alkali metals,
X⁵ =one or more alkali earth metals,
X⁶ =Si, Al, Ti and/or Zr,
a = from 1 to 6,
b = from 0.2 to 4,
c = from 0.5 to 18,
d = from 0 to 40,
e = from 0 to 2,
f = from 0 to 4,
g = from 0 to 40 and
n = a number which is determined by the valency and frequency of the elements in VII other than oxygen.

56. The process according to any of claims 53 to 55, wherein the propylene hourly space velocity on the catalyst bed of the first reaction stage is ≥ 120 1 (STP)/l•h.

57. The process according to any of claims 53 to 56, wherein the second reaction stage is charged with a gas mixture which comprises:
| | |
|---|---|
| from 4.5 to 8% | by volume of acrolein, |
| from 2.25 to 9% | by volume of molecular oxygen, |
| from 6 to 30% | by volume of propane, |
| from 32 to 72% | by volume of molecular nitrogen and |
| from 5 to 30% | by volume of steam. |

58. The process according to any of claims 53 to 57, wherein the acrolein hourly space velocity on the fixed catalyst bed of the second reaction stage is ≥ 110 1 (STP)/l•h.

59. The process according to any of claims 53 to 58, wherein the reaction temperature in the first reaction stage is from 300 to 450°C.

60. The process according to any of claims 53 to 59, wherein the reaction temperature in the second reaction stage is from 200 to 370°C.

61. The process according to any of claims 1 to 60, wherein the at least one removal step comprises a basic removal step in which the at least one target product P from gas mixture 3 is converted to the liquid phase.

62. The process according to claim 61, wherein the basic removal step comprises a fractional condensation of gas mixture 3 and/or an absorption of the target product P from gas mixture 3 into water or into aqueous solution.

63. The process according to claim 62, wherein the target product P is acrylic acid and the basic removal step is carried out in a column comprising separating internals with side draw removal of crude acrylic acid, and the crude acrylic acid is subjected to a suspension crystallization.

64. The process according to claim 63, wherein the acrylic acid suspension crystals obtained in the suspension crystallization are removed from remaining mother liquor by means of a wash column.

65. The process according to claim 64, wherein the wash column is one with forced transport of the crystal bed.

66. The process according to claim 65, wherein the wash column is a hydraulic wash column.

67. The process according to claim 66, wherein the wash liquid used is the melt of acrylic acid crystals removed beforehand in the wash column.

68. The process according to any of claims 64 to 67, wherein the removed acrylic acid suspension crystals are melted and at least one free-radical polymerization process follows, in which molten acrylic acid crystals are free-radically polymerized to prepare polymers.

69. The process according to any of claims 1 to 68, wherein bottoms liquid is withdrawn continuously from the rectification column and fed as cofeed to a cracker for paraffinic hydrocarbons.

70. The process according to any of claims 1 to 68, wherein bottoms liquid is withdrawn continuously from the rectification column and further treated by rectification.

## Revendications

1. Procédé de fabrication d'au moins un produit cible P par oxydation et/ou ammoxydation partielle de propylène, selon lequel
a) du propane auparavant purifié est soumis lors d'une première étape de réaction en présence et/ou avec exclusion d'oxygène moléculaire à au moins une déshydrogénation du groupe comprenant la déshydrogénation homogène, la déshydrogénation sous catalyse hétérogène, l'oxydéshydrogénation homogène et l'oxydéshydrogénation sous catalyse hétérogène, un mélange gazeux 1 contenant le propane non réagi et le propylène formé étant obtenu, et
b) une partie ou la totalité des constituants différents du propane et du propylène contenus dans le mélange gazeux 1 est éventuellement séparée à partir de la totalité ou d'une partie du mélange gazeux 1 et/ou transformée en d'autres composés, et un mélange gazeux 1' contenant du propane et du propylène est formé et, lors d'au moins une étape de réaction supplémentaire,
c) le mélange gazeux 1 ou le mélange gazeux 1' ou un mélange du mélange gazeux 1' formé et du mélange gazeux 1 restant est soumis en tant que constituant d'un mélange gazeux 2 à une oxydation partielle en phase gazeuse sous catalyse hétérogène et/ou à une ammoxydation partielle en phase gazeuse du propylène contenu dans le mélange gazeux 1 et/ou le mélange gazeux 1', un mélange gazeux 3 contenant ledit au moins un produit cible P étant obtenu,
d) le produit cible est séparé du mélange gazeux 3 en au moins une étape de séparation et au moins le propane est recyclé dans la première étape de réaction à partir du gaz résiduel restant, **caractérisé en ce que** le propane auparavant purifié est formé à partir d'un propane brut qui contient
≥ 90 % en poids de propane,
≤ 99 % en poids de propane et de propylène,
≥ 100 ppm en poids d'hydrocarbures en C₂ et
≥ 100 ppm en poids d'hydrocarbures en C₄,
à condition que le propane brut soit introduit dans une colonne de rectification et que le propane purifié soit soutiré au-dessus de l'emplacement d'alimentation, à condition que la teneur en hydrocarbures en C₂ en % en poids relative au propane contenu dans le propane purifié ne soit pas de moins de 80 % et pas de plus de 110 % de la teneur correspondante dans le propane brut et que la teneur en hydrocarbures en C₄ en % en poids relative au propane contenu dans le propane purifié soit d'au plus 50 % de la teneur correspondante dans le propane brut.

2. Procédé selon la revendication 1, **caractérisé en ce que** le propane brut contient ≥ 200 ppm en poids d'hydrocarbures en C₂.

3. Procédé selon la revendication 1, **caractérisé en ce que** le propane brut contient ≥ 500 ppm en poids d'hydrocarbures en C₂.

4. Procédé selon la revendication 1, **caractérisé en ce que** le propane brut contient ≥ 800 ppm en poids d'hydrocarbures en C₂.

5. Procédé selon la revendication 1, **caractérisé en ce que** le propane brut contient ≥ 1 000 ppm en poids d'hydrocarbures en C₂.

6. Procédé selon l'une quelconque des revendications 1 à 5, **caractérisé en ce que** les hydrocarbures en C₂ contenus dans le propane brut sont constitués d'au moins 90 % en poids d'éthane et d'éthylène.

7. Procédé selon l'une quelconque des revendications 1 à 5, **caractérisé en ce que** les hydrocarbures en C₂ contenus dans le propane brut sont constitués d'au moins 94 % en poids d'éthane et d'éthylène.

8. Procédé selon l'une quelconque des revendications 1 à 7, **caractérisé en ce que** les hydrocarbures en C₂ contenus dans le propane brut sont constitués d'au moins 50 % en poids d'éthane.

9. Procédé selon l'une quelconque des revendications 1 à 7, **caractérisé en ce que** les hydrocarbures en C₂ contenus dans le propane brut sont constitués d'au moins 70 % en poids d'éthane.

10. Procédé selon l'une quelconque des revendications 1 à 7, **caractérisé en ce que** les hydrocarbures en C₂ contenus dans le propane brut sont constitués d'au moins 90 % en poids d'éthane.

11. Procédé selon l'une quelconque des revendications 1 à 10, **caractérisé en ce que** le propane brut contient ≥ 200 ppm en poids d'hydrocarbures en C₄.

12. Procédé selon l'une quelconque des revendications 1 à 10, **caractérisé en ce que** le propane brut contient ≥ 500 ppm en poids d'hydrocarbures en C₄.

13. Procédé selon l'une quelconque des revendications 1 à 10, **caractérisé en ce que** le propane brut contient ≥ 1 000 ppm en poids d'hydrocarbures en C₄.

14. Procédé selon l'une quelconque des revendications 1 à 13, **caractérisé en ce que** les hydrocarbures en C₄ contenus dans le propane brut sont constitués d'au moins 80 % en poids de butane.

15. Procédé selon l'une quelconque des revendications 1 à 13, **caractérisé en ce que** les hydrocarbures en C₄ contenus dans le propane brut sont constitués d'au moins 90 % en poids de butane.

16. Procédé selon la revendication 15, **caractérisé en ce que** le butane contenu dans le propane brut est à ≥ 50 % en poids de l'isobutane.

17. Procédé selon la revendication 16, **caractérisé en ce que** le butane contenu dans le propane brut est à ≥ 70 % en poids de l'isobutane.

18. Procédé selon l'une quelconque des revendications 1 à 17, **caractérisé en ce que** la teneur en hydrocarbures en C₄ en % en poids relative au propane contenu dans le propane auparavant purifié est d'au plus 30 % de la teneur correspondante dans le propane brut.

19. Procédé selon l'une quelconque des revendications 1 à 17, **caractérisé en ce que** la teneur en hydrocarbures en C₄ en % en poids relative au propane contenu dans le propane auparavant purifié est d'au plus 10 % de la teneur correspondante dans le propane brut.

20. Procédé selon l'une quelconque des revendications 1 à 17, **caractérisé en ce que** la teneur en hydrocarbures en C₄ en % en poids relative au propane contenu dans le propane auparavant purifié est d'au plus 1 % de la teneur correspondante dans le propane brut.

21. Procédé selon l'une quelconque des revendications 1 à 20, **caractérisé en ce que** la teneur en hydrocarbures en C₂ en % en poids relative au propane contenu dans le propane auparavant purifié n'est pas de moins de 85 % de la teneur correspondante dans le propane brut.

22. Procédé selon l'une quelconque des revendications 1 à 20, **caractérisé en ce que** la teneur en hydrocarbures en C₂ en % en poids relative au propane contenu dans le propane auparavant purifié n'est pas de moins de 90 % de la teneur correspondante dans le propane brut.

23. Procédé selon l'une quelconque des revendications 1 à 20, **caractérisé en ce que** la teneur en hydrocarbures en C₂ en % en poids relative au propane contenu dans le propane auparavant purifié n'est pas de moins de 95 % de la teneur correspondante dans le propane brut.

24. Procédé selon l'une quelconque des revendications 1 à 20, **caractérisé en ce que** la teneur en hydrocarbures en C₂ en % en poids relative au propane contenu dans le propane auparavant purifié n'est pas de moins de 100 % de la teneur correspondante dans le propane brut.

25. Procédé selon l'une quelconque des revendications 1 à 20, **caractérisé en ce que** la teneur en hydrocarbures en C₂ en % en poids relative au propane contenu dans le propane auparavant purifié est de plus de 100 % de la teneur correspondante dans le propane brut.

26. Procédé selon l'une quelconque des revendications 1 à 25, **caractérisé en ce que** la teneur du propane auparavant purifié en isobutane est ≤ 1 000 ppm en poids.

27. Procédé selon l'une quelconque des revendications 1 à 25, **caractérisé en ce que** la teneur du propane auparavant purifié en isobutane est ≤ 600 ppm en poids.

28. Procédé selon l'une quelconque des revendications 1 à 25, **caractérisé en ce que** la teneur du propane auparavant purifié en isobutane est ≤ 100 ppm en poids.

29. Procédé selon l'une quelconque des revendications 1 à 28, **caractérisé en ce que** la colonne de rectification contient en tant que composants intérieurs de séparation des plateaux d'échange de matière.

30. Procédé selon l'une quelconque des revendications 1 à 29, **caractérisé en ce que** la colonne de rectification contient des plateaux à soupapes en tant que composants intérieurs de séparation.

31. Procédé selon l'une quelconque des revendications 1 à 30, **caractérisé en ce que** la colonne de rectification comprend 5 à 25 étapes de séparation théoriques.

32. Procédé selon l'une quelconque des revendications 1 à 31, **caractérisé en ce que** la pression de tête dans la colonne de rectification est ≥ 5 bar et ≤ 25 bar.

33. Procédé selon l'une quelconque des revendications 1 à 32, **caractérisé en ce que** la température de fond dans la colonne de rectification est ≤ 100 °C.

34. Procédé selon l'une quelconque des revendications 1 à 33, **caractérisé en ce que** la température de fond dans la colonne de rectification est de 40 à 90 °C.

35. Procédé selon l'une quelconque des revendications 1 à 34, **caractérisé en ce que** l'introduction du propane brut dans la colonne de rectification a lieu de sorte que le nombre d'étapes de séparation théoriques au-dessus de l'emplacement d'alimentation soit supérieur au nombre d'étapes de séparation théoriques en dessous de l'emplacement d'alimentation.

36. Procédé selon l'une quelconque des revendications 1 à 35, **caractérisé en ce que** le propane auparavant purifié est soutiré à la tête de la colonne de rectification.

37. Procédé selon l'une quelconque des revendications 1 à 36, **caractérisé en ce que** le propane auparavant purifié est soutiré sous forme gazeuse de la colonne de rectification.

38. Procédé selon l'une quelconque des revendications 1 à 37, **caractérisé en ce que** la colonne de rectification comprend un condensateur de tête refroidi par de l'eau.

39. Procédé selon la revendication 38, **caractérisé en ce que** l'eau est introduite dans le condensateur de tête à une température ≥ 0 °C et ≤ 40 °C.

40. Procédé selon l'une quelconque des revendications 1 à 39, **caractérisé en ce que** le propane auparavant purifié est soutiré sous forme liquide de la colonne de rectification.

41. Procédé selon l'une quelconque des revendications 1 à 40, **caractérisé en ce que** le propane auparavant purifié est constitué d'au moins 99 % en poids de propane, de propylène, d'éthane et d'éthylène.

42. Procédé selon l'une quelconque des revendications 1 à 41, **caractérisé en ce que** toutes les étapes de réaction sont réalisées dans une zone de réaction et sur un garnissage catalytique se trouvant dans celle-ci.

43. Procédé selon l'une quelconque des revendications 1 à 42, **caractérisé en ce que** la première étape de réaction est une oxydéshydrogénation sous catalyse hétérogène.

44. Procédé selon l'une quelconque des revendications 1 à 42, **caractérisé en ce que** la première étape de réaction est une déshydrogénation sous catalyse hétérogène.

45. Procédé selon la revendication 44, **caractérisé en ce que** la déshydrogénation sous catalyse hétérogène est réalisée de manière adiabatique.

46. Procédé selon la revendication 44, **caractérisé en ce que** la déshydrogénation sous catalyse hétérogène est réalisée de manière autotherme.

47. Procédé selon la revendication 44, **caractérisé en ce que** la déshydrogénation sous catalyse hétérogène est réalisée dans un réacteur à étages.

48. Procédé selon l'une quelconque des revendications 1 à 47, **caractérisé en ce que** le mélange gazeux 2 contient > 0 à 30 % en volume de vapeur d'eau.

49. Procédé selon l'une quelconque des revendications 1 à 48, **caractérisé en ce qu'**une introduction directe de propane auparavant purifié a lieu dans au moins une étape de réaction de l'oxydation partielle en phase gazeuse sous catalyse hétérogène et/ou de l'ammoxydation partielle en phase gazeuse de propylène.

50. Procédé selon la revendication 49, **caractérisé en ce qu'**au moins 25 % en poids de la demande totale en propane auparavant purifié est introduite directement dans au moins une étape de réaction de l'oxydation partielle en phase gazeuse sous catalyse hétérogène et/ou de l'ammoxydation partielle en phase gazeuse de propylène.

51. Procédé selon la revendication 49, **caractérisé en ce qu'**au moins 50 % en poids de la demande totale en propane auparavant purifié est introduite directement dans au moins une étape de réaction de l'oxydation partielle en phase gazeuse sous catalyse hétérogène et/ou de l'ammoxydation partielle en phase gazeuse de propylène.

52. Procédé selon l'une quelconque des revendications 1 à 51, **caractérisé en ce que** le produit cible P est l'acroléine, l'acrylonitrile, l'acide acrylique et/ou l'oxyde de propylène.

53. Procédé selon l'une quelconque des revendications 1 à 52, **caractérisé en ce que** le mélange gazeux 2 est soumis à une oxydation partielle sous catalyse hétérogène à deux étapes du propylène qu'il contient en acide acrylique.

54. Procédé selon la revendication 53, **caractérisé en ce que**, lors de la première étape d'oxydation partielle, le propylène est oxydé partiellement en acroléine, à condition que l'oxydation partielle ait lieu sur des catalyseurs se trouvant dans un lit catalytique, dont la masse active est au moins un oxyde de plusieurs métaux de formule générale (IV)
Mo₁₂BiₐFe_{b}X¹_{c}X²_{d}X³ₑX⁴_{f}Oₙ (IV)
avec
X¹ = nickel et/ou cobalt,
X² = thallium, un métal alcalin et/ou un métal alcalinoterreux,
X³ = zinc, phosphore, bore, antimoine, étain, cérium, plomb et/ou tungstène,
X⁴ = silicium, aluminium, titane et/ou zirconium,
a = 0,5 à 5,
b = 0,01 à 5, de préférence 2 à 4,
c = 0 à 10, de préférence 3 à 10,
d = 0 à 2, de préférence 0,02 à 2,
e = 0 à 8, de préférence 0 à 5,
f = 0 à 10, et
n = un nombre déterminé par la valence et la fréquence des éléments différent de l'oxygène dans IV.

55. Procédé selon la revendication 53 ou 54, **caractérisé en ce que**, lors de la seconde étape d'oxydation partielle, l'acroléine est oxydée partiellement en acide acrylique, à condition que l'oxydation partielle ait lieu sur des catalyseurs se trouvant dans un lit catalytique, dont la masse active est au moins un oxyde de plusieurs métaux de formule générale (VII)
Mo₁₂VₐX¹_{b}X²_{c}X³_{d}X⁴ₑX⁵_{f}X⁶_{g}Oₙ (VII)
avec
X¹ ₌ W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40, et
n = un nombre déterminé par la valence et la fréquence des éléments différent de l'oxygène dans VII.

56. Procédé selon l'une quelconque des revendications 53 à 55, **caractérisé en ce que** le chargement en propylène du lit catalytique de la première étape de réaction est ≥ 120 Nl/l·h.

57. Procédé selon l'une quelconque des revendications 53 à 56, **caractérisé en ce que** la seconde étape de réaction est alimentée avec un mélange gazeux qui contient
4,5 à 8 % en volume d'acroléine,
2,25 à 9 % en volume d'oxygène moléculaire,
6 à 30 % en volume de propane,
32 à 72 % en volume d'azote moléculaire et
5 à 30 % en volume de vapeur d'eau.

58. Procédé selon l'une quelconque des revendications 53 à 57, **caractérisé en ce que** le chargement en acroléine du lit catalytique fixe de la seconde étape de réaction est ≥ 110 Nl/l·h.

59. Procédé selon l'une quelconque des revendications 53 à 58, **caractérisé en ce que** la température de réaction dans la première étape de réaction est de 300 à 450 °C.

60. Procédé selon l'une quelconque des revendications 53 à 59, **caractérisé en ce que** la température de réaction dans la seconde étape de réaction est de 200 à 370 °C.

61. Procédé selon l'une quelconque des revendications 1 à 60, **caractérisé en ce que** ladite au moins une étape de séparation comprend une étape de séparation de base, selon laquelle ledit au moins un produit cible P est transféré dans la phase liquide à partir du mélange gazeux 3.

62. Procédé selon la revendication 61, **caractérisé en ce que** l'étape de séparation de base comprend une condensation fractionnée du mélange gazeux 3 et/ou une absorption du produit cible P à partir du mélange gazeux 3 dans de l'eau ou dans une solution aqueuse.

63. Procédé selon la revendication 62, **caractérisé en ce que** le produit cible P est l'acide acrylique et l'étape de séparation de base est réalisée dans une colonne contenant des composants intérieurs de séparation avec soutirage latéral d'un acide acrylique brut, et l'acide acrylique brut est soumis à une cristallisation en suspension.

64. Procédé selon la revendication 63, **caractérisé en ce que** le cristallisat en suspension d'acide acrylique formé lors de la cristallisation en suspension est séparé de la liqueur mère restante au moyen d'une colonne de lavage.

65. Procédé selon la revendication 64, **caractérisé en ce que** la colonne de lavage est une colonne à transport forcé du lit cristallin.

66. Procédé selon la revendication 65, **caractérisé en ce que** la colonne de lavage est une colonne de lavage hydraulique.

67. Procédé selon la revendication 66, **caractérisé en ce que** la masse fondue des cristaux d'acide acrylique séparés auparavant dans la colonne de lavage est utilisée en tant que liquide de lavage.

68. Procédé selon l'une quelconque des revendications 64 à 67, **caractérisé en ce que** le cristallisat en suspension d'acide acrylique séparé est fondu et au moins un procédé de polymérisation radicalaire s'ensuit, lors duquel le cristallisat d'acide acrylique fondu est polymérisé par voie radicalaire pour la fabrication de polymères.

69. Procédé selon l'une quelconque des revendications 1 à 68, **caractérisé en ce que** le liquide de fond est soutiré en continu de la colonne de rectification et introduit en tant que co-alimentation dans un craqueur pour hydrocarbures paraffiniques.

70. Procédé selon l'une quelconque des revendications 1 à 68, **caractérisé en ce que** le liquide de fond est soutiré en continu de la colonne de rectification et transformé par rectification.
